# EUROPÄISCHE PATENTANMELDUNG

(11) **EP 1 717 232 A1**
(43) Veröffentlichungstag der Anmeldung: **02.11.2006**
(21) Anmeldenummer: 05009272.5
(22) Anmeldetag: 28.04.2005
(51) Int. Cl.: C07D 239/46, C07D 239/42, C07D 239/52, C07D 251/42, C07D 251/46, C07D 401/12, C07D 409/12, A01N 43/54, A01N 43/66, A01N 43/68, A01N 43/70

(54) **Phenylsulfonylharnstoffe mit herbizider Wirkung**

(71) Anmelder: Bayer CropScience GmbH, 65929 Frankfurt/Main (DE)
(72) Erfinder: Waldraff, Christian, 61118 Bad Vilbel (DE); Dietrich, Hansjörg, 65719 Hofheim (DE); Ort, Oswald, 51375 Leverkusen (DE); Kehne, Heinz, 65719 Hofheim (DE); Hills, Martin, 65510 Idstein (DE); Auler, Thomas, 42799 Leichlingen (DE); Müller, Klaus-Helmut, 40593 Düsseldorf (DE); Feucht, Dieter, 65760 Eschborn (DE)

(57) **Zusammenfassung**

Die vorliegende Erfindung betrifft eine Verbindung der Formel (I) und/oder deren Salze 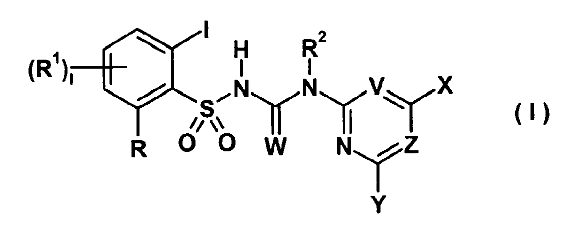 worin
R
ein Kohlenwasserstoffrest oder Kohlenwasserstoffoxyrest ist, der unsubstituiert oder substituiert ist und inklusive Substituenten 1 bis 30 C-Atome aufweist, oder ein Heterocyclylrest oder Heterocyclyloxyrest ist, welcher unsubstituiert oder substituiert ist,
oder R ein Rest OC(O)R³, S(O)ₙR³, OS(O)ₙR³, F, Br, I, OH, CN, NO₂, NH₂, SF₅, NR⁴R⁵ oder Si(R⁶)₃ ist, wobei n gleich 0, 1 oder 2 ist,
R¹
unabhängig voneinander Halogen, OH, SH, ein kohlenstofffreier stickstoffhaltiger Rest oder ein kohlenstoffhaltiger Rest sind, der 1 bis 30 C-Atome aufweist, oder R⁰ und R^{#} miteinander einen stickstoffhaltigen heterocyclischen Ring bilden,
I
0, 1, 2 oder 3 ist,
R²
ein Wasserstoffatom oder ein Kohlenwasserstoffrest ist, der unsubstituiert oder substituiert ist und inklusive Substituenten 1 bis 20 C-Atome aufweist,
R³
ein Kohlenwasserstoffrest oder Kohlenwasserstoffoxyrest ist, der unsubstituiert oder substituiert ist und inklusive Substituenten 1 bis 30 C-Atome aufweist, oder ein Heterocylylrest oder Heterocylyloxyrest ist, welcher unsubstituiert oder substituiert ist, oder R³ ein Wasserstoffatom, CN oder NR⁴R⁵ ist,
R⁴
eine Gruppe der Formel R⁰-Q⁰- ist, worin
R⁰ ein Wasserstoffatom, ein Acylrest, ein Kohlenwasserstoffrest oder ein Heterocyclylrest bedeutet, wobei jeder der letztgenannten beiden Reste unsubstituiert oder substituiert ist und inklusive Substituenten 1 bis 30 C-Atome aufweist, und
Q⁰ eine direkte Bindung oder eine divalente Gruppe der Formel -O- oder -N(R^{#})- bedeutet, wobei R^{#} ein Wasserstoffatom, ein Acylrest oder ein Kohlenwasserstoffrest bedeutet und wobei der letztgenannte Rest unsubstituiert oder substituiert ist und inklusive Substituenten 1 bis 30 C-Atome aufweist, oder R⁰ und R^{#} miteinander einen stickstoffhaltigen heterocyclischen Ring bilden,
R⁵
ein Wasserstoffatom, ein Acylrest, ein Kohlenwasserstoffrest oder ein Heterocyclylrest ist, wobei jeder der letztgenannten beiden Reste unsubstituiert oder substituiert ist und inklusive Substituenten 1 bis 30 C-Atome aufweist, oder
R⁴ und R⁵
miteinander einen stickstoffhaltigen heterocyclischen Ring bilden,
R⁶
ein Kohlenwasserstoffrest ist, der unsubstituiert oder substituiert ist,
W
ein Sauerstoffatom oder ein Schwefelatom ist,
X,Y
unabhängig voneinander für ein Wasserstoffatom, Halogen, (C₁-C₆)Alkyl, (C₁₋C₆)Alkoxy oder (C₁-C₆)Alkylthio ist, wobei jeder der letztgenannten 3 Reste unsubstituiert oder durch einen oder mehrere Reste aus der Gruppe Halogen, (C₁-C₄)Alkoxy, und (C₁-C₄)Alkylthio substituiert ist, oder Mono- oder Di[(C₁₋C₆)alkyl] amino, (C₂-C₆)Alkenyl, (C₂-C₆)Alkinyl, (C₃-C₆)Alkenyloxy oder (C₃₋C₆)Alkinyloxy bedeuten, und
V,Z
unabhängig voneinander CH oder N bedeuten.

Die Verbindung eignet sich im Bereich des Pflanzenschutzes.

## Beschreibung

Es ist bekannt, daß substituierte Phenylsulfonylharnstoffe herbizide Eigenschaften besitzen können. Dabei handelt es sich z.B. um Phenylderivate, die einfach oder mehrfach substituiert sind (z.B. US 4127405, WO 9209608, BE 853374, WO 9213845, EP 84020, WO 9406778, WO 02072560, US 4169719).

Ebenso zeigen Phenylsulfonylharnstoffe mit einem allgemeinen 1,2,3-Substitutionsmuster herbizide Eigenschaften (z.B. WO 9732861, WO 02062768).

Überraschenderweise wurden nun spezielle lod-substituierte Phenylsulfonylharnstoffe gefunden, die sich besonders vorteilhaft als Herbizide oder Pflanzenwachstumsregulatoren eignen.

Gegenstand der vorliegenden Erfindung sind somit Verbindungen der allgemeinen Formel (I) und/oder deren Salze, 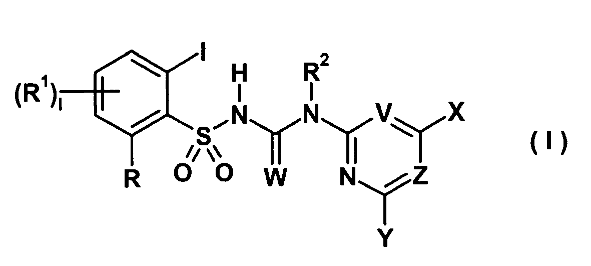 worin
- R: ein Kohlenwasserstoffrest oder Kohlenwasserstoffoxyrest, vorzugsweise ein Rest aus der Gruppe Alkyl, Alkenyl, Alkinyl, Cycloalkyl, Cycloalkenyl, Cycloalkinyl, Alkoxy, Alkenyloxy, Alkinyloxy, Cycloalkyloxy, Cycloalkenyloxy, Aryl oder Aryloxy ist, der unsubstituiert oder substituiert ist und inklusive Substituenten 1 bis 30 C-Atome, vorzugsweise 1 bis 20 C-Atome aufweist, oder R ein Heterocyclylrest oder Heterocyclyloxyrest ist, welcher unsubstituiert oder substituiert ist,
oder R ein Rest OC(O)R³, S(O)ₙR³, OS(O)ₙR³, F, Br, I, OH, CN, NO₂, NH₂, SF₅, NR⁴R⁵oder Si(R⁶)₃ ist, wobei n gleich 0, 1 oder 2 ist,
- R¹: unabhängig voneinander Halogen, OH, SH, ein kohlenstofffreier stickstoffhaltiger Rest oder ein kohlenstoffhaltiger Rest ist, der 1 bis 30 C-Atome, vorzugsweise 1 bis 20 C-Atome aufweist,
- I: 0, 1, 2 oder 3, vorzugsweise 0, 1 oder 2, besonders bevorzugt 0 oder 1, ganz besonders bevorzugt 0 ist,
- R²: ein Wasserstoffatom oder ein Kohlenwasserstoffrest ist, der unsubstituiert oder substituiert ist und inklusive Substituenten 1 bis 20 C-Atome, vorzugsweise 1 bis 10 C-Atome aufweist, z.B. unsubstituiertes oder substituiertes (C₁-C₄)Alkyl, vorzugsweise H oder CH₃,
- R³: ein Kohlenwasserstoffrest oder Kohlenwasserstoffoxyrest, vorzugsweise ein Rest aus der Gruppe Alkyl, Alkenyl, Alkinyl, Cycloalkyl, Cycloalkenyl, Cycloalkinyl, Alkoxy, Alkenyloxy, Alkinyloxy, Cycloalkyloxy, Cycloalkenyloxy, Aryl oder Aryloxy ist, der unsubstituiert oder substituiert ist und inklusive Substituenten 1 bis 30 C-Atome, vorzugsweise 1 bis 20 C-Atome aufweist, oder R³ ein Heterocylylrest oder Heterocylyloxyrest ist, welcher unsubstituiert oder substituiert ist, oder R³ ein Wasserstoffatom, CN oder NR⁴R⁵ ist,
- R⁴: eine Gruppe der Formel R⁰-Q⁰- ist, worin
R° ein Wasserstoffatom, ein Acylrest, ein Kohlenwasserstoffrest oder ein Heterocyclylrest bedeutet, wobei jeder der letztgenannten beiden Reste unsubstituiert oder substituiert ist und inklusive Substituenten 1 bis 30 C-Atome, vorzugsweise 1 bis 20 C-Atome aufweist, und
Q° eine direkte Bindung oder eine divalente Gruppe der Formel ―O- oder ― N(R^{#})- bedeutet, wobei R^{#} ein Wasserstoffatom, ein Acylrest oder ein Kohlenwasserstoffrest bedeutet und wobei der letztgenannte Rest unsubstituiert oder substituiert ist und inklusive Substituenten 1 bis 30 C-Atome, vorzugsweise 1 bis 20 C-Atome aufweist, oder R⁰ und R^{#} miteinander einen stickstoffhaltigen heterocyclischen Ring bilden,
- R⁵: ein Wasserstoffatom, ein Acylrest, ein Kohlenwasserstoffrest oder ein Heterocyclylrest ist, wobei jeder der letztgenannten beiden Reste unsubstituiert oder substituiert ist und inklusive Substituenten 1 bis 30 C-Atome, vorzugsweise 1 bis 20 C-Atome aufweist, oder
- R⁴ und R⁵: miteinander einen stickstoffhaltigen heterocyclischen Ring bilden,
- R⁶: ein Kohlenwasserstoffrest ist, der unsubstituiert oder substituiert ist und inklusive Substituenten 1 bis 30 C-Atome, vorzugsweise 1 bis 20 C-Atome aufweist, vorzugsweise (C₁-C₄)Alkyl oder (C₆-C₁₀)Aryl,
- W: ein Sauerstoffatom oder ein Schwefelatom ist,
- X,Y: unabhängig voneinander für ein Wasserstoffatom, Halogen, (C₁-C₆)Alkyl, (C₁₋C₆)Alkoxy oder (C₁-C₆)Alkylthio ist, wobei jeder der letztgenannten 3 Reste unsubstituiert oder durch einen oder mehrere Reste aus der Gruppe Halogen, (C₁-C₄)Alkoxy, und (C₁-C₄)Alkylthio substituiert ist, oder Mono- oder Di[(C₁₋C₆)alkyl] amino, (C₂-C₆)Alkenyl, (C₂-C₆)Alkinyl, (C₃-C₆)Alkenyloxy oder (C₃₋C₆)Alkinyloxy bedeuten, und
- V,Z: unabhängig voneinander CH oder N bedeuten.

Die Verbindungen der Formel (I) können Salze bilden, z.B. solche bei denen der Wasserstoff der -SO₂-NH-Gruppe durch ein für die Landwirtschaft geeignetes Kation ersetzt ist. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, H₂SO₄ oder HNO₃.

Kohlenstoffhaltige Reste sind organische Reste, die mindestens ein Kohlenstoffatom, vorzugsweise 1 bis 30 C-Atome, besonders bevorzugt 1 bis 20 C-Atome und außerdem mindestens ein Atom eines oder mehrerer anderer Elemente des Periodensystems der Elemente wie H, Si, N, P, O, S, F, Cl, Br oder J enthalten. Beispiele für kohlenstoffhaltige Reste sind unsubstituierte oder substituierte Kohlenwasserstoffreste, die direkt oder über ein Heteroatom wie N, S, P oder O an den Grundkörper gebunden sein können, unsubstituierte oder substituierte Heterocyclylreste, die direkt oder über ein Heteroatom wie N, S, P oder O an den Grundkörper gebunden sein können, kohlenstoffhaltige Acylreste oder Cyano.

In Formel (I) und allen nachfolgenden Formeln können die kohlenstoffhaltigen Reste wie Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl in der Form (C₃-C₄)Alkenyl, (C₃-C₅)Alkenyl, (C₃-C₆)Alkenyl, (C₃-C₈)Alkenyl oder (C₃-C₁₂)Alkenyl bedeutet vorzugsweise einen Alkenylrest mit 3 bis 4, 3 bis 5, 3 bis 6, 3 bis 8 bzw. 3 bis 12 C-Atomen, bei dem die Doppelbindung nicht an dem C-Atom liegt, das mit dem übrigen Molekülteil der Verbindung (I) verbunden ist ("yl"-Position). Entsprechendes gilt für (C₃-C₄)Alkinyl etc., (C₃-C₄)Alkenyloxy etc. und (C₃-C₄)Alkinyloxy etc.

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl.

Kohlenstofffreie stickstoffhaltige Reste sind Reste die vorzugsweise 1 bis 10 N-Atome, besonders bevorzugt 1 oder 2 N-Atome enthalten und außerdem vorzugsweise eines oder mehrere Atome eines oder mehrerer von Kohlenstoff verschiedener Elemente des Periodensystems der Elemente wie H, O oder S enthalten. Beispiele für kohlenstofffreie stickstoffhaltige Reste sind NH₂, NO₂, NHOH, NO, NH-NH₂ oder N₃.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl.

Ein heterocyclischer Rest oder Ring (Heterocyclyl) kann gesättigt, ungesättigt oder heteroaromatisch und unsubstituiert oder substituiert sein; er enthält vorzugsweise ein oder mehrere Heteroatome im Ring, vorzugsweise aus der Gruppe N, O und S; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Oxetanyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.
Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise Formyl, (C₁-C₄)Alkylcarbonyl oder (C₁-C₄)Alkylsulfonyl. Entsprechenes gilt für substituiertes Hydroxylamino oder Hydrazino.

Ein Acylrest bedeutet den Rest einer organischen Säure, der formal durch Abspaltung einer OH-Gruppe aus der organischen Säure entsteht, z.B. der Rest einer Carbonsäure und Reste davon abgeleiteter Säuren wie der Thiocarbonsäure, gegebenenfalls N-substituierten Iminocarbonsäuren oder die Reste von Kohlensäuremonoestern, gegebenenfalls N-substituierter Carbaminsäuren, Sulfonsäuren, Sulfinsäuren, Phosphonsäuren, Phosphinsäuren.

Ein Acylrest ist bevorzugt Formyl oder aliphatisches Acyl aus der Gruppe CO-R^{x}, CS-R^{x}, CO-OR^{x}, CS-OR^{x}, CS-SR^{x}, SOR^{Y} oder SO₂R^{Y}, wobei R^{x} und R^{Y} jeweils einen C₁-C₁₀-Kohlenwasserstoffrest bedeutet, der unsubstituiert oder substituiert ist, oder Aminocarbonyl oder Aminosulfonyl, wobei die beiden letztgenannten Reste unsubstituiert, N-monosubstituiert oder N,N-disubstituiert sind. Acyl bedeutet beispielsweise Formyl, Halogenalkylcarbonyl, Alkylcarbonyl wie (C₁-C₄)Alkylcarbonyl, Phenylcarbonyl, wobei der Phenylring substituiert sein kann, z.B. wie oben für Phenyl angegeben, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorstehenden Beispiele für Reste oder Restebereiche, die unter die allgemeinen Begriffe wie "Alkyl", "Acyl", "substituierten Reste" etc., fallen, bedeuten keine vollständige Aufzählung. Die allgemeinen Begriffe umfassen auch die weiter unten angeführten Definitionen für Restebereiche in Gruppen bevorzugter Verbindungen, insbesondere Restebereiche, welche spezifische Reste aus den Tabellenbeispielen umfassen.

Bevorzugte erfindungsgemäße Verbindungen der Formel (I) oder deren Salze sind solche, worin
- R: (C₁-C₆)Alkyl, (C₂-C₆)Alkenyl, (C₂-C₆)Alkinyl, (C₃-C₆)Cycloalkyl, (C₃₋C₆)Cycloalkenyl, (C₃-C₆)Cycloalkinyl, (C₁-C₆)Alkyloxy, (C₂-C₆)Alkenyloxy, (C₂₋C₆)Alkinyloxy, (C₃-C₆)Cycloalkyloxy, Phenyl, Phenyloxy, F, Br, I, OH, CN, NO₂, NH₂, SF₅, Si((C₁-C₆)Alkyl)₃, N((C₁-C₆)Alkyl)₂, NH(C₁-C₆)Alkyl, N((C₂₋C₆)Alkenyl)₂, NH(C₂-C₆)Alkenyl, N((C₂-C₆)Alkinyl)₂, NH(C₂-C₆)Alkinyl, NH((C₃₋C₆)Cycloalkyl)₂, NH(C₃-C₆)Cycloalkyl, N(C₁-C₆)Alkyl (C₃-C₆)Cycloalkyl, N(C₁₋C₆)Alkyl C(O)R³, NHC(O)R³, N(C₁-C₆)Alkyl S(O)ₙR³, NHS(O)ₙR³, S(O)ₙ(C₁₋C₄)Alkyl, S(O)ₙ (C₃-C₆)Cycloalkyl, S(O)ₙ(C₁-C₆)Alkenyl, S(O)ₙ(C₁-C₆)Alkinyl, S(O)ₙNHR³, S(O)ₙN(C₁-C₆)Alkyl R³, OSO₂(C₁-C₆)Alkyl, OSO₂(C₃₋C₆)Cycloalkyl, OSO₂(C₁-C₆)Alkenyl, OSO₂(C₁-C₆)Alkinyl, OS(O)ₙPhenyl, OSO₂N((C₁-C₆)Alkyl)₂, OSO₂NH(C₁-C₆)Alkyl, OSO₂N((C₃-C₆)Cycloalkyl)₂, OSO₂NH(C₃-C₆)Cycloalkyl, OSO₂N((C₂-C₆)Alkenyl)₂, OSO₂NH(C₂-C₆)Alkenyl, OSO₂N((C₂-C₆)Alkinyl)₂, OSO₂NH(C₂-C₆)Alkinyl, OC(O)R³ oder Heterocyclyl bedeutet, wobei die genannten Reste Alkyl, Alkenyl, Alkinyl, Cycloalkyl, Cycloalkenyl, Cycloalkinyl, Alkyloxy, Alkenyloxy, Alkinyloxy, Cycloalkoxy, Phenyl, Phenyloxy, Heterocyclyl unsubstituiert sind oder substituiert sind, z.B. durch einen oder mehrere Reste aus der Gruppe Halogen, CN, (C₁-C₆)Alkyl, (C₂-C₆)Alkenyl, (C₂-C₆)Alkinyl, (C₁-C₆)Alkoxy, (C₃-C₆)Cycloalkyl, Phenyl, Phenyloxy, Heterocyclyloxy, wobei die beiden letztgenannten Reste einfach oder mehrfach durch Reste aus der Gruppe Halogen, CN, Methyl, Methoxy, Trifluormethyl, Trifluormethoxy substituiert sein können,
- R¹: (C₁-C₆)Alkyl, (C₁-C₆)Haloalkyl, (C₁-C₆)Alkyloxy, (C₁-C₆)Haloalkoxy oder Halogen ist,
- l: 0, 1 oder 2, vorzugsweise 0 oder 1, besonders bevorzugt 0 ist,
- n: 0, 1 oder 2 ist,
- R²: H oder CH₃ ist,
- R³: H, (C₁-C₆)Alkyl, (C₂-C₆)Alkenyl, (C₂-C₆)Alkinyl, (C₃-C₆)Cycloalkyl, (C₁₋C₆)Alkyloxy, (C₂-C₆)Alkenyloxy, (C₂-C₆)Alkinyloxy, (C₃-C₆)Cycloalkyloxy, Phenyl, Heterocyclyl, CN, NH(C₁-C₆)Alkyl, N((C₁-C₆)Alkyl)₂ steht, wobei die genannten Reste Alkyl, Alkenyl, Alkinyl, Cycloalkyl, Alkyloxy, Alkenyloxy, Alkinyloxy, Cycloalkyloxy, Phenyl, Heterocyclyl unsubstituiert oder substituiert sind, z.B. durch einen oder mehrere Reste aus der Gruppe Halogen, CN, (C₁₋C₆)Alkyl, (C₁-C₆)Alkenyl, (C₁-C₆)Alkinyl, (C₁-C₆)Alkyloxy,
- W: ein Sauerstoffatom ist,
- X,Y: unabhängig voneinander (C₁-C₄)Alkyl, (C₁-C₄)Alkyloxy, wobei jeder der letztgenannten zwei Reste unsubstituiert oder durch einen oder mehrere Halogenatome substituiert ist, oder (C₁-C₄)Alkylthio, Halogen oder NH(C₁₋C₄)Alkyl oder N((C₁-C₄)Alkyl)₂ bedeuten, und
- V,Z: unabhängig voneinander CH oder N bedeuten.

Besonders bevorzugte Verbindungen der Formel (I) und/oder deren Salze sind solche, worin
- R: (C₁-C₄)Alkyl, (C₂-C₄)Alkenyl, (C₂-C₄)Alkinyl, (C₃-C₆)Cycloalkyl, (C₁₋C₄)Alkyloxy, (C₂-C₄)Alkenyloxy, (C₂-C₄)Alkinyloxy, (C₃-C₆)Cycloalkyloxy, Phenyl, Phenyloxy, F, Br, I, CN, NO₂, NH₂, N((C₁-C₄)Alkyl)₂, NH(C₁-C₄)Alkyl, NH(C₂-C₄)Alkenyl, NH(C₂-C₄)Alkinyl, NH(C₃-C₆)Cycloalkyl, N(C₁-C₄)Alkyl (C₃₋C₆)Cycloalkyl, S(C₁-C₄)Alkyl, S(C₂-C₄)Alkenyl, S(C₂-C₄)Alkinyl, S(C₃₋C₆)Cycloalkyl, S(O)(C₁-C₄)Alkyl, S(O)(C₁-C₄)Alkenyl, S(O)(C₂-C₄)Alkinyl, S(O)(C₃-C₆)Cycloalkyl, SO₂(C₁-C₄)Alkyl, SO₂(C₂-C₄)Alkenyl, SO₂(C₂₋C₄)Alkinyl, SO₂(C₃-C₆)Cycloalkyl, SO₂NH(C₁-C₄)Alkyl, SO₂N((C₁-C₄)Alkyl)₂, SO₂NH(C₃-C₆)Cycloalkyl, OSO₂(C₁-C₄)Alkyl, OSO₂NH(C₁-C₄)Alkyl, OSO₂N((C₁-C₄)Alkyl)₂ oder NHC(O)R³, NHSO₂R³, OC(O)R³, worin R³ gleich H, (C₁-C₄)Alkyl, (C₂-C₄)Alkenyl, (C₂-C₄)Alkinyl, (C₃₋C₆)Cycloalkyl, (C₁-C₄)Alkyloxy, (C₂-C₄)Alkenyloxy, (C₂-C₄)Alkinyloxy, (C₃₋C₆)Cycloalkyloxy, (C₁-C₄)Haloalkyl, NH(C₁-C₄)Alkyl oder N((C₁-C₄)Alkyl)₂ ist, wobei die genannten Reste Alkyl, Alkenyl, Alkinyl, Cycloalkyl, Alkyloxy, Alkenyloxy, Alkinyloxy, Cycloalkyloxy, Phenyl, Phenyloxy unsubstituiert sind oder substituiert sind, z.B. durch einen oder mehrere Reste, vorzugsweise ein, zwei oder drei Reste, aus der Gruppe Halogen (F, Cl, Br, I), (C₁-C₄)Alkyl, (C₁-C₄)Alkyloxy, (C₁-C₄)Haloalkyl, (C₁-C₄)Haloalkyloxy,
- R¹: Halogen (F, Cl, Br, I), (C₁-C₄)Alkyl, (C₁-C₄)Alkyloxy, (C₁-C₄)Haloalkyl, (C₁₋C₄)Haloalkyloxy,
- l: 0 oder 1, vorzugsweise 0 ist,
- R²: H oder (C₁-C₄)Alkyl wie Methyl,
- W: ein Sauerstoffatom,
- X,Y: unabhängig voneinander (C₁-C₄)Alkyl, (C₁-C₄)Haloalkyl, (C₁-C₄)Alkyloxy, (C₁₋C₄)Haloalkyloxy, Halogen (F, Cl, Br, I), (C₁-C₄)Alkylthio, NH(C₁-C₄)Alkyl, N((C₁-C₄)Alkyl)₂,
- V: ein Stickstoffatom, und
- Z: CH oder N bedeuten.

Ganz besonders bevorzugte erfindungsgemäße Verbindungen der Formel (I) oder deren Salze sind solche, worin
- R: CH₃, CH₂CH₃, (CH₂)₂CH₃, CH(CH₃)₂, C(CH₃)₃, CH=CH₂, C≡CH, CH₂CH=CH₂, CH₂C≡CH, Cyclopropyl, Phenyl, F, Br, I, CN, NO₂, NH₂, CH₂OCH₃, CF₃, CHF₂, NHCH₃, N(CH₃)₂, NH-cyclopropyl, N(CH₃)-cyclopropyl, NHC(O)H, NHC(O)CH₃, NHC(O)OCH₃, NHSO₂CH₃, NHSO₂CF₃, NHSO₂CHF₂, OCH₃, OCH₂CH₃, O(CH₂)₂CH₃, OCH(CH₃)₂, O(CH₂)₃CH₃, OCH₂CH(CH₃)₂, OCH(CH₃)CH₂CH₃, OC(CH₃)₃, OCH=CH₂, OC≡CH, OCH₂CH=CH₂, OCH₂C≡CH, O-cyclopropyl, OCH₂-cyclopropyl, O(CH₂)₂Cl, O(CH₂)₃Cl, OCH₂OCH₃, OPhenyl, OCH₂Phenyl, OCF₃, OCHF₂, OCH₂F, OCH₂CF₃, OCH₂CHF₂, OCH(CH₃)CF₃, OCH₂CF₂CF₃, SCH₃, SCH₂CH₃, S(O)CH₃, S(O)CH₂CH₃, SO₂CH₃, SO₂CH₂CH₃, SO₂NHCH₃, SO₂N(CH₃)₂, SO₂NHCF₃, SO₂NHCHF₂, OSO₂CH₃, OSO₂CF₃, OSO₂CHF₂, OSO₂N(CH₃)₂, OSO₂NHCF₃, OSO₂NHCHF₂, OC(O)H, OC(O)CH₃, OC(O)OCH₃, OC(O)N(CH₃)₂,
- I: 0 ist,
- R²: H ist,
- W: Sauerstoff,
- X,Y: unabhängig voneinander CH₃, CH₂CH₃, CF₃, CHF₂, CH₂CF₃, CH₂CHF₂, OCH₃, OCH₂CH₃, OCF₃, OCHF₂, OCH₂CF₃, OCH₂CHF₂, F, Cl, Br, I, SCH₃, NHCH₃, N(CH₃)₂, vorzugsweise CH₃, OCH₃, OCH₂CH₃, Cl, N(CH₃)₂,
- V: N ist, und
- Z: CH oder N bedeuten.

Besonders bevorzugt sind auch erfindungsgemäße Verbindungen der Formel (I) und deren Salze, welche eine Kombination von Resten aus den oben genannten bevorzugten Verbindungen enthalten, sowie solche, welche einzelne oder mehrere Reste aus den in der Tabelle 1 dieser Beschreibung aufgeführten Verbindungen enthalten. Ebenfalls bevorzugt sind Verbindungen der Formel (I), in denen V = N ist.

Gegenstand der vorliegenden Erfindung sind auch Verfahren zur Herstellung der erfindungsgemäßen Verbindungen der allgemeinen Formel (I) und/oder deren Salzen, wobei man
a) eine Verbindung der Formel (II) 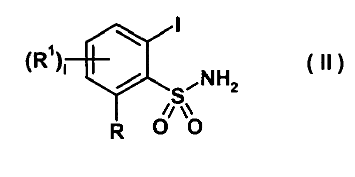 mit einem heterocyclischen (Thio)Carbamat der Formel (III), 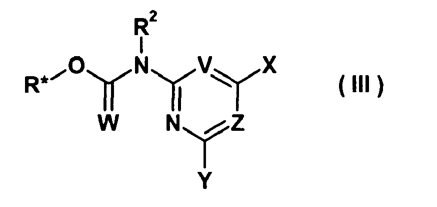 worin R* ein substituierter oder unsubstituierter C₁-C₂₀-Kohlenwasserstoffrest wie Aryl oder Alkyl, vorzugsweise gegebenenfalls substituiertes Phenyl oder gegebenenfalls substituiertes (C₁-C₄)Alkyl bedeutet, umsetzt, oder
b) ein Sulfonyl(thio)carbamat der Formel (IV), 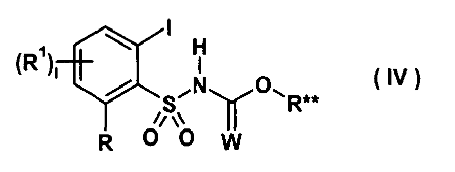 worin R** ein substituierter oder unsubstituierter C₁-C₂₀-Kohlenwasserstoffrest wie Aryl oder Alkyl, vorzugsweise gebenenfalls substituiertes Phenyl oder gegebenenfalls substituiertes (C₁-C₄)Alkyl bedeutet, mit einem Aminoheterocyclus der Formel (V) 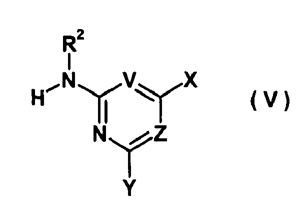 umsetzt, oder
c) ein Sulfonylisocyanat der Formel (VI) 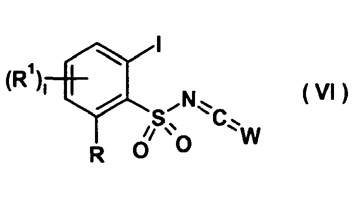 mit einem Aminoheterocyclus der Formel (V) umsetzt, oder
d) ein Sulfonamid der Formel (II) mit einem Iso(thio)cyanat der Formel (VII) 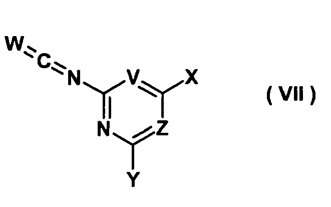 in Gegenwart einer Base umsetzt, oder
e) einen Aminoheterocyclus der Formel (V) zunächst basenkatalysiert mit einem Kohlensäureester, z.B. Diphenylcarbonat, umsetzt und das gebildete Intermediat in einer Eintopfreaktion mit einem Sulfonamid der Formel (II) (siehe Variante a) umsetzt, oder
f) ein Sulfonsäurehalogenid der Formel (VIII), 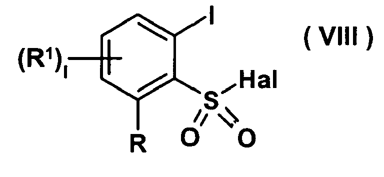 wobei Hal ein Halogenatom, vorzugsweise Chlor ist, mit einem (Thio)Cyanat, beispielsweise einem Metall(thio)cyanat, insbesondere einem Alkalimetall(thio)cyanat, wie Natrium(thio)cyanat, zu einem Iso(thio)cyanat der Formel (VI) oder einem solvatisierten (stabilisierten) Derivat davon umsetzt, und anschließend mit einem Aminoheterocyclus der Formel (V) umsetzt,
   wobei in den Formeln (II)-(VIII) die Reste, Gruppen bzw. Indizes R, R¹, R², V, W, X, Y, Z und I wie in Formel (I) definiert sind und auch die gleichen Vorzugsbereiche gelten wie für Formel (I) angegeben.

Die Umsetzung der Verbindungen der Formeln (II) und (III) gemäß Variante a) erfolgt vorzugsweise basenkatalysiert in einem inerten organischen Lösungsmittel, wie z.B. Dichlormethan, Acetonitril, Dioxan oder THF bei Temperaturen zwischen 0 °C und dem Siedepunkt des Lösungsmittels, vorzugsweise bei Raumtemperatur. Als Base werden dabei beispielsweise organische Aminbasen, wie 1,8-Diazabicyclo[5.4.0]undec-7-en (DBU), Alkali-tert.-butoxide, wie z.B. NaO-t-C₄H₉, oder Alkalihydroxide, wie z.B. NaOH, insbesondere bei R* = (subst.) Phenyl (vgl. EP-A-44 807), oder Trialkylaluminium wie Trimethylaluminium oder Triethylaluminium, letztere insbesondere bei R* = Alkyl (vgl. EP-A-166 516) verwendet. Die jeweilige Base wird dabei beispielsweise im Bereich von 1 bis 3 Moläquivalenten, bezogen auf die Verbindung der Formel (II) eingesetzt.

Die Sulfonamide der Formel (II), die Verbindungen der Formeln (IV), (VI) und (VIII) sowie die nachfolgend beschriebenen Verbindungen der Formel (XIV) sind neue Verbindungen, die ebenso wie ihre Herstellung und ihre Verwendung zur Herstellung von Verbindungen der Formel (I) und/oder deren Salzen Gegenstand der vorliegenden Erfindung sind. Für die Verbindungen der Formel (II), (IV), (VI), (VIII) und (XIV) gelten für die Reste R und R¹ sowie den Index I die gleichen Vorzugsbereiche wie für die Verbindungen der Formel (I) angegeben.

Man kann die Verbindungen der Formel (II) z.B. erhalten wie in den nachfolgenden Schemata 1 bis 8 gezeigt.

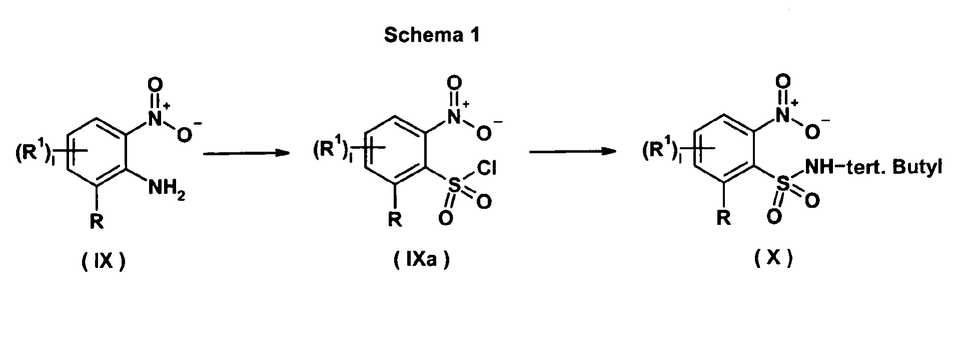

Ausgehend von kommerziell erhältlichen Verbindungen der Formel (IX) können, z.B. durch Diazotierung der Aminogruppe mit einem Alkalinitrit, wie z. B. Natriumnitrit, in Gegenwart von Salzsäure bei Temperaturen zwischen ―10°C und 10°C und nachfolgendem Austausch der resultierenden Diazogruppe z. B. mit Schwefeldioxid in Gegenwart eines Verdünnungsmittels, wie z. B. Dichlormethan, 1,2-Dichlorethan oder Essigsäure, und in Gegenwart eines Katalysators, wie z. B. Kupfer(I)chlorid und/oder Kupfer(II)chlorid, bei Temperaturen zwischen ―10°C und 50°C die Verbindungen der Formel (IXa) erhalten werden (vgl. Meerwein, Chem. Ber. 1957, 90, 841) (Schema 1).
Durch Behandlung von Sulfochloriden der Formel (IXa) mit tert.-Butylamin können Sulfonamide der Formel (X) erhalten werden. Die Sulfonamidbildung wird beispielsweise in inerten Lösungsmitteln wie z.B. Dichlormethan, Tetrahydrofuran (THF), Dioxan, Toluol oder Dimethylformamid (DMF) bei Temperaturen zwischen ― 70°C bis zum Siedepunkt des verwendeten Lösungsmittels, vorzugsweise bei 25°C durchgeführt. Dabei kommt bevorzugt eine Aminmenge von 1.5 - 2.5 Äquivalenten bezogen auf das verwendete Sulfochlorid zum Einsatz. 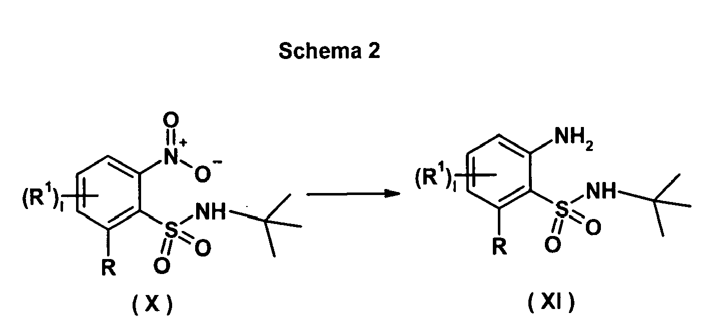 Die Reduktion der Nitroverbindungen (X) zu den Aminen der Formel (XI) erfolgt analog zu bekannten Methoden (vgl. hierzu Houben-Weyl, "Methoden der Organischen Chemie", 4. Aufl. Bd. XI/1 S. 360 ff., Thieme Verlag Stuttgart, 1957) (Schema 2). 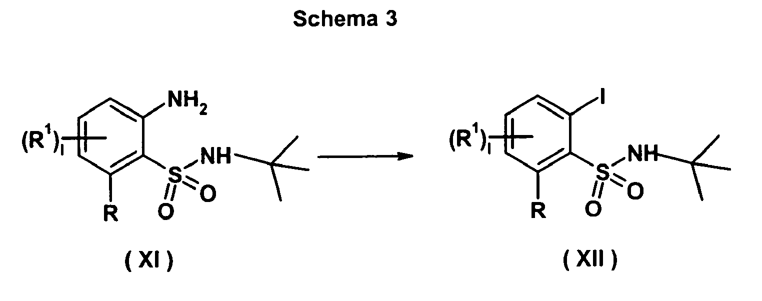 Der Verbindungen der Formel (XI) können unter üblichen Bedingungen für Diazotierungsreaktionen diazotiert und anschließend in Verbindungen der Formel (XII) überführt werden. Beispielsweise erfolgt die Diazotierung in Gegenwart der Säure H⁺X⁻, wobei X⁻ vorzugsweise Cl⁻, I⁻ oder HSO₄⁻ ist, in wässriger Lösung, gegebenenfalls unter Einsatz eines unter den Reaktionsbedingungen inerten organischen Lösungsmittels mit einem Nitrit. Beispielsweise diazotiert man mit einem Alkalimetallnitrit wie NaNO₂ (Natriumnitrit) in Mengen von 1.0 ― 1.2 Mol Nitrit, vorzugsweise 1,01 ― 1.05 Mol Nitrit, pro Mol der Verbindung der Formel (XI). Als Säuren eignen sich Mineralsäuren oder starke organische Säuren, bevorzugt sind Salzsäure, oder Schwefelsäure. Das Lösungsmittel ist Wasser oder eine Mischung aus Wasser und einem unter den Reaktionsbedingungen inerten organischen Lösungsmittel. Die Reaktionstemperatur beträgt in der Regel zwischen ―5°C und 50°C, vorzugsweise 10°C bis 20°C (Schema 3).
Die Umsetzung der erhaltenen Diazoniumsalze zu den Aryliodiden der Formel (XII) erfolgt in der Regel ohne Isolierung und wird im gleichen wässrigen oder wässrigorganischen Lösungsmittel oder Lösungsmittelgemisch wie die Diazotierung durchgeführt. Bei der Umsetzung wird die Diazoniumgruppe durch das lodatom ausgetauscht, entweder durch das Anion des Diazoniumsalzes (wenn in der Säure X⁻ = I⁻) oder (falls X⁻ nicht I⁻ ist) durch die Reaktion mit zugefügtem lodid, z. B. Alkalimetalliodid, vorzugsweise Natriumiodid oder Kaliumiodid. Die Menge an lodid beträgt dabei beispielsweise 1.1 bis 1.5 Mol lodid pro Mol der ursprünglich eingesetzten Verbindung der Formel (XI). Die Reaktionstemperatur beläuft sich dabei im allgemeinen auf 10°C bis 40°C, vorzugsweise 15°C bis 30°C (vgl. hierzu z.B. DE 19625831 und Bioorg. Med. Chem. 2004, 12, 2079) (Schema 3). 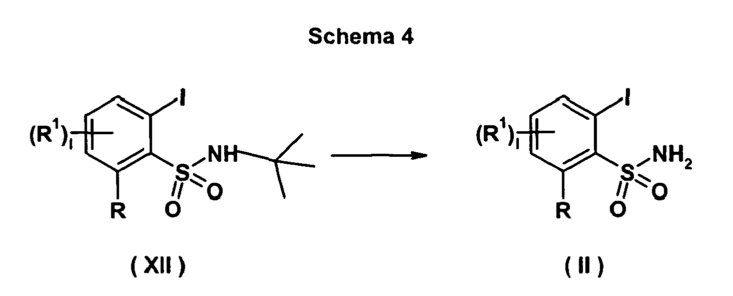 Die Abspaltung der tert.-Butyl-Schutzgruppe in den Verbindungen der Formel (XII) zu den Sulfonamiden der Formel (II) erfolgt z.B. durch Behandlung mit einer starken Säure (siehe WO 89/10921). Als starke Säuren kommen z.B. Mineralsäuren, wie H₂SO₄ oder HCl, oder starke organische Säuren, wie Trifluoressigsäure in Frage. Die Reaktion erfolgt beispielsweise bei Temperaturen von -20°C bis zur jeweiligen Rückflußtemperatur des Reaktionsgemisches, vorzugsweise bei 0°C bis 40°C. Die Umsetzung kann in Substanz oder auch in einem inerten Solvens, wie z.B. Dichlormethan oder Trichlormethan, durchgeführt werden (Schema 4). Einzelne Sulfonamide der Formel (II) sind bekannt. Eine Verbindung mit R = F-(CH₂)₂-O- und R = F-(CH₂)₃-O- ist bekannt aus WO 02/072560, mit R = NH₂- aus WO 93/21170, mit R = C₂H₅-O-CO-CO-NH- aus WO93/21171, und mit R = I aus HU 44481. 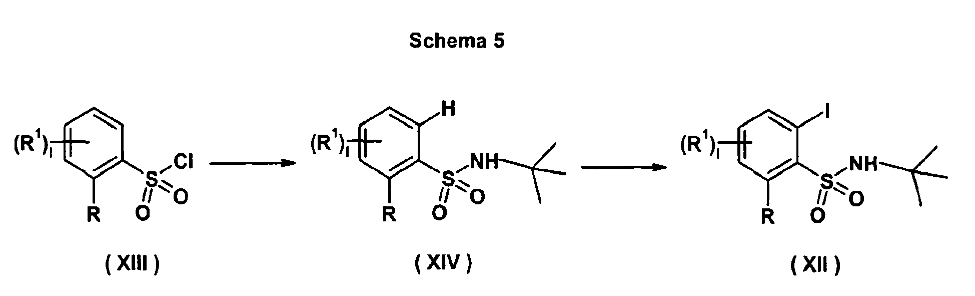 Substitituierte *t*-Butylaminosulfonylverbindungen der Formel (XII) können auch erhalten werden, indem man Verbindungen der Formel (XIV), welche durch Umsetzung von kommerziell erhältlichen Sulfochloriden der Formel (XIII) mit tert.-Butylamin (siehe Schema 1) erhalten werden können (Sulfochloride der Formel (XIII) können auch hergestellt werden durch Diazotierung der entsprechenden Aminoverbindungen und nachfolgende Sulfochlorierung wie in Schema 1) angegeben, mit einer metallorganischen Verbindung, wie z.B. Alkyl- oder Aryllithium, vorzugsweise n- oder *sec*-Butyllithium in Hexan, gegebenfalls in Gegenwart eines (weiteren) inerten Verdünnungsmittels, wie z.B. Tetrahydrofuran, und unter Inertgasatmosphäre, wie z.B. unter Argon oder Stickstoff, bei Temperaturen zwischen ―70°C und 20°C metalliert ― d. h. das in der Verbindung der Formel (XIV) in ortho-Position zur SO₂NH-tert. Butyl-Gruppe befindliche Wasserstoffatom durch ein Metallatom ersetzt ― und dann im gleichen Reaktionsmedium mit lod bei Temperaturen zwischen ―100°C und 40°C, vorzugsweise zwischen ―70°C und 20°C umsetzt ― d. h. das Metallatom durch lod ersetzt (Schema 5) (siehe hierzu auch: V. Snieckus et al., J. Org. Chem. 2001, 66, 3662 und Synlett 2000, (9), 1294). 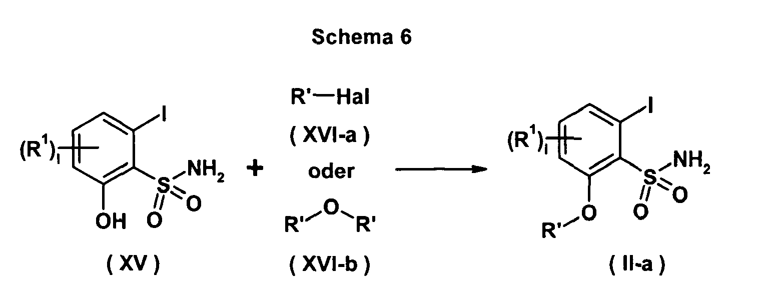 Spezielle Sulfonamide der allgemeinen Formel (II-a) mit R' = Kohlenwasserstoffrest wie Alkyl, Heterocyclylrest, CO-R³ oder S(O)ₙ-R³ können durch Reaktion von Hydroxybenzolsulfonamiden der Formel (XV) mit Verbindungen der allgemeinen Formel (XVI - a oder XVI - b) hergestellt werden, wobei ein oder mehrere Reaktionshilfsmittel verwendet werden können. Bei den in dieser Reaktion eingesetzten Verbindungen der Formel (XVI - a) steht der Rest R' z.B. für einen Kohlenwasserstoffrest wie Alkyl, einen Heterocyclylrest, CO-R³ oder S(O)ₙR³ und Hal steht für Halogen, wobei Alkyl, Halogen, n und R³ wie in Formel (I) definiert sind. Bei den Verbindungen der Formel (XVI - b) kann R' insbesondere CO-R³ oder S(O)ₙR³ sein. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetallverbindungen, wie z. B. - acetate, -amide, -carbonate, -hydrogencarbonate, -hydride, -hydroxide, -oder alkanoate ― im besonderen seien hier Kaliumcarbonat, Cäsiumcarbonat, Lithiumhydroxid, Natriumhydroxid und Natriumethanolat genannt ― weiterhin auch basische organische Stickstoffverbindungen, wie z. B. Triethylamin, Ethyl-diisopropylamin, alkylsubstituiertes Pyridine, 1,4-Diazabicyclo[2.2.2]octan (DABCO), 1,5-Diazabicyclo[4.3.0]non-5-en (DBN) oder 1,8-Diazabicyclo[5.4.0]undec-7en (DBU).
Als Lösungsmittel kommen neben Wasser vor allem inerte organische Lösungsmittel in Betracht. Insbesondere gehören hierzu Benzol, Toluol, Xylol, Dichlormethan, Chloroform, Diethylether, Dioxan, Tetrahydrofuran, Aceton, Acetonitril, N,N-Dimethylformamid, N-Methyl-pyrrolidon oder Essigsäureethylester. Die Reaktionstemperaturen variieren zwischen 0°C und der Rückflusstemperatur des verwendeten Lösungsmittel, vorzugsweise zwischen 10°C und 120°C (Schema 6) (vgl. hierzu auch WO 02/072560). 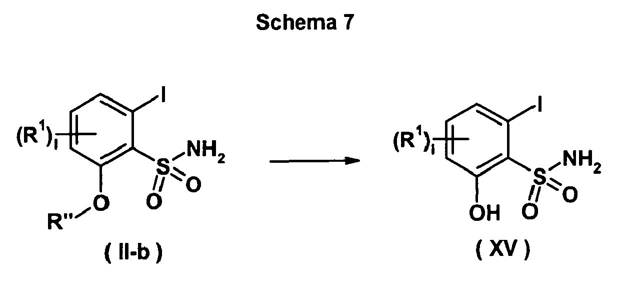 Hydroxybenzolsulfonamide der Formel (XV) können beispielsweise aus den orthoalkoxysubstituierten Benzolsulfonamiden der allgemeinen Formel (II-b) (erhältlich z.B. nach den Reaktionen der Schemata 1 - 6) erhalten werden, wobei R" insbesondere (C₁-C₄)Alkyl bedeuten kann. Hierzu kann in einem inerten Lösungsmittel wie Dichlormethan, Dichlorethan oder Chloroform, vorzugsweise Dichlormethan oder Dichlorethan, die Alkoxyverbindung der Formel (II-b) mit einer Lewis-Säure, vorzugsweise Bortrihalogeniden, wie BBr₃ behandelt werden. Die Reaktionstemperatur liegt im allgemeinen zwischen ―30°C und der Rückflusstemperatur des Lösungsmittel, vorzugsweise von 0°C bis 40°C (Schema 7) (siehe beispielsweise EP044807 und WO 97/03056). 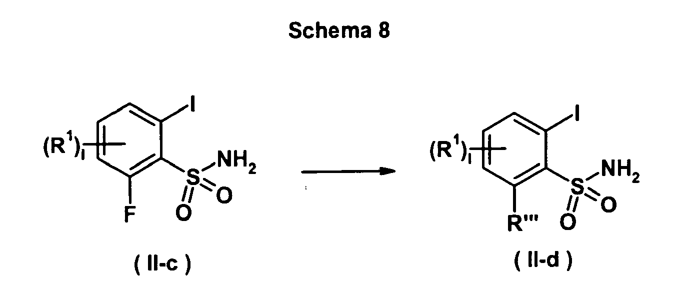 Benzolsulfonamide der allgemeinen Formel (II-d) können durch Austausch des Fluoratoms im ortho-Fluorbenzolsulfonamid der allgemeinen Formel (II-c) (erhältlich z.B. nach den Reaktionen der Schemata 1 - 6) durch Reaktion mit Nukleophilen der allgemeinen Formel R''' erhalten werden. R''' kann insbesondere Alkyloxy, Cycloalkoxy, Alkenyloxy, Alkinyloxy, Aryloxy, Heterocyclyloxy, Alkylthio, Alkenylthio, Alkinylthio, Arylthio, Heterocyclylthio, N(Alkyl)₂, NHAlkyl, N(Alkenyl)₂, NHAlkenyl, N(Alkinyl)₂, NHAlkinyl, NHAryl, NHHeterocyclyl, NH₂ sein, wobei alle genannten Reste (bis auf den letztgenannten) substituiert oder unsubstituiert sein können. Bei dieser Reaktion können auch ein oder mehrere Reaktionshilfsmittel zum Einsatz kommen wie die üblichen anorganischen oder organischen Basen oder Säureakzeptoren. Hierzu gehören vorzugsweise Alkalimetall- oder Erdalkalimetallverbindungen, wie z. B. -acetate, -amide, -carbonate, - hydrogencarbonate, -hydride, -hydroxide, -oder alkanoate ― im besonderen seien hier Kaliumcarbonat, Cäsiumcarbonat, Lithiumhydroxid, Natriumhydroxid und Natriumethanolat, und vor allem Natriumhydrid genannt ― weiterhin auch basische organische Stickstoffverbindungen, wie z. B. Triethylamin, Ethyl-diisopropylamin, alkylsubstituierte Pyridine, 1,4-Diazabicyclo[2.2.2]octan (DABCO), 1,5-Diazabicyclo[4.3.0]non-5-en (DBN) oder 1,8-Diazabicyclo[5.4.0]undec-7en (DBU). Als Lösungsmittel kommen neben Wasser vor allem inerte organische Lösungsmittel in Betracht. Insbesondere gehören hierzu Benzol, Toluol, Xylol, Dichlormethan, Chloroform, Diethylether, Dioxan, Tetrahydrofuran, Aceton, Acetonitril, N,N-Dimethylformamid, N-Methyl-pyrrolidon oder Essigsäureethylester, insbesonder seien hier Diethylether, Dioxan und Tetrahydrofuran hervorgehoben. Die Reaktionstemperatur beträgt im allgemeinen zwischen -20°C und Rückflusstemperatur des verwendeten Lösungsmittels, insbesondere zwischen 0°C und Rückflusstemperatur des verwendeten Lösungsmittel.

Neben der rein thermischen Reaktionsführung kann zur Reaktionsbeschleunigung auch Mikrowellenenergie eingesetzt werden. Hierfür kann ein kommerziell erhältliches, für chemische Zwecke ausgelegtes Mikrowellengerät eingesetzt werden. Die Reaktionen werden dabei im allgemeinen bei Temperaturen zwischen 20°C und 200°C, vorzugsweise zwischen 40°C und 170°C und mit einer Energieleistung zwischen 20 und 200 Watt, vorzugsweise zwischen 50 und 180 Watt, während einer Reaktionszeit zwischen 2 min und 60 min, vorzugsweise zwischen 5 min und 45 min durchgeführt.

Benzolsulfonamide der allgemeinen Formel (II-d) mit R''' = Alkylthio, Alkenylthio, Alkinylthio, Arylthio oder Heterocyclylthio können in Analogie zu literaturbekannten Reaktionen durch Behandlung mit Oxidationsmittel, vorzugsweise Metachlorperbenzoesäure, Wasserstoffperoxid, Natriummetaperiodat oder Oxone zu den entsprechenden Sulfoxiden oder Sulfonen umgewandelt werden (vgl. z.B. "Reactions of Organosulfur Compounds"; Academic Press, new York, 1978, S. 16).

Die Sulfonyl(thio)carbamate der allgemeinen Formel (IV) werden in Analogie zu an sich bekannten Reaktionen (vgl. EP-A-120 814) hergestellt. Man kann z.B. auch die Sulfonyliso(thio)cyanate der Formel (VI) in glatter Reaktion in einem inerten Lösungsmittel, vorzugsweise Diethylether oder Dichlormethan mit Phenol in die (Thio)Carbamate der Formel (IV) überführen. Die Aminoheterocyclen der Formel (V) sind bekannte, zum Teil im Handel erhältliche Synthesechemikalien.
Die Umsetzung der Sulfonyl(thio)carbamate der Formel (IV) mit den Aminoheterocyclen der Formel (V) erfolgt nach bekannten Verfahren (vgl. z.B. WO 2003 091228) (Schema 9).

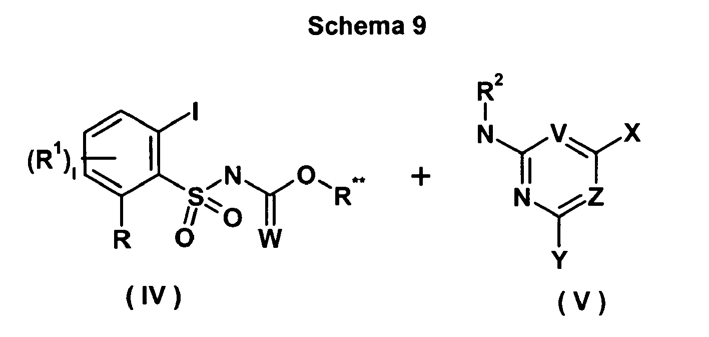

Die Sulfonyliso(thio)cyanate der allgemeinen Formel (VI) können nach an sich bekannten Verfahren aus den erfindungsgemäßen Sulfonamiden der allgemeinen Formel (II) hergestellt werden (vgl. DE 3208189, EP 23422, EP 64322, EP 44807, EP 216504). Man erhält die Arylsulfonyliso(thio)cyanate der Formel (VI), wenn man Arylsulfonamide der allgemeinen Formel (II) mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie Diazabicyclo [2.2.2] octan, und in Gegenwart eines Verdünnungsmitels, wie Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die Umsetzung der Arylsulfonyliso(thio)cyanate der allgemeinen Formel (VI) mit den Aminoheterocyclen der Formel (V) erfolgt z.B. nach bekannten Verfahren (vgl. WO 2003 091228) (Schema 10).

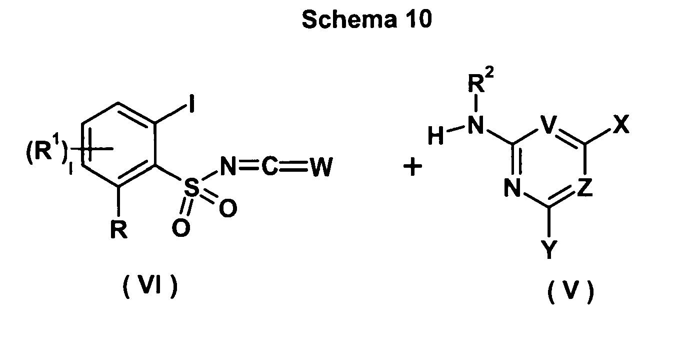

Die Iso(thio)cyanate der allgemeinen Formel (VII) erhält man z.B. aus den Aminoheterocyclen des Typs (V) durch Behandlung mit Oxalylchlorid oder (Thio)Phosphen (in Analogie nach Angew. Chem. 1971, 83, S. 407; EP 388 873). Die Umsetzung der Iso(thio)cyanate des Typs (VII) mit den Sulfonamiden der allgemeinen Formel (II) erfolgt z.B. in Analogie nach Variante c) (Schema 11).

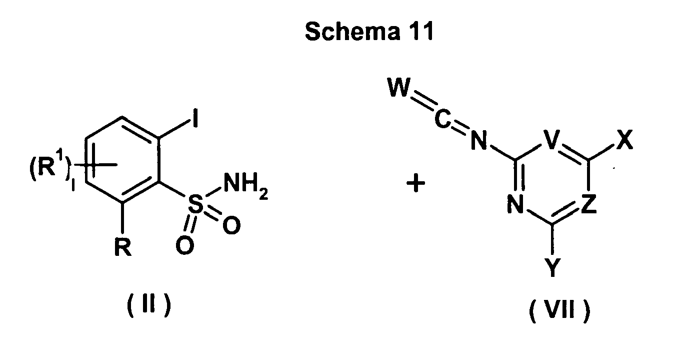

Die Sulfonsäurehalogenide der Formel (VIII) können nach verschiedenen literaturbekannten Methoden hergestellt werden, z.B. i) Oxidative Chlorierung von Thioether (Recl. Trav. Chim. Pays-Bas 1982, 101, 91, ii) Diazotierung von aromatischen Aminen mit Natriumnitrit in Salzsäure, gefolgt von der Reaktion des resultierenden Diazoniumsalzes mit Schwefeldioxid und Kupferchorid (J. Org. Chem. 1960, 1824), iii) Heteroatom- gesteuerte Lithiierung, gefolgt von Sulfonylierung (EP 73562; Org. React. 1979, 26, I), iv) Newman-Umlagerung und nachfolgende oxidative Chlorierung (US 5157119), v) Umsetzung eines Sulfonsäureamids des Typs (II) mit Thienylchlorid (Bull. Kor. Chem. Soc. 1994, 15, 323). Eine Verbindung der Formel (VIII) mit R = I ist bekannt aus FR 2649698.
In einer Ausführungsform der Variante f) wird die durch Umsetzung des Sulfonsäurehalogenids (VIII) mit einem (Thio)Cyanat erhaltene Reaktionsmischung direkt für die Kupplung mit einem Aminoheterocyclus der Formel (V) zur Synthese der Verbindung der Formel (I) eingesetzt (vgl. hierzu WO 2003 091228 und US 5550238).

Die Salze der Verbindungen der Formel (I) werden vorzugsweise in inerten polaren Lösungsmitteln wie z.B. Wasser, Methanol oder Aceton bei Temperaturen von 0°C bis 100°C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, oder Alkalialkoholate, wie Natriummethanolat oder Natrium-tert.-butylat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatorial Chemistry and Molecular Diversity: Automated Synthesis", Band 1, Verlag Escom 1997, Seite 69 bis 77 beschrieben ist.

Für die Durchführung mikrowellenunterstützter Synthesen kann ein Mikrowellengerät, z.B. Modell "Discover" der Firma CEM GmbH Mikrowellen-Analysentechnik, Carl-Friedrich-Gauß-Str. 9, 47475 Kamp-Lintfort verwendet werden.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, England, H+P Labortechnik GmbH, Bruckmannring 28, 85764 Oberschleißheim, Deutschland oder der Firma Radleys, Shirehill, Saffron Walden, Essex, CB 11 3AZ, England angeboten werden. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998.

Die Verwendung von Festphasen- unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) und/oder deren Salze, im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindungen auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Apera spica venti, Avena spp., Alopecurus spp., Brachiaria spp., Digitaria spp., Lolium spp., Echinochloa spp., Panicum spp., Phalaris spp., Poa spp., Setaria spp. sowie Bromus spp. wie Bromus catharticus, Bromus secalinus, Bromus erectus, Bromus tectorum und Bromus japonicus und Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B.

Abutilon spp., Amaranthus spp., Chenopodium spp., Chrysanthemum spp., Galium spp. wie Galium aparine, Ipomoea spp., Kochia spp., Lamium spp., Matricaria spp., Pharbitis spp., Polygonum spp.,Sida spp., Sinapis spp., Solanum spp., Stellaria spp., Veronica spp. und Viola spp., Xanthium spp., auf der annuellen Seite sowie Convolvulus, Cirsium, Rumex und Artemisia bei den perennierenden Unkräutern.

Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. zweikeimblättriger Kulturen wie Soja, Baumwolle, Raps, Zuckerrüben, oder Gramineen-Kulturen wie Weizen, Gerste, Roggen, Mais oder Reis, insbesondere Mais und Weizen, nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da die Lagerbarkeit hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044 und EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827 und WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924 und EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt (siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codierenden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.

Bei der Anwendung der erfindungsgemäßen Verbindungen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist somit auch die Verwendung der erfindungsgemäßen Verbindungen als Herbizide zur Bekämpfung von Schadpflanzen in transgenen und nicht transgenen Pflanzenkulturen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 13th edition, The British Crop Protection Council and the Royal Soc. of Chemistry, 2003 und dort zitierter Literatur beschrieben sind. Als bekannte Herbizide, die mit den erfindungsgemäßen Verbindungen kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet) und umfassen stets sämtliche Anwendungsformen wie Säuren, Salze, Ester und Isomere wie Stereoisomere und optische Isomere. Dabei sind eine und zum Teil auch mehrere Anwendungsformen genannt: 2,4-D, Acetochlor, Acifluorfen, Acifluorfen-sodium, Aclonifen, Alachlor, Alloxydim, Alloxydim-sodium, Ametryn, Amicarbazone, Amidosulfuron, Aminopyralid, Amitrole, Anilofos, Asulam, Atrazine, Azafenidin, Azimsulfuron, Beflubutamid, Benazolin, Benazolin-ethyl, Benfuresate, Bensulfuron-methyl, Bentazone, Benzfendizone, Benzobicyclon, Benzofenap, Bifenox, Bilanafos, Bispyribac-natrium, Bromacil, Bromobutide, Bromofenoxim, Bromoxynil, Butachlor, Butafenacil, Butenachlor, Butralin, Butroxydim, Butylate, Cafenstrole, Carbetamide, Carfentrazone-ethyl, Chlomethoxyfen, Chloridazon, Chlorimuron-ethyl, Chlornitrofen, Chlorotoluron, Chlorsulfuron, Cinidon-ethyl, Cinmethylin, Cinosulfuron, Clefoxydim, Clethodim, Clodinafop-propargyl, Clomazone, Clomeprop, Clopyralid, Cloransulam-methyl, Cumyluron, Cyanazine, Cyclosulfamuron, Cycloxydim, Cyhalofop-butyl, Desmedipham, Dicamba, Dichlobenil, Dichlorprop, Dichlorprop-P, Diclofop-methyl, Diclosulam, Difenzoquat, Diflufenican, Diflufenzopyr, Dikegulac-sodium, Dimefuron, Dimepiperate, Dimethachlor, Dimethametryn, Dimethenamid, Triaziflam, Diquatdibromide, Dithiopyr, Diuron, Dymron, EPTC, Esprocarb, Ethalfluralin, Ethametsulfuron-methyl, Ethofumesate, Ethoxyfen, Ethoxysulfuron, Etobenzanid, Fenoxaprop-ethyl, Fenoxaprop-P-ethyl, Fentrazamide, Flamprop-M-isopropyl, Flamprop-M-methyl, Flazasulfuron, Florasulam, Fluazifop, Fluazifop-butyl, Fluazifop-butyl, Fluazolate, Flucarbazone-sodium, Flucetosulfuron, Fluchloralin, Flufenacet, Flufenpyr, Flumetsulam, Flumiclorac-pentyl, Flumioxazin, Fluometuron, Fluorochloridone, Fluoroglycofen-ethyl, Flupoxam, Flupyrsulfuron-methyl-sodium, Fluridone, Fluroxypyr, Fluroxypyr-butoxypropyl, Fluroxypyr-meptyl, Flurprimidol, Flurtamone, Fluthiacet-methyl, Fomesafen, Foramsulfuron, Glufosinate, Glufosinate-ammonium, Glyphosate, Halosulfuron-methyl, Haloxyfop, Haloxyfop-ethoxyethyl, Haloxyfop-methyl, Haloxyfop-P-methyl, Hexazinone, Imazamethabenz-methyl, Imazamox, Imazapic, Imazapyr, Imazaquin, Imazethapyr, Imazosulfuron, Indanofan, lodosulfuron-methyl-natrium, loxynil, Isoproturon, Isouron, Isoxaben, Isoxachlortole, Isoxaflutole, Ketospiradox, Lactofen, Lenacil, Linuron, MCPA, Mecoprop, Mecoprop-P, Mefenacet, Mesosulfuron-methyl, Mesotrione, Metamifop, Metamitron, Metazachlor, Methabenzthiazuron, Methyldymron, Metobromuron, Metolachlor, Metosulam, Metoxuron, Metribuzin, Metsulfuron-methyl, Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonic acid, Pendimethalin, Pendralin, Penoxsulam, Pentoxazone, Pethoxamid, Phenmedipham, Picloram, Picolinafen, Pinoxaden, Piperophos, Pretilachlor, Primisulfuron-methyl, Profluazol, Profoxydim, Prometryn, Propachlor, Propanil, Propaquizafop, Propisochlor, Propoxycarbazone-sodium, Propyzamide, Prosulfocarb, Prosulfuron, Pyraclonil, Pyraflufen-ethyl, Pyrazolate, Pyrazosulfuronethyl, Pyrazoxyfen, Pyribenzoxim, Pyributicarb, Pyridafol, Pyridate, Pyriftalid, Pyriminobac-methyl, Pyrithiobac-sodium, Quinclorac, Quinmerac, Quinoclamine, Quizalofop-ethyl, Quizalofop-P-ethyl, Quizalofop-P-tefuryl, Rimsulfuron, Sethoxydim, Simazine, Simetryn, S-Metolachlor, Sulcotrione, Sulfentrazone, Sulfometuronmethyl, Sulfosate, Sulfosulfuron, Tebuthiuron, Tepraloxydim, Terbuthylazine, Terbutryn, Thenylchlor, Thiazopyr, Thifensulfuron-methyl, Thiobencarb, Tiocarbazil, Tralkoxydim, Triallate, Triasulfuron, Tribenuron-methyl, Triclopyr, Tridiphane, Trifloxysulfuron, Trifluralin, Triflusulfuron-methyl und Tritosulfuron.

Die erfindungsgemäßen Verbindungen können auch in Kombination mit einem oder mehreren als Safener wirkenden Verbindungen eingesetzt werden. Beispiele für Safener sind:
a) Verbindungen der Formeln (XVII) bis (XIX), 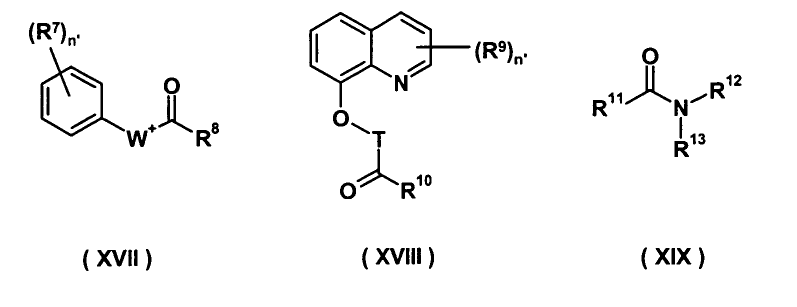 wobei die Symbole und Indizes folgende Bedeutungen haben:
   - n': ist eine natürliche Zahl von 0 bis 5, vorzugsweise 0 bis 3;
   - T: ist eine (C₁ oder C₂)Alkandiylkette, die unsubstituiert oder mit einem oder zwei (C₁-C₄)Alkylresten oder mit [(C₁-C₃)Alkoxy]carbonyl substituiert ist;
   - W⁺: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W⁺1) bis (W⁺4),

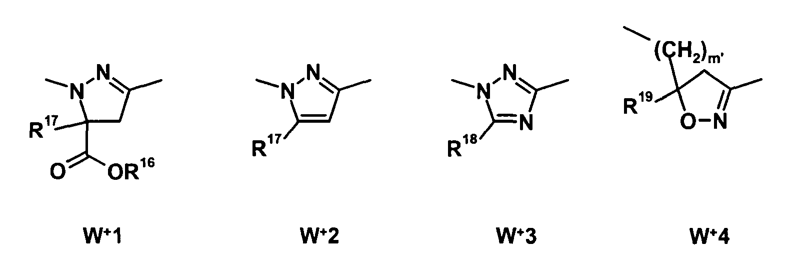

   - m': ist 0 oder 1;
   - R⁷, R⁹: sind gleich oder verschieden Wasserstoff, Halogen, (C₁-C₄)Alkyl, (C₁-C₄)Alkoxy, Nitro oder (C₁-C₄)Haloalkyl;
   - R⁸, R¹⁰: sind gleich oder verschieden OR¹⁴, SR¹⁴ oder NR¹⁴R¹⁵ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (XVII) bzw. (XVIII) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR¹⁴, NHR¹⁵ oder N(CH₃)₂, insbesondere der Formel OR¹⁴;
   - R¹⁴: ist Wasserstoff oder ein unsubstituierter oder substituierter aliphatischer Kohlenwasserstoffrest, vorzugsweise mit insgesamt 1 bis 18 C-Atomen;
   - R¹⁵: ist Wasserstoff, (C₁-C₆)Alkyl, (C₁-C₆)Alkoxy oder substituiertes oder unsubstituiertes Phenyl;
   - R¹⁶: ist Wasserstoff, (C₁-C₈)Alkyl, (C₁-C₈)Haloalkyl, (C₁-C₄)Alkoxy-( C₁-C₄)alkyl, (C₁-C₄)Hydroxyalkyl, (C₃-C₁₂)Cycloalkyl oder Tri- (C₁-C₄)alkylsilyl;
   - R¹⁷, R¹⁸, R¹⁹: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   - R¹¹: ist (C₁-C₄)Alkyl, (C₁-C₄)Haloalkyl, (C₁-C₄)Alkenyl, (C₂-C₄)Haloalkenyl, (C₃-C₇)Cycloalkyl, vorzugsweise Dichlormethyl;
   - R¹², R¹³: ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-( C₁-C₄)alkyl, Thiazolyl, Furyl, Furylalkyl, Thienyl, Piperidyl, substituiertes oder unsubstituiertes Phenyl, oder R¹² und R¹³ bilden zusammen einen substituierten oder unsubstituierten heterocyclischen Ring, vorzugsweise einen Oxazolidin-, Thiazolidin-, Piperidin-, Morpholin-, Dihydropyrimidin- oder Benzoxazinring;
   oder
b) eine oder mehreren Verbindungen aus Gruppe:
   1,8-Naphthalsäureanhydrid,
   Methyl-diphenylmethoxyacetat,
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole),
   2-Dichlormethyl-2-methyl-1,3-dioxolan (MG-191),
   N-(4-Methylphenyl)-N'-(1-methyl-1-phenylethyl)harnstoff (Dymron),
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   1-[4-(N-Naphthoylsulfamoyl)phenyl]-3,3-dimethylharnstoff,
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl 3,6-dichlor-2-methoxybenzoat (Lactidichlor)
      sowie deren Salze und Ester, vorzugsweise (C₁-C₈)-Ester;
c) N-Acylsulfonamide der Formel (XX) und ihre Salze, 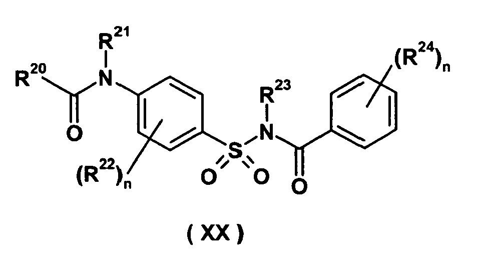 worin
   - R²⁰: Wasserstoff, einen Kohlenwasserstoffrest, einen Kohlenwasserstoffoxyrest, einen Kohlenwasserstoffthiorest oder einen Heterocyclylrest, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
   wobei jeder Kohlenwasserstoffteil vorzugsweise 1 bis 20 C-Atome aufweist und ein C-haltiger Rest R²⁰ inklusive Substituenten vorzugsweise 1 bis 30 C-Atome aufweist;
   - R²¹: Wasserstoff oder (C₁-C₄)Alkyl, vorzugsweise Wasserstoff, oder
   - R²⁰ und R²¹: zusammen mit der Gruppe der Formel -CO-N- den Rest eines 3- bis 8-gliedrigen gesättigten oder ungesättigten Rings;
   - R²²: gleich oder verschieden Halogen, Cyano, Nitro, Amino, Hydroxy, Carboxy, Formyl, CONH₂, SO₂NH₂ oder einen Rest der Formel -Z^{b}-R^{b} ;
   - R²³: Wasserstoff oder (C₁-C₄)Alkyl, vorzugsweise H;
   - R²⁴: gleich oder verschieden Halogen, Cyano, Nitro, Amino, Hydroxy, Carboxy, CHO, CONH₂, SO₂NH₂ oder einen Rest der Formel -Z^{c}-R^{c} ;
   - R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)alkyl]amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b}, R^{c}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)alkoxy, Mono- und Di-[(C₁-C₄)alkyl]amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a}: eine divalente Gruppe der Formel -O-, -S-, -CO-, -CS-, -CO-O-, -CO-S-, -O-CO-, -S-CO-, -SO-, -SO₂-, -NR⁺-, -CO-NR⁺-, -NR⁺-CO-, -SO₂-NR⁺- oder -NR⁺-SO₂-, wobei die rechts angegebene Bindung der jeweiligen divalenten Gruppe die Bindung zum Rest R^{a} ist und wobei die R⁺ in den letztgenannten 5 Resten unabhängig voneinander jeweils H, (C₁-C₄)Alkyl oder Halo-(C₁-C₄)-alkyl bedeuten;
   - Z^{b}, Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR⁺-, -SO₂-NR⁺-, -NR⁺-SO₂-, -CO-NR⁺- oder -NR⁺-CO-, wobei die rechts angegebene Bindung der jeweiligen divalenten Gruppe die Bindung zum Rest R^{b} bzw. R^{c} ist und wobei die R⁺ in den letztgenannten 5 Resten unabhängig voneinander jeweils H, (C₁-C₄)Alkyl oder Halo-(C₁-C₄)alkyl bedeuten;
   - n: eine ganze Zahl von 0 bis 4, vorzugsweise 0, 1 oder 2, insbesondere 0 oder 1, und
   - m: eine ganze Zahl von 0 bis 5, vorzugsweise 0, 1, 2 oder 3, insbesondere 0, 1 oder 2; bedeuten;
d) Acylsulfamoylbenzoesäureamide der allgemeinen Formel (XXI), gegebenenfalls auch in Salzform, 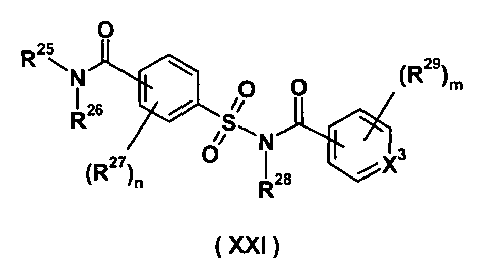 worin
   - X³: CH oder N;
   - R²⁵: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   - R²⁶: Wasserstoff, Hydroxy, (C₁-C₆)Alkyl, (C₂-C₆)Alkenyl, (C₂-C₆)Alkinyl, (C₁-C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁-C₄)Alkylthio substituiert sind, oder

   - R²⁵ und R²⁶: zusammen mit dem sie tragenden Stickstoffatom einen 3- bis 8-gliedrigen gesättigten oder ungesättigten Ring;
   - R²⁷: Halogen, Cyano, Nitro, Amino, Hydroxy, Carboxy, CHO, CONH₂, SO₂NH₂ oder Z^{b}-R^{b};
   - R²⁸: Wasserstoff, (C₁-C₄)Alkyl, (C₂-C₄)Alkenyl oder (C₂-C₄)Alkinyl;
   - R²⁹: Halogen, Cyano, Nitro, Amino, Hydroxy, Carboxy, Phosphoryl, CHO, CONH₂, SO₂NH₂ oder Z^{c}-R^{c};
   - R^{a}: einen (C₂-C₂₀)Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)alkyl]amino substituiert sind;
   - R^{b}, R^{c}: gleich oder verschieden einen (C₂-C₂₀)Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)alkyl]amino substituiert sind;
   - Z^{a}: eine divalente Einheit aus der Gruppe O, S, CO, CS, C(O)O, C(O)S, SO, SO₂, NR^{d}, C(O)NR^{d} oder SO₂NR^{d};
   - Z^{b}, Z^{c}: gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d};
   - R^{d}: Wasserstoff, (C₁-C₄)Alkyl oder (C₁-C₄)Haloalkyl,
   - n: eine ganze Zahl von 0 bis 4, und
   - m: für den Fall, daß X für CH steht, eine ganze Zahl von 0 bis 5, und für den Fall, daß X für N steht, eine ganze Zahl von 0 bis 4
   bedeuten;
e) Verbindungen der Formel (XXII), 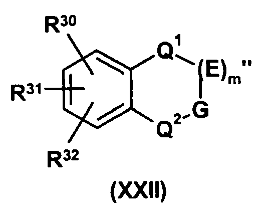 worin die Symbole und Indizes folgende Bedeutungen haben:
   - R³⁰: ist H, (C₁-C₄)Alkyl, (C₁-C₄)Alkyl substituiert mit (C₁-C₄)Alkyl-X⁴ oder (C₁-C₄)Haloalkyl-X⁴, (C₁-C₄)Haloalkyl, NO₂, CN, -COO-R³³, NR₂³⁴, SO₂NR₂³⁵ oder CONR₂³⁶;
   - R³¹: ist H, Halogen, (C₁-C₄)Alkyl, CF₃, (C₁-C₄)Alkoxy oder (C₁-C₄)Haloalkoxy;
   - R³²: ist H, Halogen oder (C₁-C₄)Alkyl;
   - Q¹, Q², E, G: sind gleich oder verschieden, -O-, -S-, -CR₂³⁷-, -CO-, NR³⁸- oder eine Gruppe der Formel (XXIII), 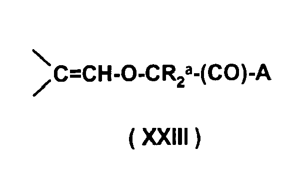 mit der Maßgabe, daß
   a) mindestens eine der Gruppen Q¹, Q², E, G eine Carbonylgruppe ist, daß genau eine dieser Gruppe ein Rest der Formel (XXIII) ist und daß die Gruppe der Formel (XXIII) einer Carbonylgruppe benachbart ist, und
   b) zwei benachbarte Gruppen Q¹, Q², E und G nicht gleichzeitig Sauerstoff sein können;
   - R^{a}: ist gleich oder verschieden H oder (C₁-C₈)Alkyl oder die beiden Reste R^{a} zusammen sind (C₂-C₆)Alkylen;
   - A: ist R^{b}―Y³- oder ―NR₂³⁹;
   - X⁴: ist -O- oder ―S(O)ₚ-;
   - Y³: ist -O- oder ―S-;
   - R^{b}: ist H, (C₁-C₈)Alkyl, (C₁-C₈)Haloalkyl, (C₁-C₄)Alkoxy-(C₁-C₈)alkyl, (C₃-C₆)-Alkenyloxy-(C₁-C₈)alkyl, oder Phenyl-(C₁-C₈)alkyl, wobei der Phenylring gegebenenfalls durch Halogen, (C₁-C₄)Alkyl, CF₃, Methoxy oder Methyl-S(O)ₚ substituiert ist; (C₃-C₆)Alkenyl, (C₃-C₆)Haloalkenyl, Phenyl-( C₃-C₆)alkenyl, (C₃-C₆)Alkinyl, Phenyl-( C₃-C₆)alkinyl, Oxetanyl, Furfuryl, Tetrahydrofuryl;
   - R³³: ist H oder (C₁-C₄)Alkyl;
   - R³⁴: ist gleich oder verschieden H, (C₁-C₄)Alkyl, (C₁-C₄)Alkylcarbonyl oder die beiden Reste R³⁴ zusammen sind (C₄-C₅)Alkylen;
   - R³⁵, R³⁶: sind unabhängig voneinander jeweils gleich oder verschieden H, (C₁-C₄)Alkyl, oder die beiden Reste R³⁵ und/oder R³⁶ zusammen sind (C₄-C₅)Alkylen, wobei eine CH₂-Gruppe durch O oder S oder eine oder zwei CH₂-Gruppen durch -NR^{c}- ersetzt sein können;
   - R^{c}: ist H oder (C₁-C₈)Alkyl;
   - R³⁷: ist gleich oder verschieden H, (C₁-C₈)Alkyl oder die beiden Reste R³⁷ zusammen sind (C₂-C₆)Alkylen;
   - R³⁸: ist H, (C₁-C₈)Alkyl, substituiertes oder unsubstituiertes Phenyl, oder unsubstituiertes oder am Phenylring substituiertes Benzyl;
   - R³⁹: ist gleich oder verschieden H, (C₁-C₈)Alkyl, Phenyl, Phenyl-(C₁-C₈)alkyl, wobei ein Phenylring durch F, Cl, Br, NO₂, CN, OCH₃, (C₁-C₄)Alkyl oder CH₃SO₂- substituiert sein kann; (C₁-C₄)Alkoxy-(C₁-C₈)alkyl, (C₃-C₆)Alkenyl, (C₃-C₆)Alkinyl, (C₃-C₆)Cycloalkyl oder zwei Reste R³⁹ zusammen sind (C₄-C₅)Alkylen, wobei eine CH₂-Gruppe durch O oder S oder eine oder zwei CH₂-Gruppen durch ―NR^{d}― ersetzt sein können;

   - R^{d}: ist H oder (C₁-C₄)Alkyl;
   - m": ist 0 oder 1 und
   - p: ist 0, 1 oder 2;
einschließlich der Stereoisomeren und der in der Landwirtschaft gebräuchlichen Salze.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der erfindungsgemäßen Verbindungen. Sie kann innerhalb weiter Grenzen schwanken, z.B. zwischen 0.001 und 10.0 kg/ha oder mehr Aktivsubstanz, vorzugsweise liegt sie zwischen 0.005 und 5 kg/ha.

### Beispiele

### A. Synthesebeispiele

### Beispiel A1

### N-(tert-Butyl)-2-methoxybenzolsulfonamid

Zu einer Lösung von 30.00 g (145.17 mmol) 2-Methoxybenzolsulfonsäurechlorid in 150 ml Dichlormethan werden bei 5-10°C 22.30 g (304.87 mmol) *tert*.-Butylamin zugetropft. Danach lässt man 2 h bei Raumtemperatur rühren. Nach Extraktion mit Wasser wird die organische Phase über Natriumsulfat getrocknet und zur Trockne eingedampft. Es werden 31.10 g (88 % d. Th.) N-(*tert*-Butyl)-2-methoxybenzol-sulfonamid erhalten.
¹H-NMR (CDCl₃): 7.91 (dd, J = 1.7, 7.8, 1 H); 7.50 (m, 1 H); 7.03 (m, 2H); 4.93 (br s, 1 H); 3.98 (s, 3H); 1.17 (s, 9H).

### Beispiel A2

### N-(tert-Butyl)-2-iodo-6-mefhoxybenzolsulfonamid (Beispiel-Nr. 2.0926)

Eine Lösung von 30.00 g (123.29 mmol) N-(*tert*-Butyl)-2-methoxybenzolsulfonamid in 400 ml Tetrahydrofuran wird auf -70°C abgekühlt und langsam mit einer Lösung von 110.96 ml (277.41 mmol) einer 2.5 molaren *n*-Butyllithium-Lösung in THF versetzt. Danach erwärmt man die Lösung kurz auf -30°C und kühlt danach wieder auf -60°C. Bei dieser Temperatur wird eine Lösung von 31.29 g (123.29 mmol) lod in 200 ml Tetrahydrofuran zugetropft. Anschließend wird die Reaktionslösung über Nacht bei Raumtemperatur gerührt. Nach Extraktion mit Wasser wird die organische Phase über Natriumsulfat getrocknet und zur Trockne eingedampft. Man erhält 42.40 g (93 % d. Th.) N-(*tert*-Butyl)-2-iodo-6-methoxybenzolsulfonamid.

### Beispiel A3

### 2-Iodo-6-methoxybenzolsulfonamid (Beispiel-Nr. 2.092a)

42.40 g (114.84 mmol) N-(*tert*-Butyl)-2-iodo-6-methoxybenzolsulfonamid werden in 265 ml Trifluoressigsäure 3 h bei Raumtemperatur gerührt. Danach gießt man die Reaktionsmischung auf Eiswasser, filtriert den Niederschlag ab und wäscht mit Wasser neutral. Man erhält 32.40 g (90 % d. Th.) 2-lodo-6-methoxybenzol-sulfonamid.

### Beispiel A4

### N-{[(4,6-Dimethoxypyrimidin-2-yl)amino]carbonyl}-2-iodo-6-methoxybenzolsulfonamid (Beispiel-Nr. 1.146)

Eine Lösung von 200 mg (0.64 mmol) 2-lodo-6-methoxybenzolsulfonamid in 3 ml Acetonitril wird bei Raumtemperatur zuerst mit 316.49 mg (1.15 mmol) N-(4,6-Dimethoxypyrimidin-2-yl)carbaminsäurephenylester und danach langsam mit 0.19 ml (1.28 mmol) 1,8-Diazabicyclo[5.4.0]undec-7-en versetzt. Nach 30 min Rühren bei Raumtemperatur wird die Lösung mittels 2 N Salzlösung langsam auf pH 1 eingestellt. Der ausgefallene Feststoff wird abgesaugt, mit Wasser nachgewaschen und getrocknet. Man erhält so 242 mg (77 % d. Th.) N-{[(4,6-Dimethoxypyrimidin-2-yl)amino]carbonyl}-2-iodo-6-methoxybenzolsulfonamid.

### Beispiel A5

### 2-Hydroxy-6-iodobenzolsulfonamid (Beispiel-Nr. 2.300a)

0.50 g (1.60 mmol) 2-lodo-6-methoxybenzolsulfonamid werden in 10 ml Dichlormethan bei Raumtemperatur vorgelegt und vorsichtig mit 0.6 g (2.40 mmol) Bortribromid versetzt. Die Reaktionslösung rührt weitere 45 min bei Raumtemperatur und wird dann auf 2 N Salzsäure gegeben. Nach Extraktion mit Dichlormethan wird die organische Phase getrocknet und eingedampft. Man erhält 0.43 g (90 % d. Th.) 2-Hydroxy-6-iodobenzolsulfonamid.

### Beispiel A6

### 2-Iodo-6-propoxybenzolsulfonamid (Beispiel-Nr. 2.095a)

5.00 g (16.72 mmol) 2-Hydroxy-6-iodobenzolsulfonamid werden in 50 ml Dimethylformamid vorgelegt und mit 2,54 g (18.39 mmol) Kaliumcarbonat versetzt. Diese Mischung wird 1 h bei Raumtemperatur gerührt. Danach werden 3.13 g (18.39 mmol) Propyliodid zugetropft und die Reaktionsmischung wird 3 h bei Raumtemperatur gerührt. Dann wird auf Wasser gegossen, wobei das Produkt ausfiel. Der Feststoff wird mit Wasser nachgewaschen und getrocknet. Man erhält 4.00 g (70 % d. Th.) 2-lodo-6-propoxybenzolsulfonamid.

### Beispiel A7

### N-(tert-Butyl)-2-fluoro-6-iodobenzolsulfonamid (Beispiel-Nr. 2.001 b)

30 g (0.13 mol) (N-*tert*-Butyl)-2-fluorobenzolsulfonamid, erhalten aus der Umsetzung von 2-Fluorobenzolsulfonsäurechlorid mit N-tert-Butylamin analog Beispiel A1, werden in 300 ml trockenem Tetrahydrofuran vorgelegt. Die Lösung wird auf ―70°C abgekühlt und eine Lösung von 18.28 g (0.285 mol) n-Butyllithium (2.5 molar in Tetrahydrofuran) wird zugetropft. Danach wird die Reaktionslösung während 30 min auf ―30°C erwärmt um dann wieder auf ―70°C abgekühlt zu werden. Dann werden 36.21 g (0.143 mol) lod in 200 ml trockenem Tetrahydrofuran zugetropft. Nach der Zugabe wird die Reaktionslösung langsam auf Raumtemperatur erwärmt und 12 h gerührt. Danach wird mit 50%iger wässriger Natriumthiosulfat-Lösung und Wasser gewaschen. Die organische Phase wird getrocknet und eingedampft. Man erhält 40.8 g (88 % d. Th.) N-(*tert*-Butyl)-2-fluoro-6-iodobenzolsulfonamid.

### Beispiel A8

### 2-(2,2-Difluoroethoxy)-6-iodobenzolsulfonamid (Beispiel-Nr. 2.187a)

0.64 g (26.57 mmol) Natriumhydrid werden in 10 ml trockenem Tetrahydrofuran vorgelegt und bei Raumtemperatur langsam mit 2.18 g (26.57 mmol) 2,2-Difluoroethanol versetzt. Die Reaktionsmischung wird bis zum Ende der Gasentwicklung bei Raumtemperatur gerührt. Danach werden 4.00 g (13.29 mmol) 2-Fluor-6-iodo-benzolsulfonamid, erhalten aus der Reaktion von N-(*tert*-Butyl)-2-fluoro-6-iodobenzolsulfonamid mit Trifluoressigsäure analog Beispiel A3, gelöst in 20 ml trockenem Tetrahydrofuran zugetropft. Diese Reaktionsmischung wird 30 min bei 150°C 100 Watt Mikrowellenenergie ausgesetzt. Danach wird mit 2 N Salzsäure auf ph 4-5 gestellt und in Wasser/Ethylacetat verteilt, die organische Phase wird getrocknet und eingedampft. Man erhält 3.00 g (62 % d. Th.) 2-(2,2-Difluoroethoxy)-6-iodobenzolsulfonamid.

### Beispiel A9

### 2-Iodo-6-(methylthio)benzolsulfonamid (Beispiel-Nr. 2.208a)

30.00 g (99.64 mmol) 2-Fluoro-6-iodobenzolsulfonamid, erhalten aus der Reaktion von N-(*tert*-Butyl)-2-fluoro-6-iodobenzolsulfonamid mit Trifluoressigsäure analog Beispiel A3, werden zusammen mit 15.15 g (109.61 mmol) Kaliumcarbonat in 250 ml Dimethylformamid vorgelegt. Bei Raumtemperatur werden 7.68 g (109.61 mmol) Natriumthiomethylat portionsweise zugegeben, wonach 12 h bei Raumtemperatur gerührt wird. Die Reaktionsmischung wird auf 150 ml Eiswasser gegossen, mit 2 N Salzsäure auf pH 4-5 gestellt und mit Ethylacetat extrahiert. Die organische Phase wird getrocknet und eingedampft. Präparative HPLC (Reversed phase, 0.05 % Trifluoressigsäure in Wasser / Acetonitril, Gradient: in 30 min, 25 bis 100 % Acetonitril) ergeben 7.40 g (23 % d. Th.) 2-lodo-6-(methylthio)benzolsulfonamid.

Die in den nachfolgenden Tabellen 1 und 2 beschriebenen Verbindungen werden analog den obigen Beispielen A1 ― A9 erhalten.

Abkürzungen in den nachfolgenden Tabellen 1 und 2:
- * =: ¹H-NMR Daten sind nachfolgend zu den Tabellen 1 bzw. 2 aufgeführt
- Me =: Methyl
- Ph =: Phenyl
- Het =: Heterocyclus, wobei Het für einen der nachfolgenden Reste H1 bis H23 steht

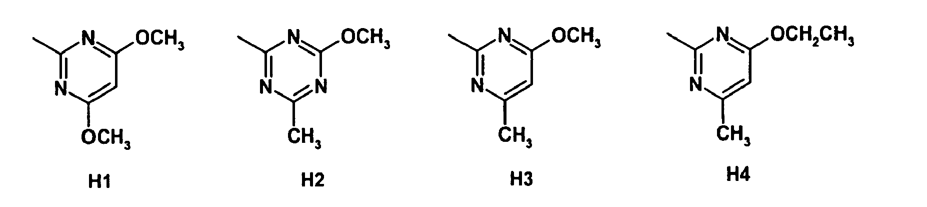

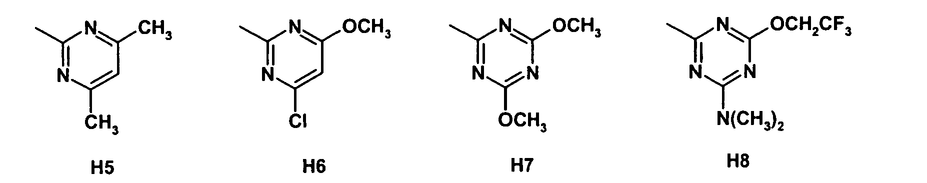

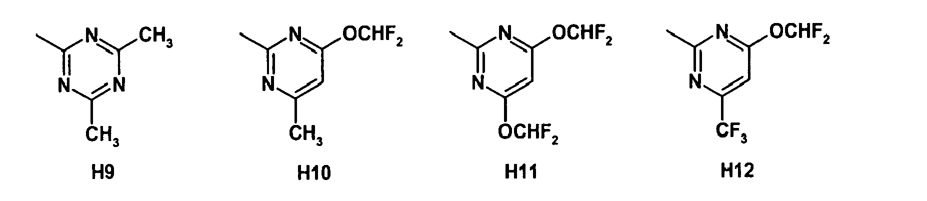

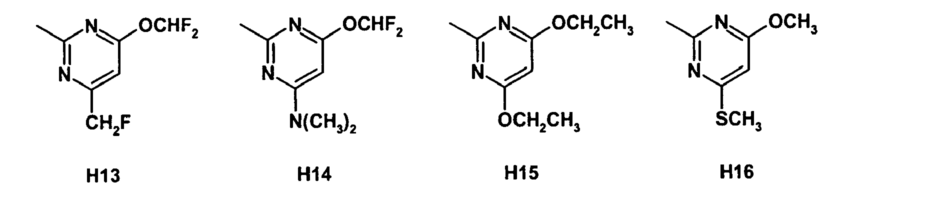

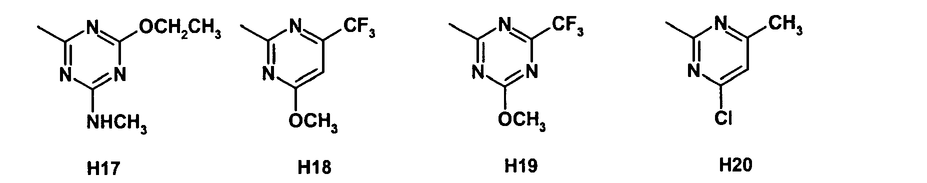

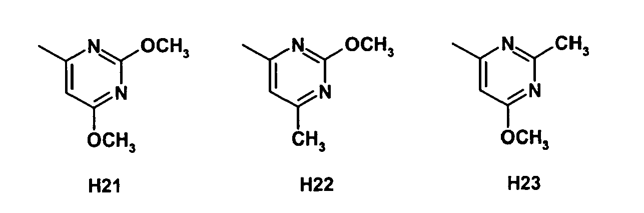

**Tabelle 1: Verbindungen der allgemeinen Formel (I-a)**

| | | | | | | |
|---|---|---|---|---|---|---|
| 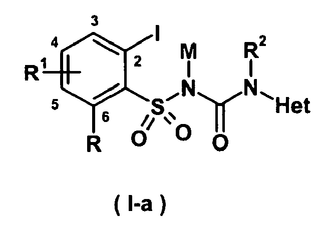 | | | | | | |

| | R | R¹ | R² | M | Het | ¹H-NMR |
|---|---|---|---|---|---|---|
| | | | | | | |
| 1.001 | F | - | H | H | H1 | * |
| 1.002 | F | - | H | H | H2 | * |
| 1.003 | F | - | H | H | H5 | * |
| 1.004 | F | - | H | H | H6 | * |
| 1.005 | F | - | H | H | H7 | |
| 1.006 | Br | - | H | H | H1 | |
| 1.007 | Br | - | H | H | H2 | |
| 1.008 | Br | - | H | H | H6 | |
| 1.009 | I | - | H | H | H1 | |
| 1.010 | I | - | H | H | H2 | |
| 1.011 | I | - | H | H | H6 | |
| 1.012 | CH₃ | - | H | H | H1 | * |
| 1.013 | CH₃ | - | H | Na | H1 | |
| 1.014 | CH₃ | 5-CH₃ | H | H | H1 | |
| 1.015 | CH₃ | - | CH₃ | H | H1 | |
| 1.016 | CH₃ | - | H | H | H2 | * |
| 1.017 | CH₃ | - | H | Na | H2 | |
| 1.018 | CH₃ | 5-CH₃ | H | H | H2 | |
| 1.019 | CH₃ | - | CH₃ | H | H2 | |
| 1.020 | CH₃ | - | H | H | H5 | * |
| 1.021 | CH₃ | - | H | H | H6 | * |
| 1.022 | CH₃ | - | H | Na | H6 | |
| 1.023 | CH₃ | 5-CH₃ | H | H | H6 | |
| 1.024 | CH₃ | - | CH₃ | H | H6 | |
| 1.025 | CH₃ | - | H | H | H7 | * |
| 1.026 | CH₂CH₃ | - | H | H | H1 | |
| 1.027 | CH₂CH₃ | - | H | H | H2 | |
| 1.028 | (CH₂)₂CH₃ | - | H | H | H1 | |
| 1.029 | (CH₂)₂CH₃ | - | H | H | H2 | |
| 1.030 | CH(CH₃)₂ | - | H | H | H1 | |
| 1.031 | CH(CH₃)₂ | - | H | H | H2 | |
| 1.032 | (CH₂)₃CH₃ | - | H | H | H1 | |
| 1.033 | CH(CH₃)CH₂CH₃ | - | H | H | H1 | |
| 1.034 | CH₂CH(CH₃)₂ | - | H | H | H1 | |
| 1.035 | C(CH₃)₃ | - | H | H | H1 | |
| 1.036 | CH=CH₂ | - | H | H | H1 | |
| 1.037 | CH=CH₂ | - | H | H | H2 | |
| 1.038 | C(CH₃)=CH₂ | - | H | H | H1 | |
| 1.039 | C≡CH | - | H | H | H1 | |
| 1.040 | C≡CH | - | H | H | H2 | |
| 1.041 | C≡CCH₃ | - | H | H | H1 | |
| 1.042 | C≡CCH₂CH₃ | - | H | H | H1 | |
| 1.043 | CH₂CH=CH₂ | - | H | H | H1 | |
| 1.044 | CH₂C(CH₃)=CH₂ | - | H | H | H1 | |
| 1.045 | CH₂C≡CH | - | H | H | H1 | |
| 1.046 | CH₂C≡CCH₃ | - | H | H | H1 | |
| 1.047 | CH₂C≡CCH₂CH₃ | - | H | H | H1 | |
| 1.048 | cyclopropyl | - | H | H | H1 | |
| 1.049 | cyclopropyl | - | H | H | H2 | |
| 1.050 | 2,2-di-F-cyclopropyl | - | H | H | H1 | |
| 1.051 | 2,2-di-F-cyclopropyl | - | H | H | H2 | |
| 1.052 | 2,2-di-Cl-cyclopropyl | - | H | H | H1 | |
| 1.053 | 2,2-di-CH₃-cyclopropyl | - | H | H | H1 | |
| 1.054 | cyclobutyl | - | H | H | H1 | |
| 1.055 | cyclopentyl | - | H | H | H1 | |
| 1.056 | cyclohexyl | - | H | H | H1 | |
| 1.057 | CH₂cyclopropyl | - | H | H | H1 | |
| 1.058 | CH₂cyclobutyl | - | H | H | H1 | |
| 1.059 | CH₂cyclopentyl | - | H | H | H1 | |
| 1.060 | CH₂cyclohexyl | - | H | H | H1 | |
| 1.061 | CH₂OCH₃ | - | H | H | H1 | |
| 1.062 | CH₂OCH₂CH₃ | - | H | H | H1 | |
| 1.063 | CH(CH₃)OCH₃ | - | H | H | H1 | |
| 1.064 | Ph | - | H | H | H1 | |
| 1.065 | Ph | - | H | H | H2 | |
| 1.066 | 2-F-Ph | - | H | H | H1 | |
| 1.067 | 3-F-Ph | - | H | H | H1 | |
| 1.068 | 4-F-Ph | - | H | H | H1 | |
| 1.069 | 2,6-di-F-Ph | - | H | H | H1 | |
| 1.070 | 2,4-di-F-Ph | - | H | H | H1 | |
| 1.071 | 2-Cl-Ph | - | H | H | H1 | |
| 1.072 | 3-Cl-Ph | - | H | H | H1 | |
| 1.073 | 4-Cl-Ph | - | H | H | H1 | |
| 1.074 | 2,6-di-Cl-Ph | | H | H | H1 | |
| 1.075 | 2,4-di-Cl-Ph | - | H | H | H1 | |
| 1.076 | 2-MeO-Ph | - | H | H | H1 | |
| 1.077 | 3-MeO-Ph | - | H | H | H1 | |
| 1.078 | 4-MeO-Ph | - | H | H | H1 | |
| 1.079 | 2,4-di-MeO-Ph | - | H | H | H1 | |
| 1.080 | 2-Me-Ph | - | H | H | H1 | |
| 1.081 | 3-Me-Ph | - | H | H | H1 | |
| 1.082 | 4-Me-Ph | - | H | H | H1 | |
| 1.083 | 2-CF₃-Ph | - | H | H | H1 | |
| 1.084 | 3-CF₃-Ph | - | H | H | H1 | |
| 1.085 | 4-CF₃-Ph | - | H | H | H1 | |
| 1.086 | CH₂Ph | - | H | H | H1 | |
| 1.087 | CH₂-2-F-Ph | - | H | H | H1 | |
| 1.088 | CH₂-2,4-di-F-Ph | - | H | H | H1 | |
| 1.089 | CH₂-2-MeO-Ph | - | H | H | H1 | |
| 1.090 | CH₂-3-MeO-Ph | - | H | H | H1 | |
| 1.091 | CF₃ | - | H | H | H1 | * |
| 1.092 | CF₃ | - | H | Na | H1 | |
| 1.093 | CF₃ | 5-CH₃ | H | H | H1 | |
| 1.094 | CF₃ | - | CH₃ | H | H1 | |
| 1.095 | CF₃ | - | H | H | H2 | * |
| 1.096 | CF₃ | - | H | Na | H2 | |
| 1.097 | CF₃ | 5-CH₃ | H | H | H2 | |
| 1.098 | CF₃ | - | CH₃ | H | H2 | |
| 1.099 | CF₃ | - | H | H | H5 | * |
| 1.100 | CF₃ | - | H | H | H6 | * |
| 1.101 | CF₃ | - | H | Na | H6 | |
| 1.102 | CF₃ | 5-CH₃ | H | H | H6 | |
| 1.103 | CF₃ | - | CH₃ | H | H6 | |
| 1.104 | CF₃ | - | H | H | H7 | * |
| 1.105 | CF₃ | - | H | H | H10 | |
| 1.106 | CF₃ | - | H | H | H11 | |
| 1.107 | CF₃ | - | H | H | H12 | |
| 1.108 | CF₃ | - | H | H | H13 | |
| 1.109 | CHF₂ | - | H | H | H1 | |
| 1.110 | CHF₂ | - | H | H | H2 | |
| 1.111 | CHF₂ | - | H | H | H6 | |
| 1.112 | CH₂F | - | H | H | H1 | |
| 1.113 | CH₂CF₃ | - | H | H | H1 | |
| 1.114 | CH₂CHF₂ | - | H | H | H1 | |
| 1.115 | CH₂CH₂F | - | H | H | H1 | |
| 1.116 | CF=CH₂ | - | H | H | H1 | |
| 1.117 | CH=CF₂ | - | H | H | H1 | |
| 1.118 | CF₂CH=CH₂ | - | H | H | H1 | |
| 1.119 | CH=CH-CF₃ | - | H | H | H1 | |
| 1.120 | CHFCH=CH₂ | - | H | H | H1 | |
| 1.121 | CN | - | H | H | H1 | |
| 1.122 | CN | - | H | H | H2 | |
| 1.123 | NO₂ | - | H | H | H1 | |
| 1.124 | NH₂ | - | H | H | H1 | |
| 1.125 | NHCH₃ | - | H | H | H1 | |
| 1.126 | N(CH₃)₂ | - | H | H | H1 | |
| 1.127 | N(CH₃)CH₂CH=CH₂ | - | H | H | H1 | |
| 1.128 | N(CH₃)CH₂C≡CH | - | H | H | H1 | |
| 1.129 | NH-cyclopropyl | - | H | H | H1 | |
| 1.130 | N(CH₃)-cyclopropyl | - | H | H | H1 | |
| 1.131 | N(CH₂CH₃)-cyclopropyl | - | H | H | H1 | |
| 1.132 | NHC(O)H | - | H | H | H1 | |
| 1.133 | NHC(O)H | - | H | H | H2 | |
| 1.134 | NHC(O)CH₃ | | H | H | H1 | |
| 1.135 | NHC(O)CH₃ | - | H | H | H2 | |
| 1.136 | NHC(O)OCH₃ | - | H | H | H1 | |
| 1.137 | NHC(O)OCH₃ | - | H | H | H2 | |
| 1.138 | NHSO₂CH₃ | - | H | H | H1 | |
| 1.139 | NHSO₂CH₃ | - | H | H | H2 | |
| 1.140 | NHSO₂CF₃ | - | H | H | H1 | |
| 1.141 | NHSO₂CF₃ | - | H | H | H2 | |
| 1.142 | NHSO₂CHF₂ | - | H | H | H1 | |
| 1.143 | NHSO₂CHF₂ | - | H | H | H2 | |
| 1.144 | NHSO₂CH₂F | - | H | H | H1 | |
| 1.145 | OH | - | H | H | H1 | |
| 1.146 | OCH₃ | - | H | H | H1 | * |
| 1.147 | OCH₃ | - | H | Na | H1 | |
| 1.148 | OCH₃ | 5-CH₃ | H | H | H1 | |
| 1.149 | OCH₃ | - | CH₃ | H | H1 | |
| 1.150 | OCH₃ | - | H | H | H2 | * |
| 1.151 | OCH₃ | - | H | Na | H2 | |
| 1.152 | OCH₃ | 5-CH₃ | H | H | H2 | |
| 1.153 | OCH₃ | - | CH₃ | H | H2 | |
| 1.154 | OCH₃ | - | H | H | H5 | * |
| 1.155 | OCH₃ | - | H | H | H6 | * |
| 1.156 | OCH₃ | - | H | Na | H6 | |
| 1.157 | OCH₃ | 5-CH₃ | H | H | H6 | |
| 1.158 | OCH₃ | - | CH₃ | H | H6 | |
| 1.159 | OCH₃ | - | H | H | H7 | * |
| 1.160 | OCH₃ | - | H | H | H10 | |
| 1.161 | OCH₃ | - | H | H | H11 | |
| 1.162 | OCH₃ | - | H | H | H12 | |
| 1.163 | OCH₃ | - | H | H | H13 | |
| 1.164 | OCH₂CH₃ | - | H | H | H1 | * |
| 1.165 | OCH₂CH₃ | - | H | H | H2 | * |
| 1.166 | OCH₂CH₃ | - | H | H | H5 | * |
| 1.167 | OCH₂CH₃ | - | H | H | H6 | * |
| 1.168 | OCH₂CH₃ | - | H | H | H7 | * |
| 1.169 | OCH₂CH₃ | - | H | H | H10 | |
| 1.170 | OCH₂CH₃ | - | H | H | H11 | |
| 1.171 | O(CH₂)₂CH₃ | - | H | H | H1 | * |
| 1.172 | O(CH₂)₂CH₃ | - | H | H | H2 | |
| 1.173 | O(CH₂)₂CH₃ | - | H | H | H5 | * |
| 1.174 | O(CH₂)₂CH₃ | - | H | H | H6 | * |
| 1.175 | O(CH₂)₂CH₃ | - | H | H | H7 | * |
| 1.176 | O(CH₂)₂CH₃ | - | H | H | H10 | |
| 1.177 | O(CH₂)₂CH₃ | - | H | H | H11 | |
| 1.178 | OCH(CH₃)₂ | - | H | H | H1 | * |
| 1.179 | OCH(CH₃)₂ | - | H | Na | H1 | |
| 1.180 | OCH(CH₃)₂ | 5-CH₃ | H | H | H1 | |
| 1.181 | OCH(CH₃)₂ | - | CH₃ | H | H1 | |
| 1.182 | OCH(CH₃)₂ | - | H | H | H2 | * |
| 1.183 | OCH(CH₃)₂ | - | H | Na | H2 | |
| 1.184 | OCH(CH₃)₂ | 5-CH₃ | H | H | H2 | |
| 1.185 | OCH(CH₃)₂ | - | CH₃ | H | H2 | |
| 1.186 | OCH(CH₃)₂ | - | H | H | H5 | * |
| 1.187 | OCH(CH₃)₂ | - | H | H | H6 | * |
| 1.188 | OCH(CH₃)₂ | - | H | Na | H6 | |
| 1.189 | OCH(CH₃)₂ | 5-CH₃ | H | H | H6 | |
| 1.190 | OCH(CH₃)₂ | - | CH₃ | H | H6 | |
| 1.191 | OCH(CH₃)₂ | - | H | H | H7 | * |
| 1.192 | O(CH₂)₃CH₃ | - | H | H | H1 | |
| 1.193 | O(CH₂)₃CH₃ | - | H | H | H10 | |
| 1.194 | O(CH₂)₃CH₃ | - | H | H | H11 | |
| 1.195 | O(CH₂)₃CH₃ | - | H | H | H12 | |
| 1.196 | OCH(CH₃)CH₂CH₃ | - | H | H | H1 | |
| 1.197 | OCH₂CH(CH₃)₂ | - | H | H | H1 | |
| 1.198 | OC(CH₃)₃ | - | H | H | H1 | |
| 1.199 | OC(CH₃)₃ | - | H | H | H2 | |
| 1.200 | OCH=CH₂ | - | H | H | H1 | |
| 1.201 | OC(CH₃)=CH₂ | - | H | H | H1 | |
| 1.202 | OCH=CH(CH₃) | - | H | H | H1 | |
| 1.203 | OCH=C(CH₃)₂ | - | H | H | H1 | |
| 1.204 | OC(CH₃)=CHCH₃ | - | H | H | H1 | |
| 1.205 | OC(CH₃)=C(CH₃)₂ | - | H | H | H1 | |
| 1.206 | OC≡CH | - | H | H | H1 | |
| 1.207 | OC≡CCH₃ | - | H | H | H1 | |
| 1.208 | OC≡CCH₂CH₃ | - | H | H | H1 | |
| 1.209 | OCH₂CH=CH₂ | - | H | H | H1 | |
| 1.210 | OCH₂CH=CH₂ | - | H | H | H2 | |
| 1.211 | OCH₂C(CH₃)=CH₂ | - | H | H | H1 | |
| 1.212 | OCH₂CH=CHCH₃ | - | H | H | H1 | |
| 1.213 | OCH₂CH=C(CH₃)₂ | - | H | H | H1 | |
| 1.214 | OCH₂C(CH₃)=CHCH₃ | - | H | H | H1 | |
| 1.215 | OCH₂C(CH₃)=C(CH₃)₂ | - | H | H | H1 | |
| 1.216 | OCH(CH₃)CH=CH₂ | - | H | H | H1 | |
| 1.217 | OCH₂C≡CH | - | H | H | H1 | |
| 1.218 | OCH₂C≡CH | - | H | H | H2 | |
| 1.219 | OCH₂C≡CCH₃ | - | H | H | H1 | |
| 1.220 | OCH₂C≡CCH₂CH₃ | - | H | H | H1 | |
| 1.221 | OCH(CH₃)C≡CH | - | H | H | H1 | |
| 1.222 | O-cyclopropyl | - | H | H | H1 | |
| 1.223 | O-cyclopropyl | - | H | H | H2 | |
| 1.224 | O-2,2-di-Cl-cyclopropyl | - | H | H | H1 | |
| 1.225 | O-2,2-di-F-cyclopropyl | - | H | H | H1 | |
| 1.226 | O-cyclobutyl | - | H | H | H1 | |
| 1.227 | O-cyclopentyl | - | H | H | H1 | |
| 1.228 | O-cyclohexyl | - | H | H | H1 | |
| 1.229 | OCH₂-cyclopropyl | - | H | H | H1 | * |
| 1.230 | OCH₂-cyclopropyl | - | H | Na | H1 | |
| 1.231 | OCH₂-cyclopropyl | 5-CH₃ | H | H | H1 | |
| 1.232 | OCH₂-cyclopropyl | - | CH₃ | H | H1 | |
| 1.233 | OCH₂-cyclopropyl | - | H | H | H2 | * |
| 1.234 | OCH₂-cyclopropyl | - | H | Na | H2 | |
| 1.235 | OCH₂-cyclopropyl | 5-CH₃ | H | H | H2 | |
| 1.236 | OCH₂-cyclopropyl | - | CH₃ | H | H2 | |
| 1.237 | OCH₂-cyclopropyl | - | H | H | H5 | * |
| 1.238 | OCH₂-cyclopropyl | - | H | H | H6 | * |
| 1.239 | OCH₂-cyclopropyl | - | H | Na | H6 | |
| 1.240 | OCH₂-cyclopropyl | 5-CH₃ | H | H | H6 | |
| 1.241 | OCH₂-cyclopropyl | - | CH₃ | H | H6 | |
| 1.242 | OCH₂-cyclopropyl | - | H | H | H7 | * |
| 1.243 | OCH₂-cyclopropyl | - | H | H | H10 | |
| 1.244 | OCH₂-cyclopropyl | - | H | H | H11 | |
| 1.245 | OCH₂-cyclopropyl | - | H | H | H12 | |
| 1.246 | OCH₂-cyclopropyl | - | H | H | H13 | |
| 1.247 | OCH(CH₃)-cyclopropyl | - | H | H | H1 | |
| 1.248 | OCH(CH₃)-cyclopropyl | - | H | H | H2 | |
| 1.249 | OCH₂-2-Me-cyclopropyl | - | H | H | H1 | |
| 1.250 | OCH₂-2,2-di-Me-cyclopropyl | - | H | H | H1 | |
| 1.251 | OCH₂-2,2-di-Cl-cyclopropyl | - | H | H | H1 | |
| 1.252 | OCH₂-2,2-di-F-cyclopropyl | - | H | H | H1 | |
| 1.253 | OCH₂-cyclobutyl | - | H | H | H1 | |
| 1.254 | OCH₂ cyclopentyl | - | H | H | H1 | |
| 1.255 | OCH(CH₃)-cyclopentyl | - | H | H | H1 | |
| 1.256 | OCH₂-cyclohexyl | - | H | H | H1 | |
| 1.257 | OCH(CH₃)-cyclohexyl | - | H | H | H1 | |
| 1.258 | OCH₂OCH₃ | - | H | H | H1 | |
| 1.259 | O(CH₂)₂OCH₃ | - | H | H | H1 | |
| 1.260 | OCH₂OCH₂CH₃ | - | H | H | H1 | |
| 1.261 | O(CH₂)₂OCH₂CH₃ | - | H | H | H1 | |
| 1.262 | OCH(CH₃)OCH₃ | - | H | H | H1 | |
| 1.263 | OPh | - | H | H | H1 | |
| 1.264 | OPh | - | H | H | H2 | |
| 1.265 | O-2-F-Ph | - | H | H | H1 | |
| 1.266 | O-3-F-Ph | - | H | H | H1 | |
| 1.267 | O-4-F-Ph | - | H | H | H1 | |
| 1.268 | O-2,6-di-F-Ph | - | H | H | H1 | |
| 1.269 | O-2,4-di-F-Ph | - | H | H | H1 | |
| 1.270 | O-2-Cl-Ph | - | H | H | H1 | |
| 1.271 | O-3-Cl-Ph | - | H | H | H1 | |
| 1.272 | O-4-Cl-Ph | - | H | H | H1 | |
| 1.273 | O-2,6-di-Cl-Ph | - | H | H | H1 | |
| 1.274 | O-2,4-di-Cl-Ph | - | H | H | H1 | |
| 1.275 | O-2-CF₃-Ph | - | H | H | H1 | |
| 1.276 | O-3-CF₃-Ph | - | H | H | H1 | |
| 1.277 | O-4-CF₃-Ph | - | H | H | H1 | |
| 1.278 | O-2-MeO-Ph | - | H | H | H1 | |
| 1.279 | O-3-MeO-Ph | - | H | H | H1 | |
| 1.280 | O-4-MeO-Ph | - | H | H | H1 | |
| 1.281 | O-2,4-di-MeO-Ph | - | H | H | H1 | |
| 1.282 | O-2-Me-Ph | - | H | H | H1 | |
| 1.283 | O-3-Me-Ph | - | H | H | H1 | |
| 1.284 | O-4-Me-Ph | | H | H | H1 | |
| 1.285 | OCH₂Ph | - | H | H | H1 | |
| 1.286 | OCH₂Ph | - | H | H | H2 | |
| 1.287 | OCH(CH₃)Ph | - | H | H | H1 | |
| 1.288 | OCH₂-2-F-Ph | - | H | H | H1 | |
| 1.289 | OCH₂-3-F-Ph | - | H | H | H1 | |
| 1.290 | OCH₂-4-F-Ph | - | H | H | H1 | |
| 1.291 | OCH₂-2,4-di-F-Ph | - | H | H | H1 | |
| 1.292 | OCH₂-2-Cl-Ph | - | H | H | H1 | |
| 1.293 | OCH₂-3-Cl-Ph | - | H | H | H1 | |
| 1.294 | OCH₂-4-Cl-Ph | - | H | H | H1 | |
| 1.295 | OCH₂-2,4-di-Cl-Ph | - | H | H | H1 | |
| 1.296 | OCH₂-2-MeO-Ph | - | H | H | H1 | |
| 1.297 | OCH₂-3-MeO-Ph | - | H | H | H1 | |
| 1.298 | OCH₂-4-MeO-Ph | - | H | H | H1 | |
| 1.299 | OCH₂-2-CF₃-Ph | - | H | H | H1 | |
| 1.300 | OCH₂-3-CF₃-Ph | - | H | H | H1 | |
| 1.301 | OCH₂-4-CF₃-Ph | - | H | H | H1 | |
| 1.302 | OCF₃ | - | H | H | H1 | * |
| 1.303 | OCF₃ | - | H | Na | H1 | |
| 1.304 | OCF₃ | 5-CH₃ | H | H | H1 | |
| 1.305 | OCF₃ | - | CH₃ | H | H1 | |
| 1.306 | OCF₃ | - | H | H | H2 | * |
| 1.307 | OCF₃ | - | H | Na | H2 | |
| 1.308 | OCF₃ | 5-CH₃ | H | H | H2 | |
| 1.309 | OCF₃ | - | CH₃ | H | H2 | |
| 1.310 | OCF₃ | - | H | H | H5 | * |
| 1.311 | OCF₃ | - | H | H | H6 | * |
| 1.312 | OCF₃ | - | H | Na | H6 | |
| 1.313 | OCF₃ | 5-CH₃ | H | H | H6 | |
| 1.314 | OCF₃ | - | CH₃ | H | H6 | |
| 1.315 | OCF₃ | - | H | H | H7 | * |
| 1.316 | OCF₃ | - | H | H | H3 | |
| 1.317 | OCF₃ | - | H | H | H10 | |
| 1.318 | OCF₃ | - | H | H | H11 | |
| 1.319 | OCF₃ | - | H | H | H12 | |
| 1.320 | OCF₃ | - | H | H | H13 | |
| 1.321 | OCHF₂ | - | H | H | H1 | * |
| 1.322 | OCHF₂ | - | H | Na | H1 | |
| 1.323 | OCHF₂ | 5-CH₃ | H | H | H1 | |
| 1.324 | OCHF₂ | - | CH₃ | H | H1 | |
| 1.325 | OCHF₂ | - | H | H | H2 | * |
| 1.326 | OCHF₂ | - | H | Na | H2 | |
| 1.327 | OCHF₂ | 5-CH₃ | H | H | H2 | |
| 1.328 | OCHF₂ | - | CH₃ | H | H2 | |
| 1.329 | OCHF₂ | - | H | H | H5 | * |
| 1.330 | OCHF₂ | - | H | H | H6 | * |
| 1.331 | OCHF₂ | - | H | Na | H6 | |
| 1.332 | OCHF₂ | 5-CH₃ | H | H | H6 | |
| 1.333 | OCHF₂ | - | CH₃ | H | H6 | |
| 1.334 | OCHF₂ | - | H | H | H7 | |
| 1.335 | OCH₂F | - | H | H | H1 | |
| 1.336 | OCH₂F | - | H | H | H3 | |
| 1.337 | OCH₂F | - | H | H | H10 | |
| 1.338 | OCH₂F | - | H | H | H11 | |
| 1.339 | OCH₂F | - | H | H | H12 | |
| 1.340 | OCH₂F | - | H | H | H13 | |
| 1.341 | OCH₂CF₃ | - | H | H | H1 | |
| 1.342 | OCH₂CF₃ | - | H | H | H2 | * |
| 1.343 | OCH₂CF₃ | - | H | H | H5 | * |
| 1.344 | OCH₂CF₃ | | H | H | H6 | * |
| 1.345 | OCH₂CF₃ | - | H | H | H7 | |
| 1.346 | OCH₂CF₃ | - | H | H | H3 | |
| 1.347 | OCH₂CF₃ | - | H | H | H10 | |
| 1.348 | OCH₂CF₃ | - | H | H | H11 | |
| 1.349 | OCH₂CF₃ | - | H | H | H12 | |
| 1.350 | OCH₂CF₃ | - | H | H | H13 | |
| 1.351 | OCH₂CHF₂ | - | H | H | H1 | |
| 1.352 | OCH₂CHF₂ | - | H | H | H2 | * |
| 1.353 | OCH₂CHF₂ | - | H | H | H5 | * |
| 1.354 | OCH₂CHF₂ | - | H | H | H6 | * |
| 1.355 | OCH₂CHF₂ | - | H | H | H7 | * |
| 1.356 | OCH₂CH₂F | - | H | H | H1 | |
| 1.357 | OCH₂CH₂F | - | H | H | H3 | |
| 1.358 | OCH₂CH₂F | - | H | H | H10 | |
| 1.359 | OCH₂CH₂F | - | H | H | H11 | |
| 1.360 | OCH₂CH₂F | - | H | H | H12 | |
| 1.361 | OCH₂CH₂F | - | H | H | H13 | |
| 1.362 | OCH(CH₃)CF₃ | - | H | H | H1 | * |
| 1.363 | OCH(CH₃)CF₃ | - | H | H | H2 | * |
| 1.364 | OCH(CH₃)CF₃ | - | H | H | H5 | * |
| 1.365 | OCH(CH₃)CF₃ | - | H | H | H6 | * |
| 1.366 | OCH(CH₃)CF₃ | - | H | H | H7 | * |
| 1.367 | OCH(CH₃)CHF₂ | - | H | H | H1 | |
| 1.368 | OCH(CH₃)CH₂F | - | H | H | H1 | |
| 1.369 | OCH₂CF₂CF₃ | - | H | H | H1 | |
| 1.370 | OCH₂CF₂CF₃ | - | H | H | H2 | * |
| 1.371 | OCH₂CF₂CF₃ | - | H | H | H5 | * |
| 1.372 | OCH₂CF₂CF₃ | - | H | H | H6 | * |
| 1.373 | OCH₂CF₂CF₃ | - | H | H | H7 | * |
| 1.374 | OCH₂CF₂CHF₂ | - | H | H | H1 | |
| 1.375 | OCH₂CF₂CH₂F | - | H | H | H1 | |
| 1.376 | OCH(CH₃)CF₂CF₃ | - | H | H | H1 | |
| 1.377 | OCH(CH₃)CF₂CHF₂ | - | H | H | H1 | |
| 1.378 | OCH(CH₃)CF₂CH₂F | - | H | H | H1 | |
| 1.379 | OCH₂CHFCF₃ | - | H | H | H1 | |
| 1.380 | O(CH₂)₂CF₃ | - | H | H | H1 | |
| 1.381 | O(CH₂)₂CHF₂ | - | H | H | H1 | |
| 1.382 | O(CH₂)₃CF₃ | - | H | H | H1 | |
| 1.383 | O(CH₂)₃CHF₂ | - | H | H | H1 | |
| 1.384 | OCF=CH₂ | - | H | H | H1 | |
| 1.385 | OCH=CF₂ | - | H | H | H1 | |
| 1.386 | OCF₂CH=CH₂ | - | H | H | H1 | |
| 1.387 | OCHFCH=CH₂ | - | H | H | H1 | |
| 1.388 | OCH=CHCF₃ | - | H | H | H1 | |
| 1.389 | SCH₃ | - | H | H | H1 | * |
| 1.390 | SCH₃ | - | H | H | H2 | * |
| 1.391 | SCH₂CH₃ | - | H | H | H1 | |
| 1.392 | SCH₂CH₃ | - | H | H | H2 | |
| 1.393 | S(CH₂)₂CH₃ | - | H | H | H1 | |
| 1.394 | SCH(CH₃)₂ | - | H | H | H1 | |
| 1.395 | SCH(CH₃)₂ | - | H | H | H2 | |
| 1.396 | SC(CH₃)₃ | - | H | H | H1 | |
| 1.397 | SCH₂Ph | - | H | H | H1 | |
| 1.398 | SPh | - | H | H | H1 | |
| 1.399 | SCF₃ | - | H | H | H1 | |
| 1.400 | SCF₃ | - | H | H | H2 | |
| 1.401 | SCHF₂ | - | H | H | H1 | |
| 1.402 | SCHF₂ | - | H | H | H2 | |
| 1.403 | SCH₂F | - | H | H | H1 | |
| 1.404 | SCH=CH₂ | - | H | H | H1 | |
| 1.405 | SCH₂CH=CH₂ | - | H | H | H1 | |
| 1.406 | SCH₂CH=CH₂ | - | H | H | H2 | |
| 1.407 | SC≡CH | - | H | H | H1 | |
| 1.408 | SCH₂C≡CH | - | H | H | H1 | |
| 1.409 | SCH₂C≡CH | - | H | H | H2 | |
| 1.410 | S-cyclopropyl | - | H | H | H1 | |
| 1.411 | SCH₂-cyclopropyl | - | H | H | H1 | |
| 1.412 | SCH₂-cyclopropyl | - | H | H | H2 | |
| 1.413 | SF₅ | - | H | H | H1 | |
| 1.414 | S(O)CH₃ | - | H | H | H1 | |
| 1.415 | S(O)CH₂CH₃ | - | H | H | H1 | |
| 1.416 | S(O)(CH₂)₂CH₃ | - | H | H | H1 | |
| 1.417 | S(O)CH(CH₃)₂ | - | H | H | H1 | |
| 1.418 | S(O)C(CH₃)₃ | - | H | H | H1 | |
| 1.419 | S(O)CH₂Ph | - | H | H | H1 | |
| 1.420 | S(O)Ph | - | H | H | H1 | |
| 1.421 | S(O)CF₃ | - | H | H | H1 | |
| 1.422 | S(O)CHF₂ | - | H | H | H1 | |
| 1.423 | S(O)CH₂F | - | H | H | H1 | |
| 1.424 | S(O)CH=CH₂ | - | H | H | H1 | |
| 1.425 | S(O)CH₂CH=CH₂ | - | H | H | H1 | |
| 1.426 | S(O)C≡CH | - | H | H | H1 | |
| 1.427 | S(O)CH₂C≡CH | - | H | H | H1 | |
| 1.428 | S(O)-cyclopropyl | - | H | H | H1 | |
| 1.429 | S(O)CH₂-cyclopropyl | - | H | H | H1 | |
| 1.430 | SO₂CH₃ | - | H | H | H1 | |
| 1.431 | SO₂CH₃ | - | H | H | H2 | |
| 1.432 | SO₂CH₂CH₃ | - | H | H | H1 | |
| 1.433 | SO₂CH₂CH₃ | - | H | H | H2 | |
| 1.434 | SO₂(CH₂)₂CH₃ | - | H | H | H1 | |
| 1.435 | SO₂CH(CH₃)₂ | - | H | H | H1 | |
| 1.436 | SO₂CH(CH₃)₂ | - | H | H | H2 | |
| 1.437 | SO₂C(CH₃)₃ | - | H | H | H1 | |
| 1.438 | SO₂CH₂Ph | - | H | H | H1 | |
| 1.439 | SO₂Ph | - | H | H | H1 | |
| 1.440 | SO₂Ph | - | H | H | H2 | |
| 1.441 | SO₂CF₃ | - | H | H | H1 | |
| 1.442 | SO₂CF₃ | - | H | H | H2 | |
| 1.443 | SO₂CHF₂ | - | H | H | H1 | |
| 1.444 | SO₂CHF₂ | - | H | H | H2 | |
| 1.445 | SO₂CH₂F | - | H | H | H1 | |
| 1.446 | SO₂CH=CH₂ | - | H | H | H1 | |
| 1.447 | SO₂CH2CH=CH2 | - | H | H | H1 | |
| 1.448 | SO₂CH2CH=CH2 | - | H | H | H2 | |
| 1.449 | SO₂C≡CH | - | H | H | H1 | |
| 1.450 | SO₂CH₂C≡CH | - | H | H | H1 | |
| 1.451 | SO₂CH₂C≡CH | - | H | H | H2 | |
| 1.452 | SO₂-cyclopropyl | - | H | H | H1 | |
| 1.453 | SO₂-cyclopropyl | - | H | H | H2 | |
| 1.454 | SO₂CH₂-cyclopropyl | - | H | H | H1 | |
| 1.455 | SO₂CH₂-cyclopropyl | - | H | H | H2 | |
| 1.456 | SO₂NHCH₃ | - | H | H | H1 | |
| 1.457 | SO₂N(CH₃)₂ | - | H | H | H1 | |
| 1.458 | SO₂N(CH₃)₂ | - | H | H | H2 | |
| 1.459 | SO₂NHCF₃ | - | H | H | H1 | |
| 1.460 | SO₂NHCF₃ | - | H | H | H2 | |
| 1.461 | SO₂NHCHF₂ | - | H | H | H1 | |
| 1.462 | SO₂NHCHF₂ | - | H | H | H2 | |
| 1.463 | OSO₂CH₃ | - | H | H | H1 | * |
| 1.464 | OSO₂CH₃ | | H | Na | H1 | |
| 1.465 | OSO₂CH₃ | 5-CH₃ | H | H | H1 | |
| 1.466 | OSO₂CH₃ | - | CH₃ | H | H1 | |
| 1.467 | OSO₂CH₃ | - | H | H | H2 | |
| 1.468 | OSO₂CH₃ | - | H | Na | H2 | |
| 1.469 | OSO₂CH₃ | 5-CH₃ | H | H | H2 | |
| 1.470 | OSO₂CH₃ | - | CH₃ | H | H2 | |
| 1.471 | OSO₂CH₃ | - | H | H | H5 | * |
| 1.472 | OSO₂CH₃ | - | H | H | H6 | * |
| 1.473 | OSO₂CH₃ | - | H | Na | H6 | |
| 1.474 | OSO₂CH₃ | 5-CH₃ | H | H | H6 | |
| 1.475 | OSO₂CH₃ | - | CH₃ | H | H6 | |
| 1.476 | OSO₂CH₃ | - | H | H | H7 | * |
| 1.477 | OSO₂CH₃ | - | H | H | H3 | |
| 1.478 | OSO₂CH₃ | - | H | H | H10 | |
| 1.479 | OSO₂CH₃ | - | H | H | H11 | |
| 1.480 | OSO₂CH₃ | - | H | H | H12 | |
| 1.481 | OSO₂CH₃ | - | H | H | H13 | |
| 1.482 | OSO₂CH₂CH₃ | - | H | H | H1 | |
| 1.483 | OSO₂CH(CH₃)₂ | - | H | H | H1 | |
| 1.484 | OSO₂C(CH₃)₃ | - | H | H | H1 | |
| 1.485 | OSO₂CH₂Ph | - | H | H | H1 | |
| 1.486 | OSO₂CF₃ | - | H | H | H1 | |
| 1.487 | OSO₂CF₃ | - | H | H | H2 | |
| 1.488 | OSO₂CHF₂ | - | H | H | H1 | |
| 1.489 | OSO₂CHF₂ | - | H | H | H2 | |
| 1.490 | OSO₂CH₂F | - | H | H | H1 | |
| 1.491 | OSO₂CH₂CF₃ | - | H | H | H1 | |
| 1.492 | OSO₂CH₂CHF₂ | - | H | H | H1 | |
| 1.493 | OSO₂(CH₂)₂F | - | H | H | H1 | |
| 1.494 | OSO₂CH=CH₂ | - | H | H | H1 | |
| 1.495 | OSO₂CH₂CH=CH₂ | - | H | H | H1 | |
| 1.496 | OSO₂CH₂CH=CH₂ | - | H | H | H2 | |
| 1.497 | OSO₂C≡CH | - | H | H | H1 | |
| 1.498 | OSO₂CH₂C≡CH | - | H | H | H1 | |
| 1.499 | OSO₂CH₂C≡CH | - | H | H | H2 | |
| 1.500 | OS0₂-cyclopropyl | - | H | H | H1 | |
| 1.501 | OSO₂-cyclopropyl | - | H | H | H2 | |
| 1.502 | OSO₂CH₂-cyclopropyl | - | H | H | H1 | |
| 1.503 | OSO₂CH₂-cyclopropyl | - | H | H | H2 | |
| 1.504 | OSO₂CH₂CN | - | H | H | H1 | |
| 1.505 | OSO₂CH₂CN | - | H | H | H2 | |
| 1.506 | OSO₂NHCH₃ | - | H | H | H1 | |
| 1.507 | OSO₂N(CH₂)₂ | - | H | H | H1 | * |
| 1.508 | OSO₂N(CH₂)₂ | - | H | H | H2 | |
| 1.509 | OSO₂N(CH₂)₂ | - | H | H | H5 | * |
| 1.510 | OSO₂N(CH₂)₂ | - | H | H | H6 | * |
| 1.511 | OSO₂N(CH₂)₂ | - | H | H | H7 | * |
| 1.512 | OSO₂NHCH₂CH=CH₂ | - | H | H | H1 | |
| 1.513 | OSO₂NHCH₂C≡CH | - | H | H | H1 | |
| 1.514 | OSO₂NHCF₃ | - | H | H | H1 | |
| 1.515 | OSO₂NHCF₃ | - | H | H | H2 | |
| 1.516 | OSO₂NHCHF₂ | - | H | H | H1 | |
| 1.517 | OSO₂NHCH₂F | - | H | H | H1 | |
| 1.518 | OC(O)H | - | H | H | H1 | |
| 1.519 | OC(O)H | - | H | H | H2 | |
| 1.520 | OC(O)CH₃ | - | H | H | H1 | |
| 1.521 | OC(O)CH₃ | - | H | H | H2 | |
| 1.522 | OC(O)CH₂CH₃ | - | H | H | H1 | |
| 1.523 | OC(O)CH₂CH₃ | - | H | H | H2 | |
| 1.524 | OC(O)OCH₃ | - | H | H | H1 | |
| 1.525 | OC(O)OCH₃ | - | H | H | H2 | |
| 1.526 | OC(O)OCH₂CH₃ | - | H | H | H1 | |
| 1.527 | OC(O)OCH₂CH₃ | - | H | H | H2 | |
| 1.528 | OC(O)NH₂ | - | H | H | H1 | |
| 1.529 | OC(O)NHCH₃ | - | H | H | H1 | |
| 1.530 | OC(O)N(CH₃)₂ | - | H | H | H1 | |
| 1.531 | OC(O)N(CH₃)₂ | - | H | H | H2 | |
| 1.532 | OC(O)N(CH₂CH₃)₂ | - | H | H | H1 | |
| 1.533 | Si(CH₃)₃ | - | H | H | H1 | |
| 1.534 | Si(CH₃)₃ | - | H | H | H2 | |
| 1.535 | 2-thienyl | - | H | H | H1 | |
| 1.536 | 2-thienyl | - | H | H | H2 | |
| 1.537 | 3-thienyl | - | H | H | H1 | |
| 1.538 | 3-thienyl | - | H | H | H2 | |
| 1.539 | 2-pyridyl | - | H | H | H1 | |
| 1.540 | 2-pyridyl | - | H | H | H2 | |
| 1.541 | 3-pyridyl | - | H | H | H1 | |
| 1.542 | 3-pyridyl | - | H | H | H2 | |
| 1.543 | 4-pyridyl | - | H | H | H1 | |
| 1.544 | 4-pyridyl | - | H | H | H2 | |
| 1.545 | OH | - | H | H | H1 | |
| 1.546 | OH | - | H | H | H2 | |
| 1.547 | SCH₃ | - | H | Na | H1 | |
| 1.548 | SCH₃ | 5-CH₃ | H | H | H1 | |
| 1.549 | SCH₃ | - | CH₃ | H | H1 | |
| 1.550 | SCH₃ | - | H | H | H5 | * |
| 1.551 | SCH₃ | - | H | H | H6 | * |
| 1.552 | SCH₃ | - | H | H | H7 | |
| 1.553 | O(CH₂)₃CH₃ | - | H | H | H2 | |
| 1.554 | O(CH₂)₃CH₃ | - | H | H | H5 | |
| 1.555 | O(CH₂)₃CH₃ | - | H | H | H6 | |
| 1.556 | O(CH₂)₃CH₃ | - | H | H | H7 | |
| 1.557 | OCH(CH₃)CH₂CH₃ | - | H | H | H2 | |
| 1.558 | OCH(CH₃)CH₂CH₃ | - | H | H | H5 | |
| 1.559 | OCH(CH₃)CH₂CH₃ | - | H | H | H6 | |
| 1.560 | OCH(CH₃)CH₂CH₃ | - | H | H | H7 | |
| 1.561 | OCH₂CH(CH₃)₂ | - | H | H | H2 | |
| 1.562 | OCH₂CH(CH₃)₂ | - | H | H | H5 | |
| 1.563 | OCH₂CH(CH₃)₂ | - | H | H | H6 | |
| 1.564 | OCH₂CH(CH₃)₂ | - | H | H | H7 | |
| 1.565 | OC(CH₃)₃ | - | H | H | H5 | |
| 1.566 | OC(CH₃)₃ | - | H | H | H6 | |
| 1.567 | OC(CH₃)₃ | - | H | H | H7 | |
| 1.568 | O(CH₂)₂Cl | - | H | H | H1 | |
| 1.569 | O(CH₂)₂Cl | - | H | H | H2 | |
| 1.570 | O(CH₂)₂Cl | - | H | H | H5 | |
| 1.571 | O(CH₂)₂Cl | - | H | H | H6 | |
| 1.572 | O(CH₂)₂Cl | - | H | H | H7 | |
| 1.573 | O(CH₂)₃Cl | - | H | H | H1 | |
| 1.574 | O(CH₂)₃Cl | - | H | H | H2 | |
| 1.575 | O(CH₂)₃Cl | - | H | H | H5 | |
| 1.576 | O(CH₂)₃Cl | - | H | H | H6 | |
| 1.577 | O(CH₂)₃Cl | - | H | H | H7 | |
| 1.578 | O-cyclopropyl | - | H | H | H5 | |
| 1.579 | O-cyclopropyl | - | H | H | H6 | |
| 1.580 | O-cyclopropyl | - | H | H | H7 | |
| 1.581 | SCH₂CH₃ | - | H | H | H5 | |
| 1.582 | SCH₂CH₃ | - | H | H | H6 | |
| 1.583 | SCH₂CH₃ | - | H | H | H7 | |
| 1.584 | S(O)CH₃ | - | H | H | H2 | |
| 1.585 | S(O)CH₃ | - | H | H | H5 | |
| 1.586 | S(O)CH₃ | - | H | H | H6 | |
| 1.587 | S(O)CH₃ | - | H | H | H7 | |
| 1.588 | S(O)CH₂CH₃ | - | H | H | H2 | |
| 1.589 | S(O)CH₂CH₃ | - | H | H | H5 | |
| 1.590 | S(O)CH₂CH₃ | - | H | H | H6 | |
| 1.591 | S(O)CH₂CH₃ | - | H | H | H7 | |
| 1.592 | SO₂CH₃ | - | H | H | H5 | |
| 1.593 | SO₂CH₃ | - | H | H | H6 | |
| 1.594 | SO₂CH₃ | - | H | H | H7 | |
| 1.595 | SO₂CH₂CH₃ | - | H | H | H5 | |
| 1.596 | SO₂CH₂CH₃ | - | H | H | H6 | |
| 1.597 | SO₂CH₂CH₃ | - | H | H | H7 | |

¹H-NMR Daten:
Beispiel-Nr.: 1.001 (d₆-DMSO): 13.11 (br s, 1 H); 10.74 (br s, 1 H); 8.04 (br d, J = 7.6, 1 H); 7.49 (ddd, J = 1.1, 8.4, 11.3, 1 H); 7.41 (dt, J = 5.3, 7.9, 1 H); 6.02 (s, 1 H); 3.94 (s, 6H).
Beispiel-Nr.: 1.002 (d₆-DMSO): 13.01 (br s, 1 H); 11.15 (s, 1 H); 8.04 (br d, J = 7.6, 1 H); 7.49 (ddd, J = 1.1, 8.4, 11.2, 1 H); 7.42 (dt, J = 5.3, 7.8, 1 H); 3.99 (s, 3H); 2.48 (s, 3H).
Beispiel-Nr.: 1.003 (CDCl₃): 13.51 (br s, 1H); 8.14 (br s, 1H); 7.90 (m, 1H); 7.17 (m, 2H); 6.74 (s, 1 H); 2.43 (s, 6H).
Beispiel-Nr.: 1.004 (CDCl₃): 12.45 (br s, 1 H); 7.96 (m, 1 H); 7.43 (br s, 1 H); 7.22 (m, 2H); 6.51 (s, 1H); 4.05 (s, 6H).
Beispiel-Nr.: 1.012 (CDCl₃): 12.81 (s, 1 H); 8.02 (d, J = 7.8, 1 H); 7.33 (d, J = 7.8, 1 H); 7.12 (br s, 1 H); 7.05 (t, J = 7.8, 1 H); 5.80 (s, 1 H); 4.00 (s, 6H); 2.89 (s, 3H).
Beispiel-Nr.: 1.016 (CDCl₃): 12.76 (br s, 1 H); 8.02 (br d, J = 7.8, 1 H); 7.34 (br s, 1 H); 7.33 (br d, J = 7.5, 1 H); 7.06 (t, J = 7.8, 1 H); 4.06 (s, 3H); 2.88 (s, 3H); 2.59 (s, 3H).
Beispiel-Nr.: 1.020 (CDCl₃): 13.29 (br s, 1 H); 8.01 (dd, J = 0.7, 7.8, 1 H); 7.44 (br s, 1 H); 7.32 (br d, J = 7.5, 1 H); 7.03 (t, J = 7.8, 1 H); 6.78 (s, 1 H); 2.90 (s, 3H); 2.48 (s, 3H).
Beispiel-Nr.: 1.021 (CDCl₃): 12.37 (br s, 1 H); 8.02 (d, J = 7.8, 1 H); 7.35 (br s, 1 H); 7.33 (d, J = 7.5, 1 H); 7.05 (t, J = 7.8, 1H); 6.50 (s, 1 H); 4.06 (s, 3H); 2.89 (s, 3H).
Beispiel-Nr.: 1.025 (CDCl₃): 12.47 (br s, 1 H); 8.02 (dd, J = 0.7, 7.8, 1 H); 7.33 (br d, J = 7.8, 1H); 7.25 (br s, 1 H); 7.06 (t, J = 7.8, 1H); 4.08 (s, 6H); 2.88 (s, 3H).
Beispiel-Nr.: 1.091 (CDCl₃): 13.09 (br s, 1 H); 8.38 (dd, J = 1.3, 8.0, 1 H); 7.96 (br dd, J = 0.7, 8.0, 1 H); 7.28 (td, J = 0.7, 8.0, 1H); 7.23 (br s, 1 H); 5.81 (s, 1 H); 3.98 (s, 6H).
Beispiel-Nr.: 1.095 (CDCl₃): 13.00 (br s, 1 H); 9.96 (s, 1 H); 8.26 (dd, J = 1.2, 8.0, 1 H); 7.84 (br d, J = 8.0, 1 H); 7.19 (t, J = 7.9, 1 H); 3.93 (s, 3H); 2.44 (s, 3H).
Beispiel-Nr.: 1.099 (d₆-DMSO): 14.08 (br s, 1 H); 10.67 (s, 1 H); 8.47 (dd, J = 1.1, 7.9, 1 H); 8.02 (dd, J = 1.0, 8.0, 1 H); 7.46 (t, J = 8.1, 1 H); 7.02 (s, 1 H); 2.43 (s, 6H).
Beispiel-Nr.: 1.100 (CDCl₃): 12.62 (br s, 1 H); 8.38 (dd, J = 1.3, 8.0, 1 H); 7.97 (br dd, J = 0.7, 8.0, 1 H); 7.63 (br s, 1 H); 7.30 ( td, J = 0.7, 8.0, 1 H); 6.51 (s, 1 H); 4.04 (s, 3H).
Beispiel-Nr.: 1.104 (CDCl₃): 12.76 (br s, 1 H); 9.40 (br s. 1 H); 8.32 (dd, J = 1.2, 8.0, 1H); 7.90 (dd, J = 0.7, 8.0, 1H); 7.24 (m, 1H); 4.00 (s, 6H).
Beispiel-Nr.: 1.146 (d₆-DMSO): 12.69 (br s, 1 H); 10.52 (br s, 1 H); 7.81 (m, 1 H); 7.28 (m, 2H); 6.03 (s, 1 H); 3.93 (s, 6H); 3.73 (s, 3H).
Beispiel-Nr.: 1.150 (d₆-DMSO): 12.41 (br s, 1 H); 10.98 (br s, 1 H); 7.81 (m, 1 H); 7.29 (m, 2H); 3.99 (s, 3H); 3.79 (s, 3H); 2.50 (s, 3H).
Beispiel-Nr.: 1.154 (d₆-DMSO): 13.05 (s, 1 H); 10.45 (s, 1 H); 7.77 (m, 1 H); 7.23 (m, 2H); 7.01 (s, 1 H); 3.70 (s, 3H); 2.42 (s, 6H).
Beispiel-Nr. 1.155 (d₆-DMSO): 11.97 (s, 1H); 10.73 (s, 1H); 7.79 (m, 1H); 7.26 (m, 2H); 6.88 (s, 1 H); 3.98 (s, 3H); 3.78 (s, 3H).
Beispiel-Nr.: 1.159 (CDCl₃): 12.13 (s, 1 H); 7.81 (dd, J = 1.1, 7.9, 1 H); 7.33 (br s, 1 H); 7.11 (t, J = 8.3, 1H); 7.01 (dd, J = 1.0, 8.5, 1H); 4.07 (s, 6H); 3.91 (s, 3H).
Beispiel-Nr.: 1.164 (CDCl₃): 12.46 (s, 1 H); 7.82 (dd, J = 1.2, 7.8, 1 H); 7.18 (br s, 1 H); 7.08 (t, J = 8.4, 1 H); 6.99 (dd, J = 1.2, 8.4, 1 H); 5.78 (s, 1 H); 4.14 (q, J = 7.0, 2H); 3.97 (s, 6H); 1.32 (t, J = 7.0, 3H).
Beispiel-Nr.: 1.165 (d₆-DMSO): 12.25 (s, 1 H); 10.99 (s, 1 H); 7.79 (m, 1 H); 7.26 (m, 2H); 4.14 (q, J = 7.7, 2H); 3.98 (s, 3 H); 2.47 (s, 3H); 1.21 (t, J = 7.0, 3H).
Beispiel-Nr.: 1.166 (CDCl₃): 12.68 (s, 1 H); 7.81 (dd, J = 1.2, 7.8, 1H); 7.60 (br s, 1 H); 7.07 (t, J = 8.3, 1 H); 6.98 (dd, J = 1.2, 8.4, 1H); 6.76 (s, 1 H); 4.13 (q, J = 7.0, 2H); 2.46 (s, 6H); 1.37 (t, J = 7.0, 3H).
Beispiel-Nr.: 1.167 (CDCl₃): 11.83 (s, 1 H); 7.82 (dd, J = 1.1, 7.7 1 H); 7.36 (br s, 1 H); 7.09 (t, J = 8.3, 1H); 7.00 (dd, J = 1.1, 8.4, 1H); 6.49 (s, 1H); 4.17 (q, J = 7.0, 2H); 4.01 (s, 3H); 1.41 (t, J = 7.0, 3H).
Beispiel-Nr.: 1.168 (CDCl₃): 11.99 (s, 1H); 7.82 (dd, J = 1.3, 7.8, 1H); 7.29 (br s, 1H); 7.09 (t, J = 8.4, 1 H); 7.00 (dd, J = 1.2, 8.4, 1 H); 4.16 (q, J = 7.0, 2H); 4.06 (s, 6H); 1.43 (t, J = 7.0, 3H).
Beispiel-Nr.: 1.171 (CDCl₃): 12.41 (s, 1 H); 7.82 (dd, J = 1.2, 7.8, 1 H); 7.17 (br s, 1 H); 7.07 (t, J = 8.4, 1 H); 6.99 (dd, J = 1.1, 8.4, 1 H); 5.79 (s, 1 H); 4.02 (t, J = 6.7, 2H); 3.97 (s, 6H); 1.74 (m, 2H); 0.96 (t, J = 7.4, 3H).
Beispiel-Nr.: 1.173 (d₆-DMSO): 12.81 (br s, 1H); 10.52 (s, 1H); 7.79 (dd, J = 1.7,7.1, 1 H); 7.24 (m, 2H); 7.02 (s, 1 H); 4.00 (t, J = 6.4, 2H); 2.41 (s, 6H); 1.56 (m, 2H); 0.87 (t, J = 7.4, 3H).
Beispiel-Nr.: 1.174 (CDCl₃): 11.77 (s, 1 H); 7.81 (dd, J = 1.2, 7.8, 1 H); 7.30 (br s, 1H); 7.08 (t, J = 8.4, 1 H); 7.00 (dd, J = 1.1, 8.4, 1 H); 6.48 (s, 1 H); 4.04 (t, J = 6.6, 2H); 4.00 (s, 3H); 1.82 (m, 2H); 1.01 (t, J = 7.4, 3H).
Beispiel-Nr.: 1.175 (CDCl₃): 11,96 (s, 1 H); 7.80 (dd, J = 1.2, 7.8, 1 H); 7.46 (br s, 1 H); 7.08 (t, J = 8.4, 1 H); 6.99 (dd, J = 1.1, 8.4, 1 H); 4.05 (s, 6H); 4.03 (t, J = 6.6, 2H); 1.83 (m, 2H); 1.03 (t, J = 7.4, 3H).
Beispiel-Nr.: 1.178 (CDCl₃): 12.32 (s, 1 H); 7.80 (dd, J = 1.2, 7.7, 1 H); 7.17 (br s, 1 H); 7.06 (t, J = 8.4, 1 H); 6.99 (br d, J = 8.2, 1 H); 5.78 (s, 1 H); 4.70 (m, 1 H); 3.97 (s, 6H); 1.28 (d, J = 6.1, 6H).
Beispiel-Nr.: 1.182 (CDCl₃): 12.00 (br s, 1 H); 7.80 (dd, J = 1.0, 7.8, 1 H); 7.43 (br s, 1 H); 7.08 (t, J = 8.2, 1 H); 7.00 (br d, J = 8.1, 1 H); 4.71 (m, 1 H); 4.05 (s, 3H); 2.58 (s, 3H); 1.35 (d, J = 6.0, 6H).
Beispiel-Nr.: 1.186 (CDCl₃): 12.51 (br s, 1 H); 7.80 (m, 1 H); 7.39 (br s, 1 H); 7.06 (m, 1 H); 6.98 (m, 1 H); 6.76 (s, 1 H); 4.69 (m, 1 H); 2.47 (s, 6H); 1.32 (br d, J = 6.0, 6H).
Beispiel-Nr.: 1.187 (CDCl₃): 11.68 (s, 1 H); 7.80 (dd, J = 1.1, 7.8, 1H); 7.33 (br s, 1 H); 7.07 (t, J = 8.4, 11H); 7.00 (br d, J = 7.8, 1H); 6.48 (s, 1H); 4.71 (m, 1H); 4.00 (s, 3H); 1.35(d,J=6.1,6H).
Beispiel-Nr.: 1.191 (CDCl₃): 11.90 (br s, 1 H); 7.79 (dd, J = 1.3, 7.7, 1 H); 7.39 (br s, 1 H); 7.08 (t, J = 8.4, 1H); 7.00 (dd, J = 1.2, 8.5, 1 H); 4.71 (m, 1 H); 4.06 (s, 6H); 1.35 (d, J = 6.1, 6H).
Beispiel-Nr.: 1.229 (CDCl₃): 12.51 (br s, 1 H); 7.82 (dd, J = 1.1, 7.7, 1 H); 7.26 (br s, 1 H); 7.06 (t, J = 8.4, 1 H); 6.97 (dd, J = 1.1, 8.5, 1H); 5.78 (s, 1 H); 3.97 (s, 6H); 3.89 (d, J = 6.9, 2H); 1.15 (m, 1 H); 0.47 (m, 2H); 0.23 (m, 2H).
Beispiel-Nr.: 1.233(CDCl₃): 12.11 (br s, 1 H); 7.83 (dd, J = 1.1, 7.8, 1 H); 7.44 (br s, 1 H); 7.09 (t, J = 8.2, 1 H); 7.00 (dd, J = 1.0, 8.4, 1H); 4.05 (s, 3H); 3.94 (d, J = 6.8, 2H); 2.55 (s, 3H); 1.25 (m, 1 H); 0.54 (m, 2H); 0.32 (m, 2H).
Beispiel-Nr.: 1.237 (CDCl₃): 12.70 (br s, 1 H); 7.83 (dd, J = 1.1, 7.8, 1H); 7.41 (br s, 1 H); 7.07 (t, J = 8.0, 1 H); 6.99 (dd, J = 1.0, 8.4, 1H); 6.76 (s, 1 H); 3.93 (d, J = 6.7, 2H); 2.45 (s, 6H); 1.22 (m, 1 H); 0.47 (m, 2H); 0.29 (m, 2H).
Beispiel-Nr.: 1.238 (CDCl₃): 11.82 (br s, 1 H); 7.83 (dd, J = 1.1, 7.8, 1 H); 7.33 (br s, 1 H); 7.08 (t, J = 8.3, 1 H); 6.99 (dd, J = 1.0, 8.4, 1 H); 6.48 (s, 1 H); 4.02 (s, 3H); 3.93 (d, J = 6.9, 2H); 1.25 (m, 1 H); 0.53 (m, 1 H); 0.29 (m, 2H).
Beispiel-Nr.: 1.242 (CDCl₃): 11.97 (br s, 1 H); 7.83 (dd, J = 1.1, 7.8, 1H); 7.32 (br s, 1 H); 7.09 (t, J = 8.3, 1 H); 6.99 (dd, J = 1.0, 8.4, 1 H); 4.05 (s, 6H); 3.93 (d, J = 7.0, 2H); 1.28 (m, 1 H); 0.57 (m, 2H); 0.32 (m, 2H).
Beispiel-Nr.: 1.302 (d₆-DMSO): 13.11 (br s, 1 H); 10.76 (br s, 1 H); 8.26 (dd, J = 0.8, 8.0, 1H); 7.63 (dt, J = 0.8, 8.0, 1 H); 7.47 (t, J = 8.0, 1H); 6.03 (s, 1 H); 3.95 (s, 6H).
Beispiel-Nr.: 1.306 (CDCl₃): 12.77 (br s, 1 H); 8.16 (dd, J = 1.2, 8.0, 1H); 7.43 (m, 2H); 7.24 (t, J = 8.1, 1H); 4.05 (s, 3H); 2.58 (s, 3H).
Beispiel-Nr.: 1.310 (d₆-DMSO): 13.86 (s, 1 H); 10.72 (s, 1H); 8.24 (dd, J = 1.2, 7.9, 1 H); 7.60 (dt, J = 1.3, 8.3, 1H); 7.44 (t, J = 8.1, 1 H); 7.03 (s, 1 H); 2.42 (s, 3H).
Beispiel-Nr.: 1.311 (CDCl₃): 12.38 (br s, 1 H); 8.17 (dd, J = 0.8, 8.0, 1H); 7.44 (dt, J = 0.8, 8.0, 1 H); 7.37 (br s, 1 H); 7.24 (t, J = 8.0, 1 H); 6.52 (s, 1 H); 4.04 (s, 3H).
Beispiel-Nr.: 1.315 (CDCl₃): 12.51 (s, 1 H); 8.16 (dd, J = 1.2, 8.0, 1 H); 7.43 (dt, J = 1.2, 8.3, 1 H); 7.38 (s, 1 H); 7.24 (t, J = 8.0, 1 H); 4.07 (s, 6H).
Beispiel-Nr.: 1.321 (CDCl₃): 12.87 (br s, 1 H); 8.12 (dd, J = 1.3, 7.9, 1 H); 7.34 (dd, J = 1.2, 8.2, 1 H); 7.21 (br s, 1 H); 7.21 (t, J = 8.0, 1 H); 6.69 (t, J = 74.5, 1 H); 5.81 (s, 1 H); 3.98 (s, 6H).
Beispiel-Nr.: 1.325 (CDCl₃): 12.84 (br s, 1 H); 9.55 (br s, 1 H); 8.02 (dd, J = 1.2, 7.9, 1 H); 7.25 (dd, J = 1.0, 8.0, 1 H); 7.15 (t, J = 8.0, 1 H); 6.67 (t, J = 74.6, 1 H); 3.97 (s, 3H); 2.49 (s, 3H).
Beispiel-Nr.: 1.329 (CDCl₃): 13.46 (br s, 1 H); 8.08 (dd, J = 1.2, 7.9, 1 H); 7.97 (br s, 1 H); 7.30 (dd, J = 1.1, 8.2, 1 H); 7.18 (t, J = 8.0, 1 H); 6.76 (s, 1 H); 6.69 (t, J = 74.8, 1 H); 2.45 (s, 6H).
Beispiel-Nr.: 1.330 (CDCl₃): 12.35 (br s, 1 H); 8.11 (dd, J = 1.1, 7.9, 1 H); 7.39 (br s, 1 H); 7.34 (dd, J = 1.1, 8.2, 1 H); 7.22 (t, J = 8.0, 1 H); 6.68 (t, J = 74.3, 1 H); 6.51 (s, 1H); 4.04 (s, 3H).
Beispiel-Nr.: 1.342 (CDCl₃): 12.45 (br s, 1 H); 7.99 (dd, J = 0.9, 7.8, 1 H); 7.37 (br s, 1 H); 7.18 (t, J = 8.2, 1 H); 7.08 (dd, J = 0.9, 8.3, 1 H); 4.49 (q, J = 8.1, 2H); 4.05 (s, 3H); 2.58 (s, 3H).
Beispiel-Nr.: 1.343 (CDCl₃): 13.03 (br s, 1 H); 7.98 (br d, J = 7.8, 1H); 7.46 (br s, 1 H); 7.15 (t, J = 8.2, 1H); 7.07 (br d, J = 8.1, 1H); 6.76 (s, 1H); 4.48 (q, J = 8.2, 2H); 2.45 (s, 6H).
Beispiel-Nr.: 1.344 (CDCl₃): 12.11 (s, 1 H); 7.98 (br d, J = 7.6, 1 H); 7.32 (br s, 1 H); 7.17 (t, J = 8.2, 1 H); 7.09 (br d, J = 8.0, 1 H); 6.50 (s, 1 H); 4.50 (q, J = 8.2, 2H); 4.02 (s, 3H).
Beispiel-Nr.: 1.352 (CDCl₃): 12.37 (br s, 1 H); 8.31 (br s, 1H); 7.89 (br d, J = 7.6, 1H); 7.13 (t, J = 8.1, 1 H); 7.03 (br d, J = 8.2, 1 H); 6.15 (tt, J = 3.9, 54.9, 1 H); 4.29 (td, J = 3.7, 12.9, 2H); 4.02 (s, 3H); 2.54 (s, 3H).
Beispiel-Nr.: 1.353 (d₆-DMSO): 13.04 (br s, 1H); 9.33 (br s, 1 H); 7.81 (dd, J = 1.1, 7.7, 1H); 7.08 (t, J = 8.1, 1H); 7.02 (dd, J = 1.1, 8.3, 1 H); 6.69 (s, 1H); 6.04 (tt, J = 3.9, 55.0, 1 H); 4.24 (td, J = 3.9, 13.2, 2H); 2.37 (s, 6H).
Beispiel-Nr. 1.354 (CDCl₃): 12.10 (br s, 1H); 9.25 (br s, 1 H); 7.91 (dd, J = 1.1, 7.8, 1H); 7.16 (t, J = 8.2, 1H); 7.08 (dd, J = 1.0, 8.4, 1H); 6.47 (s, 1H); 6.17 (tt, J = 3.9, 54.9, 1 H); 4.33 (td, J = 4.0, 13.2, 2H); 4.02 (s, 3H).
Beispiel-Nr.: 1.355 (CDCl₃): 12.23 (br s, 1 H); 8.48 (br s, 1 H); 7.88 (br d, J = 7.7, 1 H); 7.12 (t, J = 8.3, 1 H); 7.02 (br d, J = 8.3, 1 H); 6.18 (tt, J = 4.0, 54.8, 1 H); 4.28 (td, J = 4.0, 13.0, 2H); 4.02 (s, 6H).
Beispiel-Nr.: 1.362 (CDCl₃): 12.52 (br s, 1 H); 7.94 (dd, J = 1.1, 7.8, 1 H); 7.13 (br s, 1 H); 7.13 (t, J = 8.3, 1 H); 7.04 (br d, J = 8.2, 1 H); 5.79 (s, 1 H); 4.85 (m, 1 H); 3.96 (s, 6H); 1.52 (d, J = 6.4, 3H).
Beispiel-Nr.: 1.363 (CDCl₃): 12.30 (br s, 1 H); 7.94 (d, J = 7.7, 1 H); 7.44 (br s, 1 H); 7.14 (t, J = 8.2, 1 H); 7.04 (d, J = 8.2, 1 H); 4.84 (m, 1 H); 4.04 (s, 3H); 2.00 (s, 3H); 1.55 (d, J = 6.1, 3H).
Beispiel-Nr.: 1.364 (CDCl₃): 7.79 (dd, J = 1.0, 7.7, 1H); 7.01 (t, J = 8.3, 1H), 6.96 (br d, J = 7.8, 1 H); 6.65 (s, 1 H); 4.79 (m, 1 H); 2.33 (s, 6H); 1.37 (d, J = 6.4, 3H).
Beispiel-Nr.: 1.365 (CDCl₃): 11.95 (br s, 1 H); 7.94 (dd, J = 1.1, 7.8, 1H); 7.29 (br s, 1 H); 7.14 (t, J = 8.4, 1 H); 7.05 (br d, J = 8.2, 1H); 6.49 (s, 1 H); 4.84 (m, 1 H); 4.00 (s, 3H); 1.57 (d, J = 6.5, 3H).
Beispiel-Nr. 1.366 (CDCl₃): 12.17 (br s, 1 H); 7.93 (dd, J = 1.1, 7.8, 1H); 7.29 (br s, 1 H); 7.14 (t, J = 8.4, 1H); 7.06 (br d, J = 8.1, 1 H); 4.83 (m, 1 H); 4.06 (s, 6H); 1.58 (d, J = 6.5, 3H).
Beispiel-Nr.: 1.370 (CDCl₃): 12.40 (br s, 1 H); 8.01 (dd, J = 1.0, 7.7, 1 H); 7.35 (br s, 1 H); 7.19 (t, J = 8.3, 1 H); 7.11 (dd, J = 1.0, 8.3, 1 H); 4.57 (br t, J = 13.2, 2H); 4.05 (s, 3H); 2.57 (s, 3H).
Beispiel-Nr.: 1.371 (CDCl₃): 8.00 (dd, J = 1.3, 7.7, 1 H); 7.59 (br s, 1 H); 7.20 (t, J = 6.9, 1 H); 7.10 (dd, J = 1.3, 8.3, 1 H); 6.79 (s, 1 H); 4.57 (t, J = 13.2, 2H); 2.47 (s, 6H).
Beispiel-Nr.: 1.372 (CDCl₃): 12.08 (s, 1 H); 8.00 (dd, J = 1.4, 7.6, 1 H); 7.28 (br s, 1 H); 7.19 (t, J = 8.2, 1 H); 7.13 (dd, J = 1.2, 8.2, 1H); 6.50 (s, 1 H); 4.59 (br t, J = 12.8, 2H); 4.01 (s, 3H).
Beispiel-Nr.: 1.373 (CDCl₃): 12.27 (br s, 1 H); 7.98 (br d, J = 7.5, 1 H); 7.46 (br s, 1 H); 7.18 (t, J = 8.3, 1 H); 7.12 (br d, J = 7.3, 1 H); 4.58 (br t, J = 13.2, 2H); 4.05 (s, 6H).
Beispiel-Nr. 1.389 (CDCl₃): 12.99 (br.s, 1 H); 8.46 (br s, 1 H); 7.96 (dd, J = 1.0, 7.7, 1 H); 7.35 (d, J = 7.8, 1 H); 7.07 (t, J = 8.0, 1 H); 5.79 (s, 1 H); 4.00 (s, 6H); 2.45 (s, 3H).
Beispiel-Nr 1.390 (CDCl₃): 12.93 (br s, 1 H); 10.23 (br s, 1 H); 7.95 (dd, J = 1.1, 7.8, 1 H); 7.36 (d, J = 8.2, 1 H); 7.08 (t, J = 7.8, 1 H); 4.06 (s, 3H); 2.56 (s, 3H); 2.45 (s, 3H).
Beispiel-Nr.: 1.463 (CDCl₃): 13.00 (br s, 1 H); 8.71 (br s, 1 H); 7.98 (dd, J = 1.1, 8.0, 1 H); 7.50 (dd, J = 1.1, 8.3, 1 H); 7.12 (t, J = 8.1, 1 H); 5.66 (s, 1 H); 3.85 (s, 6H); 3.26 (s, 3H).
Beispiel-Nr.: 1.471 (CDCl₃): 13.54 (br s, 1 H); 8.11 (dd, J = 1.2, 8.0, 1 H); 7.67 (dd, J = 1.2, 8.3, 1 H); 7.44 (br s, 1 H); 7.23 (t, J = 8.1, 1 H); 6.78 (s, 1 H); 3.38 (s, 3H); 2.48 (s, 6H).
Beispiel-Nr.: 1.472 (CDCl₃): 12.45 (br s, 1 H); 8.12 (br d, J = 8.0, 1 H); 7.65 (br d, J = 8.3, 1 H); 7.39 (br s, 1 H); 7.25 (br t, J = 8.0, 1 H); 6.52 (s, 1 H); 4.04 (s, 3H), 3.41 (s, 3H).
Beispiel-Nr.: 1.476 (CDCl₃): 12.62 (br s, 1 H); 9.35 (s, 1 H); 8.05 (dd, J = 1.2, 8.0, 1 H); 7.55 (dd, J = 1.2, 8.3, 1 H); 7.18 (t, J = 8.1, 1 H); 4.00 (s, 6H); 3.37 (s, 3H).
Beispiel-Nr.: 1.507 (CDCl₃): 12.90 (br s, 1 H); 8.02 (dd, J = 1.2, 7.9, 1 H); 7.74 (dd, J = 1.2, 8.4, 1 H); 7.20 (dd, J = 8.0, 8.3, 1 H); 5.80 (s, 1 H); 4.00 (s, 6H); 3.10 (s, 6H).
Beispiel-Nr.: 1.509 (CDCl₃): 13.38 (br s, 1 H); 8.02 (dd, J = 1.1, 7.9, 1 H); 7.76 (dd, J = 1.1,8.4, 1 H); 7.40 (br s, 1 H); 7.19 (t, J = 8.0, 1 H); 6.77 (s, 1 H); 3.10 (s, 6H); 2.47 (s, 6H).
Beispiel-Nr.: 1.510 (CDCl₃): 12.31 (br s, 1 H); 8.03 (dd, = 1.1, 7.9, 1 H); 7.75 (dd, J = 1.1, 8.4, 1 H); 7.39 (br s, 1 H); 7.21 (t, J = 8.2, 1 H); 6.50 (s, 1 H); 4.03 (s, 3H); 3.10 (s, 6H).
Beispiel-Nr.: 1.511 (CDCl₃): 12.41 (br s, 1 H); 8.04 (dd, J = 1.2, 7.9, 1 H); 7.75 (dd, J = 1.2, 8.4, 1 H); 7.32 (br s, 1 H); 7.21 (dd, J = 8.0, 8.4, 1 H); 4.07 (s, 6H); 3.10 (s, 6H).
Beispiel-Nr. 1.550 (CDCl₃): 8.88 (br s, 1 H); 7.94 (dd, J = 1.1, 7.8, 1 H); 7.34 (dd, J = 0.5, 8.1, 1 H); 7.06 (t, J = 7.9, 1 H); 6.81 (br s, 1 H); 2.49 (s, 6H); 2.45 (s, 3H).
Beispiel-Nr. 1.551 (CDCl₃): 12.41 (br s, 1H); 7.95 (dd, J = 1.1, 7.8, 1H); 7.35 (d, J = 8.2, 1 H); 7.28 (br s, 1 H); 7.06 (t, J = 7.9, 1 H); 6.50 (s, 1 H); 4.06 (s, 3H); 2.47 (s, 3H).

**Tabelle 2: Verbindungen der allgemeinen Formel (II*)**

| | | | | | | | | |
|---|---|---|---|---|---|---|---|---|
| 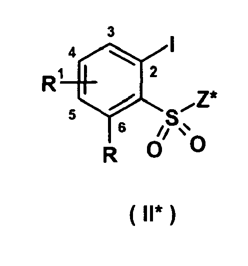 | | | | | | | | |
| Verbindungen a: Z* = NH₂ | | | | | | | | |
| Verbindungen b: Z* = NH-tert. Butyl | | | | | | | | |
| Verbindungen c: Z* = NH-C(O)O-Phenyl | | | | | | | | |
| Verbindungen d: Z* = NH-C(S)O-Phenyl | | | | | | | | |
| Verbindungen e: Z* = NCO | | | | | | | | |
| Verbindungen f: Z* = NCS | | | | | | | | |

| | R | R¹ | Z* | | | | | |
|---|---|---|---|---|---|---|---|---|
| | | | a | b | c | d | e | f |
| | | | | | | | | |
| 2.001 a-f | F | - | * | * | | | | |
| 2.002 a-f | Br | - | | | | | | |
| 2.003 a-f | I | - | | | | | | |
| 2.004 a-f | CH₃ | - | * | * | | | | |
| 2.005 a-f | CH₃ | 5-CH₃ | | | | | | |
| 2.006 a-f | CH₂CH₃ | - | | | | | | |
| 2.007 a-f | (CH₂)₂CH₃ | - | | | | | | |
| 2.008 a-f | CH(CH₃)₂ | - | | | | | | |
| 2.009 a-f | (CH₂)₃CH₃ | - | | | | | | |
| 2.010 a-f | CH(CH₃)CH₂CH₃ | - | | | | | | |
| 2.011 a-f | CH₂CH(CH₃)₂ | - | | | | | | |
| 2.012 a-f | C(CH₃)₃ | - | | | | | | |
| 2.013 a-f | CH=CH₂ | - | | | | | | |
| 2.014 a-f | C(CH₃)=CH₂ | - | | | | | | |
| 2.015 a-f | C≡CH | - | | | | | | |
| 2.016 a-f | C≡CCH₃ | - | | | | | | |
| 2.017 a-f | C≡CCH₂CH₃ | - | | | | | | |
| 2.018 a-f | CH₂CH=CH₂ | - | | | | | | |
| 2.019 a-f | CH₂C(CH₃)=CH₂ | - | | | | | | |
| 2.020 a-f | CH₂C≡CH | - | | | | | | |
| 2.021 a-f | CH₂C≡CCH₃ | - | | | | | | |
| 2.022 a-f | CH₂C≡CCH₂CH₃ | - | | | | | | |
| 2.023 a-f | Cyclopropyl | - | | | | | | |
| 2.024 a-f | 2,2-di-F-cyclopropyl | - | | | | | | |
| 2.025 a-f | 2,2-di-Cl-cyclopropyl | - | | | | | | |
| 2.026 a-f | 2,2-di-CH₃-cyclopropyl | - | | | | | | |
| 2.027 a-f | Cyclobutyl | - | | | | | | |
| 2.028 a-f | Cyclopentyl | - | | | | | | |
| 2.029 a-f | Cyclohexyl | - | | | | | | |
| 2.030 a-f | CH₂cyclopropyl | - | | | | | | |
| 2.031 a-f | CH₂cyclobutyl | - | | | | | | |
| 2.032 a-f | CH₂cyclopentyl | - | | | | | | |
| 2.033 a-f | CH₂cyclohexyl | - | | | | | | |
| 2.034 a-f | CH₂OCH₃ | - | | | | | | |
| 2.035 a-f | CH₂OCH₂CH₃ | - | | | | | | |
| 2.036 a-f | CH(CH₃)OCH₃ | - | | | | | | |
| 2.037 a-f | Ph | - | | | | | | |
| 2.038 a-f | 2-F-Ph | - | | | | | | |
| 2.039 a-f | 3-F-Ph | - | | | | | | |
| 2.040 a-f | 4-F-Ph | - | | | | | | |
| 2.041 a-f | 2,6-di-F-Ph | - | | | | | | |
| 2.042 a-f | 2,4-di-F-Ph | - | | | | | | |
| 2.043 a-f | 2-Cl-Ph | - | | | | | | |
| 2.044 a-f | 3-Cl-Ph | - | | | | | | |
| 2.045 a-f | 4-Cl-Ph | - | | | | | | |
| 2.046 a-f | 2,6-di-Cl-Ph | - | | | | | | |
| 2.047 a-f | 2,4-di-Cl-Ph | - | | | | | | |
| 2.048 a-f | 2-MeO-Ph | - | | | | | | |
| 2.049 a-f | 3-MeO-Ph | - | | | | | | |
| 2.050 a-f | 4-MeO-Ph | - | | | | | | |
| 2.051 a-f | 2,4-di-MeO-Ph | - | | | | | | |
| 2.052 a-f | 2-Me-Ph | - | | | | | | |
| 2.053 a-f | 3-Me-Ph | - | | | | | | |
| 2.054 a-f | 4-Me-Ph | - | | | | | | |
| 2.055 a-f | 2-CF₃-Ph | - | | | | | | |
| 2.056 a-f | 3-CF₃-Ph | - | | | | | | |
| 2.057 a-f | 4-CF₃-Ph | - | | | | | | |
| 2.058 a-f | CH₂Ph | - | | | | | | |
| 2.059 a-f | CH₂-2-F-Ph | - | | | | | | |
| 2.060 a-f | CH₂-2,4-di-F-Ph | - | | | | | | |
| 2.061 a-f | CH₂-2-MeO-Ph | - | | | | | | |
| 2.062 a-f | CH₂-3-MeO-Ph | - | | | | | | |
| 2.063 a-f | CF₃ | - | * | * | | | | |
| 2.064 a-f | CF₃ | 5-CH₃ | | | | | | |
| 2.065 a-f | CHF₂ | - | | | | | | |
| 2.066 a-f | CH₂F | - | | | | | | |
| 2.067 a-f | CH₂CF₃ | - | | | | | | |
| 2.068 a-f | CH₂CHF₂ | - | | | | | | |
| 2.069 a-f | CH₂CH₂F | - | | | | | | |
| 2.070 a-f | CF=CH₂ | - | | | | | | |
| 2.071 a-f | CH=CF₂ | - | | | | | | |
| 2.072 a-f | CF₂CH=CH₂ | - | | | | | | |
| 2.073 a-f | CH=CH-CF₃ | - | | | | | | |
| 2.074 a-f | CHFCH=CH₂ | - | | | | | | |
| 2.075 a-f | CN | - | | | | | | |
| 2.076 a-f | NO₂ | - | | | | | | |
| 2.077 a-f | NH₂ | - | | | | | | |
| 2.078 a-f | NHCH₃ | - | | | | | | |
| 2.079 a-f | N(CH₃)₂ | - | | | | | | |
| 2.080 a-f | N(CH₃)CH₂CH=CH₂ | - | | | | | | |
| 2.081 a-f | N(CH₃)CH₂C≡CH | - | | | | | | |
| 2.082 a-f | NH-cyclopropyl | - | | | | | | |
| 2.083 a-f | N(CH₃)-cyclopropyl | - | | | | | | |
| 2.084 a-f | N(CH₂CH₃)-cyclopropyl | - | | | | | | |
| 2.085 a-f | NHC(O)H | - | | | | | | |
| 2.086 a-f | NHC(O)CH₃ | - | | | | | | |
| 2.087 a-f | NHC(O)OCH₃ | - | | | | | | |
| 2.088 a-f | NHSO₂CH₃ | - | | | | | | |
| 2.089 a-f | NHSO₂CF₃ | - | | | | | | |
| 2.090 a-f | NHSO₂CHF₂ | - | | | | | | |
| 2.091 a-f | NHSO₂CH₂F | - | | | | | | |
| 2.092 a-f | OCH₃ | - | * | * | | | | |
| 2.093 a-f | OCH₃ | 5-CH₃ | | | | | | |
| 2.094 a-f | OCH₂CH₃ | - | * | | | | | |
| 2.095 a-f | O(CH₂)₂CH₃ | - | * | | | | | |
| 2.096 a-f | OCH(CH₃)₂ | - | * | | | | | |
| 2.097 a-f | OCH(CH₃)₂ | 5-CH₃ | | | | | | |
| 2.098 a-f | O(CH₂)₃CH₃ | - | | | | | | |
| 2.099 a-f | OCH(CH₃)CH₂CH₃ | - | | | | | | |
| 2.100 a-f | OCH₂CH(CH₃)₂ | - | | | | | | |
| 2.101 a-f | OC(CH₃)₃ | - | | | | | | |
| 2.102 a-f | OCH=CH₂ | - | | | | | | |
| 2.103 a-f | OC(CH₃)=CH₂ | - | | | | | | |
| 2.104 a-f | OCH=CH(CH₃) | - | | | | | | |
| 2.105 a-f | OCH=C(CH₃)₂ | - | | | | | | |
| 2.106 a-f | OC(CH₃)=CHCH₃ | - | | | | | | |
| 2.107 a-f | OC(CH₃)=C(CH₃)₂ | - | | | | | | |
| 2.108 a-f | OC≡CH | - | | | | | | |
| 2.109 a-f | OC≡CCH₃ | - | | | | | | |
| 2.110 a-f | OC≡CCH₂CH₃ | - | | | | | | |
| 2.111 a-f | OCH₂CH=CH₂ | - | | | | | | |
| 2.112 a-f | OCH₂C(CH₃)=CH₂ | - | | | | | | |
| 2.113 a-f | OCH₂CH=CHCH₃ | - | | | | | | |
| 2.114 a-f | OCH₂CH=C(CH₃)₂ | - | | | | | | |
| 2.115 a-f | OCH₂C(CH₃)=CHCH₃ | - | | | | | | |
| 2.116 a-f | OCH₂C(CH₃)=C(CH₃)₂ | - | | | | | | |
| 2.117 a-f | OCH(CH₃)CH=CH₂ | - | | | | | | |
| 2.118 a-f | OCH₂C≡CH | - | | | | | | |
| 2.119 a-f | OCH₂C≡CCH₃ | - | | | | | | |
| 2.120 a-f | OCH₂C≡CCH₂CH₃ | - | | | | | | |
| 2.121 a-f | OCH(CH₃)C≡CH | - | | | | | | |
| 2.122 a-f | O-cyclopropyl | - | | | | | | |
| 2.123 a-f | O-2,2-di-Cl-cyclopropyl | - | | | | | | |
| 2.124 a-f | O-2,2-di-F-cyclopropyl | - | | | | | | |
| 2.125 a-f | O-cyclobutyl | - | | | | | | |
| 2.126 a-f | O-cyclopentyl | - | | | | | | |
| 2.127 a-f | O-cyclohexyl | - | | | | | | |
| 2.128 a-f | OCH₂-cyclopropyl | - | * | | | | | |
| 2.129 a-f | OCH₂-cyclopropyl | 5-CH₃ | | | | | | |
| 2.130 a-f | OCH(CH₃)-cyclopropyl | - | | | | | | |
| 2.131 a-f | OCH₂-2-Me-cyclopropyl | - | | | | | | |
| 2.132 a-f | OCH₂-2,2-di-Me-cyclopropyl | - | | | | | | |
| 2.133 a-f | OCH₂-2,2-di-Cl-cyclopropyl | - | | | | | | |
| 2.134 a-f | OCH₂-2,2-di-F-cyclopropyl | - | | | | | | |
| 2.135 a-f | OCH₂-cyclobutyl | - | | | | | | |
| 2.136 a-f | OCH₂-cyclopentyl | - | | | | | | |
| 2.137 a-f | OCH(CH₃)-cyclopentyl | - | | | | | | |
| 2.138 a-f | OCH₂-cyclohexyl | - | | | | | | |
| 2.139 a-f | OCH(CH₃)-cyclohexyl | - | | | | | | |
| 2.140 a-f | OCH₂OCH₃ | - | | | | | | |
| 2.141 a-f | O(CH₂)₂OCH₃ | - | | | | | | |
| 2.142 a-f | OCH₂OCH₂CH₃ | - | | | | | | |
| 2.143 a-f | O(CH₂)₂OCH₂CH₃ | - | | | | | | |
| 2.144 a-f | OCH(CH₃)OCH₃ | - | | | | | | |
| 2.145 a-f | OPh | - | | | | | | |
| 2.146 a-f | O-2-F-Ph | - | | | | | | |
| 2.147 a-f | O-3-F-Ph | - | | | | | | |
| 2.148 a-f | O-4-F-Ph | - | | | | | | |
| 2.149 a-f | O-2,6-di-F-Ph | - | | | | | | |
| 2.150 a-f | O-2,4-di-F-Ph | - | | | | | | |
| 2.151 a-f | O-2-Cl-Ph | - | | | | | | |
| 2.152 a-f | O-3-Cl-Ph | - | | | | | | |
| 2.153 a-f | O-4-Cl-Ph | - | | | | | | |
| 2.154 a-f | O-2,6-di-Cl-Ph | - | | | | | | |
| 2.155 a-f | O-2,4-di-Cl-Ph | - | | | | | | |
| 2.156 a-f | O-2-CF₃-Ph | - | | | | | | |
| 2.157 a-f | O-3-CF₃-Ph | - | | | | | | |
| 2.158 a-f | O-4-CF₃-Ph | - | | | | | | |
| 2.159 a-f | O-2-MeO-Ph | - | | | | | | |
| 2.160 a-f | O-3-MeO-Ph | - | | | | | | |
| 2.161 a-f | O-4-MeO-Ph | - | | | | | | |
| 2.162 a-f | O-2,4-di-MeO-Ph | - | | | | | | |
| 2.163 a-f | O-2-Me-Ph | - | | | | | | |
| 2.164 a-f | O-3-Me-Ph | - | | | | | | |
| 2.165 a-f | O-4-Me-Ph | - | | | | | | |
| 2.166 a-f | OCH₂Ph | - | | | | | | |
| 2.167 a-f | OCH(CH₃)Ph | - | | | | | | |
| 2.168 a-f | OCH₂-2-F-Ph | - | | | | | | |
| 2.169 a-f | OCH₂-3-F-Ph | - | | | | | | |
| 2.170 a-f | OCH₂-4-F-Ph | - | | | | | | |
| 2.171 a-f | OCH₂-2,4-di-F-Ph | - | | | | | | |
| 2.172 a-f | OCH₂-2-Cl-Ph | - | | | | | | |
| 2.173 a-f | OCH₂-3-Cl-Ph | - | | | | | | |
| 2.174 a-f | OCH₂-4-Cl-Ph | - | | | | | | |
| 2.175 a-f | OCH₂-2,4-di-Cl-Ph | - | | | | | | |
| 2.176 a-f | OCH₂-2-MeO-Ph | - | | | | | | |
| 2.177 a-f | OCH₂-3-MeO-Ph | - | | | | | | |
| 2.178 a-f | OCH₂-4-MeO-Ph | - | | | | | | |
| 2.179 a-f | OCH₂-2-CF₃-Ph | - | | | | | | |
| 2.180 a-f | OCH₂-3-CF₃-Ph | - | | | | | | |
| 2.181 a-f | OCH₂-4-CF₃-Ph | - | | | | | | |
| 2.182 a-f | OCF₃ | - | * | * | | | | |
| 2.183 a-f | OCF₃ | 5-CH₃ | | | | | | |
| 2.184 a-f | OCHF₂ | - | * | | | | | |
| 2.185 a-f | OCHF₂ | 5-CH₃ | | | | | | |
| 2.186 a-f | OCH₂CF₃ | - | * | | | | | |
| 2.187 a-f | OCH₂CHF₂ | - | * | | | | | |
| 2.188 a-f | OCH₂CH₂F | - | | | | | | |
| 2.189 a-f | OCH(CH₃)CF₃ | - | * | | | | | |
| 2.190 a-f | OCH(CH₃)CHF₂ | - | | | | | | |
| 2.191 a-f | OCH(CH₃)CH₂F | - | | | | | | |
| 2.192 a-f | OCH₂CF₂CF₃ | - | * | | | | | |
| 2.193 a-f | OCH₂CF₂CHF₂ | - | | | | | | |
| 2.194 a-f | OCH₂CF₂CH₂F | - | | | | | | |
| 2.195 a-f | OCH(CH₃)CF₂CF₃ | - | | | | | | |
| 2.196 a-f | OCH(CH₃)CF₂CHF₂ | - | | | | | | |
| 2.197 a-f | OCH(CH₃)CF₂CH₂F | - | | | | | | |
| 2.198 a-f | OCH₂CHFCF₃ | - | | | | | | |
| 2.199 a-f | O(CH₂)₂CF₃ | - | | | | | | |
| 2.200 a-f | O(CH₂)₂CHF₂ | - | | | | | | |
| 2.201 a-f | O(CH₂)₃CF₃ | - | | | | | | |
| 2.202 a-f | O(CH₂)₃CHF₂ | - | | | | | | |
| 2.203 a-f | OCF=CH₂ | - | | | | | | |
| 2.204 a-f | OCH=CF₂ | - | | | | | | |
| 2.205 a-f | OCF₂CH=CH₂ | - | | | | | | |
| 2.206 a-f | OCHFCH=CH₂ | - | | | | | | |
| 2.207 a-f | OCH=CHCF₃ | - | | | | | | |
| 2.208 a-f | SCH₃ | - | * | * | | | | |
| 2.209 a-f | SCH₂CH₃ | | | | | | | |
| 2.210 a-f | S(CH₂)₂CH₃ | - | | | | | | |
| 2.211 a-f | SCH(CH₃)₂ | - | | | | | | |
| 2.212 a-f | SC(CH₃)₃ | - | | | | | | |
| 2.213 a-f | SCH₂Ph | - | | | | | | |
| 2.214 a-f | SPh | - | | | | | | |
| 2.215 a-f | SCF₃ | - | | | | | | |
| 2.216 a-f | SCHF₂ | - | | | | | | |
| 2.217 a-f | SCH₂F | - | | | | | | |
| 2.218 a-f | SCH=CH₂ | - | | | | | | |
| 2.219 a-f | SCH₂CH=CH₂ | - | | | | | | |
| 2.220 a-f | SC≡CH | - | | | | | | |
| 2.221 a-f | SCH₂C≡CH | - | | | | | | |
| 2.222 a-f | S-cyclopropyl | - | | | | | | |
| 2.223 a-f | SCH₂-cyclopropyl | - | | | | | | |
| 2.224 a-f | SF₅ | - | | | | | | |
| 2.225 a-f | S(O)CH₃ | - | | | | | | |
| 2.226 a-f | S(O)CH₂CH₃ | - | | | | | | |
| 2.227 a-f | S(O)(CH₂)₂CH₃ | - | | | | | | |
| 2.228 a-f | S(O)CH(CH₃)₂ | - | | | | | | |
| 2.229 a-f | S(O)C(CH₃)₃ | - | | | | | | |
| 2.230 a-f | S(O)CH₂Ph | - | | | | | | |
| 2.231 a-f | S(O)Ph | - | | | | | | |
| 2.232 a-f | S(O)CF₃ | - | | | | | | |
| 2.233 a-f | S(O)CHF₂ | - | | | | | | |
| 2.234 a-f | S(O)CH₂F | - | | | | | | |
| 2.235 a-f | S(O)CH=CH₂ | - | | | | | | |
| 2.236 a-f | S(O)CH₂CH=CH₂ | - | | | | | | |
| 2.237 a-f | S(O)C≡CH | - | | | | | | |
| 2.238 a-f | S(O)CH₂C≡CH | - | | | | | | |
| 2.239 a-f | S(O)-cyclopropyl | - | | | | | | |
| 2.240 a-f | S(O)CH₂-cyclopropyl | - | | | | | | |
| 2.241 a-f | SO₂CH₃ | - | | | | | | |
| 2.242 a-f | SO₂CH₂CH₃ | - | | | | | | |
| 2.243 a-f | SO₂(CH₂)₂CH₃ | - | | | | | | |
| 2.244 a-f | SO₂CH(CH₃)₂ | - | | | | | | |
| 2.245 a-f | SO₂C(CH₃)₃ | - | | | | | | |
| 2.246 a-f | SO₂CH₂Ph | - | | | | | | |
| 2.247 a-f | SO₂Ph | - | | | | | | |
| 2.248 a-f | SO₂CF₃ | - | | | | | | |
| 2.249 a-f | SO₂CHF₂ | - | | | | | | |
| 2.250 a-f | SO₂CH₂F | - | | | | | | |
| 2.251 a-f | SO₂CH=CH₂ | - | | | | | | |
| 2.252 a-f | SO₂CH2CH=CH2 | - | | | | | | |
| 2.253 a-f | SO₂C≡CH | - | | | | | | |
| 2.254 a-f | SO₂CH₂C≡CH | - | | | | | | |
| 2.255 a-f | SO₂-cyclopropyl | - | | | | | | |
| 2.256 a-f | SO₂CH₂-cyclopropyl | - | | | | | | |
| 2.257 a-f | SO₂NHCH₃ | - | | | | | | |
| 2.258 a-f | SO₂N(CH₃)₂ | - | | | | | | |
| 2.259 a-f | OSO₂CH₃ | - | * | | | | | |
| 2.260 a-f | OSO₂CH₃ | 5-CH₃ | | | | | | |
| 2.261 a-f | OSO₂CH₂CH₃ | - | | | | | | |
| 2.262 a-f | OSO₂CH(CH₃)₂ | - | | | | | | |
| 2.263 a-f | OSO₂C(CH₃)₃ | - | | | | | | |
| 2.264 a-f | OSO₂CH₂Ph | - | | | | | | |
| 2.265 a-f | OSO₂CF₃ | - | | | | | | |
| 2.266 a-f | OSO₂CHF₂ | - | | | | | | |
| 2.267 a-f | OSO₂CH₂F | - | | | | | | |
| 2.268 a-f | OSO₂CH₂CF₃ | - | | | | | | |
| 2.269 a-f | OSO₂CH₂CHF₂ | - | | | | | | |
| 2.270 a-f | OSO₂(CH₂)₂F | - | | | | | | |
| 2.271 a-f | OSO₂CH=CH₂ | - | | | | | | |
| 2.272 a-f | OSO₂CH₂CH=CH₂ | - | | | | | | |
| 2.273 a-f | OSO₂C≡CH | - | | | | | | |
| 2.274 a-f | OSO₂CH₂C≡CH | - | | | | | | |
| 2.275 a-f | OSO₂-cyclopropyl | - | | | | | | |
| 2.276 a-f | OSO₂CH₂-cyclopropyl | - | | | | | | |
| 2.277 a-f | OSO₂CH₂CN | - | | | | | | |
| 2.278 a-f | OSO₂NHCH₃ | - | | | | | | |
| 2.279 a-f | OSO₂N(CH₂)₂ | - | * | | | | | |
| 2.280 a-f | OSO₂NHCH₂CH=CH₂ | - | | | | | | |
| 2.281 a-f | OSO₂NHCH₂C≡CH | - | | | | | | |
| 2.282 a-f | OSO₂NHCF₃ | - | | | | | | |
| 2.283 a-f | OSO₂NHCHF₂ | - | | | | | | |
| 2.284 a-f | OSO₂NHCH₂F | - | | | | | | |
| 2.285 a-f | OC(O)H | - | | | | | | |
| 2.286 a-f | OC(O)CH₃ | - | | | | | | |
| 2.287 a-f | OC(O)CH₂CH₃ | - | | | | | | |
| 2.288 a-f | OC(O)OCH₃ | - | | | | | | |
| 2.289 a-f | OC(O)OCH₂CH₃ | - | | | | | | |
| 2.290 a-f | OC(O)NH₂ | - | | | | | | |
| 2.291 a-f | OC(O)NHCH₃ | - | | | | | | |
| 2.292 a-f | OC(O)N(CH₃)₂ | - | | | | | | |
| 2.293 a-f | OC(O)N(CH₂CH₃)₂ | - | | | | | | |
| 2.294 a-f | Si(CH₃)₃ | - | | | | | | |
| 2.295 a-f | 2-thienyl | - | | | | | | |
| 2.296 a-f | 3-thienyl | - | | | | | | |
| 2.297 a-f | 2-pyridyl | - | | | | | | |
| 2.298 a-f | 3-pyridyl | - | | | | | | |
| 2.299 a-f | 4-pyridyl | - | | | | | | |
| 2.300 a-f | OH | - | * | | | | | |

¹H-NMR Daten:
Beispiel-Nr.: 2.001 a (d₆-DMSO): 7.96 (dt, J = 0.9, 7.8, 1 H); 7.78 (br s, 2H); 7.43 (ddd, J = 1.1, 8.3, 11.2, 1 H); 7.28 (dt, J = 5.4, 8.0, 1 H).
Beispiel-Nr.: 2.004a (d₆-DMSO): 7.89 (br d, J = 7.8, 1 H); 7.55 (td, J = 0.9, 7.6, 1 H); 7.49 (br s, 2H); 7.40 (td, J = 1.2, 7.9, 1 H), 3.34 (s, 3H).
Beispiel-Nr.: 2.063a (d₆-DMSO): 8.48 (dd, J = 1.0, 7.9, 1 H); 7.95 (dd, J = 1.0, 8.0, 1 H); 7.74 (br s, 2H); 7.39 (br t, J = 8.3, 1 H).
Beispiel-Nr.: 2.092a (d₆-DMSO): 7.70 (dd, J = 1.1, 7.7, 1 H); 7.25 (dd, J = 0.9, 8.4, 1 H); 7.20 (br s, 2H); 7.17 (t, J = 7.9, 1 H); 3.90 (s, 3H).
Beispiel-Nr.: 2.094a (d₆-DMSO): 7.70 (dd, J = 1.0, 7.8, 1 H); 7.26 (br d, J = 8.4, 1 H); 7.15 (t, J = 8.0, 1 H); 7.03 (br s, 2H); 4.23 (q, J = 7.0, 2H); 1.37 (t, J = 6.9, 3H).
Beispiel-Nr.: 2.095a (d₆-DMSO): 7.70 (dd, J = 1.0, 7.8, 1 H); 7.26 (dd, J = 0.9, 8.4, 1 H); 7.15 (t, J = 7.9, 1 H); 6.98 (br s, 2H); 4.12 (t, J = 6.6, 2H); 1.80 (m, 2H); 0.96 (t, J = 7.4, 3H).
Beispiel-Nr.: 2.096a (d₆-DMSO): 7.69 (dd, J = 1.0, 1 H); 7.29 (br d, J = 8.1, 1 H); 7.14 (t, J = 8.0, 1 H); 6.91 (br s, 2H); 4.82 (m, 1 H); 1.34 (d, J = 6.0, 6H).
Beispiel-Nr.: 2.128a (d₆-DMSO): 7.71 (dd, J = 1.1, 7.8, 1H); 7.27 (dd, J = 1.1, 8.4, 1H); 7.15 (t, J = 7.8, 1H); 7.01 (br s, 2H); 4.04 (d, J = 7.2, 2H); 1.33 (m, 1H); 0.56 (m, 2H); 0.36 (m, 2H).
Beispiel-Nr.: 2.182a (d₆-DMSO): 8.19 (dd, J = 1.1, 7.9, 1 H); 7.67 (br s, 2H); 7.55 (dt, J = 1.3, 8.3, 1 H); 7.34 (t, J = 8.1, 1 H).
Beispiel-Nr.: 2.184a (CDCl₃): 7.96 (dd, J = 1.2, 8.0, 1 H); 7.25 (m, 1 H); 7.07 (t, J = 8.1, 1 H); 6.51 (t, J = 73.8, 1 H); 6.37 (br s, 2H).
Beispiel-Nr.: 2.186a (d₆-DMSO): 7.84 (dd, J = 0.9, 7.9, 1 H); 7.40 (br d, J = 8.2, 1 H); 7.23 (t, J = 8.0, 1 H); 7.07 (br s, 2H); 4.99 (q, J = 8.8, 2H).
Beispiel-Nr.: 2.187a (d₆-DMSO): 7.79 (dd, J = 1.0, 7.8, 1H); 7.35 (dd, J = 0.8, 8.4, 1 H); 7.20 (t, J = 7.9, 1 H); 7.10 (br s, 2H); 6.53 (tt, J = 3.7, 54.7, 1H); 4.51 (td, J = 3.7, 14.3, 2H).
Beispiel-Nr.: 2.189a (d₆-DMSO): 7.83 (dd, J = 0.8, 7.9, 1 H); 7.46 (br d, J = 8.3, 1 H); 7.22 (t, J = 8.1, 1 H); 6.91 (br s, 2H); 5.50 (m, 1 H); 1.48 (d, J = 6.4, 3H).
Beispiel-Nr.: 2.192a (d₆-DMSO): 7.85 (dd, J = 1.0, 7.9, 1 H); 7.42 (br d, J = 8.3, 1 H); 7.24 (t, J = 8.0, 1 H); 7.00 (br s, 2H); 5.07 (t, J = 13.9, 2H).
Beispiel-Nr.: 2.208a (d₆-DMSO): 7.95 (dd, J = 0.9, 7.7, 1 H); 7.46 (m, 3H); 7.12 (t, J = 7.9, 1H); 2.41 (s, 3H).
Beispiel-Nr.: 2.259a (d₆-DMSO): 8.13 (dd, J = 1.2, 7.9, 1 H); 7.60 (br s, 2H); 7.53 (dd, J = 1.2, 8.2, 1 H); 7.31 (t, J = 8.0, 1 H); 3.53 (s, 3H).
Beispiel-Nr.: 2.279a (d₆-DMSO): 8.07 (dd, J = 1.2, 7.9, 1 H); 7.52 (dd, J = 1.2, 8.2, 1 H); 7.19 (t, J = 8.1, 1 H); 5.48 (br s, 2H); 3.10 (s, 6H).
Beispiel-Nr.: 2.300a (CDCl₃): 9.91 (s, 1 H); 7.60 (m, 1 H); 7.05 (m, 2H); 5.49 (br s, 2H).
Beispiel-Nr. 2.001 b (d₆-DMSO): 7.99 (dt, J = 0.7, 7.7, 1 H); 7.79 (br s, 1 H); 7.42 (ddd, J = 1.1, 8.1, 11.0, 1 H); 7.28 (td, J = 5.1, 7.7, 1 H); 1.13 (s, 9H).
Beispiel-Nr. 2.004b (d₆-DMSO): 7.92 (dd, J = 1.1, 7.7, 1 H); 7.55 (m, 2H); 7.39 (m, 1 H); 3.31 (s, 3H); 1.14 (s, 9H).
Beispiel-Nr. 2.063b (d₆-DMSO): 8.49 (dd, J = 1.1, 7.7, 1 H); 7.97 (dd, J = 1.1, 8.1, 1 H); 7.78 (br s, 1 H); 7.40 (td, J = 0.7, 8.1, 1 H); 1.07 (s, 9H).
Beispiel-Nr. 2.092b (CDCl₃): 7.80 (m, 1 H); 7.06 (m, 2H); 5.28 (br s, 1 H); 3.99 (s, 3H); 1.22 (s, 9H).
Beispiel-Nr. 2.182b (CDCl₃): 8.12 (dd, J = 0.8, 8.0, 1 H); 7.41 (dt, J = 0.8, 8.0, 1 H); 7.18 (t, J = 8.0, 1 H); 5.12 (br s, 1 H); 1.27 (s, 9H).
Beispiel-Nr. 2.208b (CDCl₃): 7.96 (dd, J = 1.0, 7.5, 1 H); 7.35 (dd, J = 0.7, 8.5, 1 H); 7.00 (t, J = 7.8, 1 H); 5.74 (br s, 1 H); 2.47 (s, 3H); 1.25 (s, 9H).

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen Nr. 1.001, 1.002, 1.003, 1.004, 1.012, 1.016, 1.020, 1.021, 1.025, 1.091, 1.095, 1.099, 1.100, 1.104, 1.146, 1.150, 1.154, 1.155, 1.159, 1.164, 1.165, 1.166, 1.167, 1.168, 1.171, 1.173, 1.174, 1.175, 1.178, 1.182, 1.186, 1.187, 1.191, 1.229, 1.233, 1.237, 1.238, 1.242, 1.302, 1.306, 1.310, 1.311, 1.315, 1.321, 1.325, 1.329, 1.330, 1.342, 1.343, 1.344, 1.352, 1.353, 1.354, 1.355,1.362,1.363, 1.364,1.365, 1.366,1.370, 1.371,1.372, 1.373,1.389, 1.390, 1.463,1.471,1.472,1.476, 1.507, 1.509,1.510,1.511, 1.550, 1.551 und andere Verbindungen aus Tabelle 1 sehr gute herbizide Wirkung gegen Schadpflanzen wie Sinapis alba, Chrysanthemum segetum, Avena sativa, Stellaria media, Echinochloa crus-galli, Lolium multiflorum, Setaria viridis, Abutilon theophrasti, Amaranthus retroflexus und Panicum miliaceum im Vorauflaufverfahren bei einer Aufwandmenge von 0.3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen Nr. 1.001, 1.002, 1.003, 1.004, 1.012, 1.016, 1.020, 1.021, 1.025, 1.091, 1.095, 1.099, 1.100, 1.104, 1.146, 1.150, 1.154, 1.155, 1.159, 1.164, 1.165, 1.166, 1.167, 1.168, 1.171, 1.173, 1.174, 1.175, 1.178, 1.182, 1.186, 1.187, 1.191, 1.229, 1.233, 1.237, 1.238, 1.242, 1.302, 1.306, 1.310, 1.311, 1.315, 1.321, 1.325, 1.329, 1.330, 1.342, 1.343, 1.344, 1.352, 1.353, 1.354, 1.355,1.362,1.363, 1.364,1.365, 1.366,1.370, 1.371,1.372, 1.373,1.389, 1.390, 1.463,1.471,1.472,1.476, 1.507, 1.509,1.510,1.511, 1.550, 1.551 und andere Verbindungen aus Tabelle 1 sehr gute herbizide Wirkung gegen Schadpflanzen wie Sinapis alba, Echinochloa crus-galli, Lolium multiflorum, Chrysanthemum segetum, Setaria viridis, Abutilon theophrasti, Amaranthus retroflexus, Panicum miliaceum und Avena sativa im Nachauflaufverfahren bei einer Aufwandmenge von 0.3 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Verbindungen in unterschiedlichen Dosierungen besprüht. Vier bis fünf Wochen nach der Applikation und Standzeit im Gewächshaus wurde mittels optischer Bonitur festgestellt, daß erfindungsgemäße
Verbindungen zweikeimblättrige Kulturen wie Soja, Baumwolle, Raps, Zuckerrüben oder Kartoffeln im Vor- und Nachauflaufverfahren selbst bei hohen Wirkstoffdosierungen ungeschädigt ließen. Einige Substanzen schonten darüber hinaus auch Gramineen-Kulturen wie Gerste, Weizen, Roggen, Hirsen, Mais oder Reis. Die erfindungsgemäßen Verbindungen zeigen teilweise eine hohe Selektivität und eignen sich deshalb zur Bekämpfung von unerwünschten Pflanzenwuchs in landwirtschaftlichen Kulturen.

## Patentansprüche

1. Verbindung der Formel (I) und/oder deren Salze 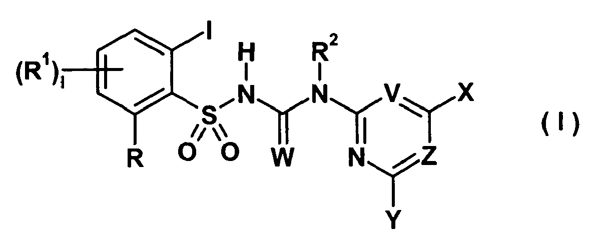 worin
R ein Kohlenwasserstoffrest oder Kohlenwasserstoffoxyrest ist, der unsubstituiert oder substituiert ist und inklusive Substituenten 1 bis 30 C-Atome aufweist, oder ein Heterocyclylrest oder Heterocyclyloxyrest ist, welcher unsubstituiert oder substituiert ist,
oder R ein Rest OC(O)R³, S(O)ₙR³, OS(O)ₙR³, F, Br, I, OH, CN, NO₂, NH₂, SF₅, NR⁴R⁵ oder Si(R⁶)₃ ist, wobei n gleich 0, 1 oder 2 ist,
R¹ unabhängig voneinander Halogen, OH, SH, ein kohlenstofffreier stickstoffhaltiger Rest oder ein kohlenstoffhaltiger Rest sind, der 1 bis 30 C-Atome aufweist,
I 0, 1, 2 oder 3 ist,
R² ein Wasserstoffatom oder ein Kohlenwasserstoffrest ist, der unsubstituiert oder substituiert ist und inklusive Substituenten 1 bis 20 C-Atome aufweist,
R³ ein Kohlenwasserstoffrest oder Kohlenwasserstoffoxyrest ist, der unsubstituiert oder substituiert ist und inklusive Substituenten 1 bis 30 C-Atome aufweist, oder ein Heterocylylrest oder Heterocylyloxyrest ist, welcher unsubstituiert oder substituiert ist, oder R³ ein Wasserstoffatom, CN oder NR⁴R⁵ ist,
R⁴ eine Gruppe der Formel R⁰-Q⁰- ist, worin
R⁰ ein Wasserstoffatom, ein Acylrest, ein Kohlenwasserstoffrest oder ein Heterocyclylrest bedeutet, wobei jeder der letztgenannten beiden Reste unsubstituiert oder substituiert ist und inklusive Substituenten 1 bis 30 C-Atome aufweist, und
Q⁰ eine direkte Bindung oder eine divalente Gruppe der Formel ―O- oder ― N(R^{#})- bedeutet, wobei R^{#} ein Wasserstoffatom, ein Acylrest oder ein Kohlenwasserstoffrest bedeutet und wobei der letztgenannte Rest unsubstituiert oder substituiert ist und inklusive Substituenten 1 bis 30 C-Atome aufweist, oder R⁰ und R^{#} miteinander einen stickstoffhaltigen heterocyclischen Ring bilden,
R⁵ ein Wasserstoffatom, ein Acylrest, ein Kohlenwasserstoffrest oder ein Heterocyclylrest ist, wobei jeder der letztgenannten beiden Reste unsubstituiert oder substituiert ist und inklusive Substituenten 1 bis 30 C-Atome aufweist, oder
R⁴ und R⁵ miteinander einen stickstoffhaltigen heterocyclischen Ring bilden,
R⁶ ein Kohlenwasserstoffrest ist, der unsubstituiert oder substituiert ist und inklusive Substituenten 1 bis 30 C-Atome aufweist,
W ein Sauerstoffatom oder ein Schwefelatom ist,
X,Y unabhängig voneinander für ein Wasserstoffatom, Halogen, (C₁-C₆)Alkyl, (C₁₋C₆)Alkoxy oder (C₁-C₆)Alkylthio ist, wobei jeder der letztgenannten 3 Reste unsubstituiert oder durch einen oder mehrere Reste aus der Gruppe Halogen, (C₁-C₄)Alkoxy, und (C₁-C₄)Alkylthio substituiert ist, oder Mono- oder Di[(C₁₋C₆)alkyl] amino, (C₂-C₆)Alkenyl, (C₂-C₆)Alkinyl, (C₃-C₆)Alkenyloxy oder (C₃₋C₆)Alkinyloxy bedeuten, und
V,Z unabhängig voneinander CH oder N bedeuten.

2. Verbindung der Formel (I) und/oder deren Salze gemäß Anspruch 1, worin
R (C₁-C₆)Alkyl, (C₂-C₆)Alkenyl, (C₂-C₆)Alkinyl, (C₃-C₆)Cycloalkyl, (C₃₋C₆)Cycloalkenyl, (C₃-C₆)Cycloalkinyl, (C₁-C₆)Alkyloxy, (C₂-C₆)Alkenyloxy, (C₂₋C₆)Alkinyloxy, (C₃-C₆)Cycloalkyloxy, Phenyl, Phenyloxy, F, Br, I, OH, CN, NO₂, NH₂, SF₅, Si((C₁-C₆)Alkyl)₃, N((C₁-C₆)Alkyl)₂, NH(C₁-C₆)Alkyl, N((C₂₋C₆)Alkenyl)₂, NH(C₂-C₆)Alkenyl, N((C₂-C₆)Alkinyl)₂, NH(C₂-C₆)Alkinyl, NH((C₃₋C₆)Cycloalkyl)₂, NH(C₃-C₆)Cycloalkyl, N(C₁-C₆)Alkyl (C₃-C₆)Cycloalkyl, N(C₁₋C₆)Alkyl C(O)R³, NHC(O)R³, N(C₁-C₆)Alkyl S(O)ₙR³, NHS(O)ₙR³, S(O)ₙ(C₁₋C₄)Alkyl, S(O)ₙ (C₃-C₆)Cycloalkyl, S(O)ₙ(C₁-C₆)Alkenyl, S(O)ₙ(C₁-C₆)Alkinyl, S(O)ₙNHR³, S(O)ₙN(C₁-C₆)Alkyl R³, OSO₂(C₁-C₆)Alkyl, OSO₂(C₃₋C₆)Cycloalkyl, OSO₂(C₁-C₆)Alkenyl, OSO₂(C₁-C₆)Alkinyl, OS(O)ₙPhenyl, OSO₂N((C₁-C₆)Alkyl)₂, OSO₂NH(C₁-C₆)Alkyl, OSO₂N((C₃-C₆)Cycloalkyl)₂, OSO₂NH(C₃-C₆)Cycloalkyl, OSO₂N((C₂-C₆)Alkenyl)₂, OSO₂NH(C₂-C₆)Alkenyl, OSO₂N((C₂-C₆)Alkinyl)₂, OSO₂NH(C₂-C₆)Alkinyl, OC(O)R³ oder Heterocyclyl bedeutet, wobei die genannten Reste Alkyl, Alkenyl, Alkinyl, Cycloalkyl, Cycloalkenyl, Cycloalkinyl, Alkyloxy, Alkenyloxy, Alkinyloxy, Cycloalkoxy, Phenyl, Phenyloxy, Heterocyclyl unsubstituiert sind oder substituiert sind,
R¹ (C₁-C₆)Alkyl, (C₁-C₆)Haloalkyl, (C₁-C₆)Alkyloxy, (C₁-C₆)Haloalkoxy oder Halogen ist,
l 0, 1 oder 2 ist,
n 0, 1 oder 2 ist,
R² H oder CH₃ ist,
R³ H, (C₁-C₆)Alkyl, (C₂-C₆)Alkenyl, (C₂-C₆)Alkinyl, (C₃-C₆)Cycloalkyl, (C₁₋C₆)Alkyloxy, (C₂-C₆)Alkenyloxy, (C₂-C₆)Alkinyloxy, (C₃-C₆)Cycloalkyloxy, Phenyl, Heterocyclyl, CN, NH(C₁-C₆)Alkyl, N((C₁-C₆)Alkyl)₂ steht, wobei die genannten Reste Alkyl, Alkenyl, Alkinyl, Cycloalkyl, Alkyloxy, Alkenyloxy, Alkinyloxy, Cycloalkyloxy, Phenyl, Heterocyclyl unsubstituiert oder substituiert sind,
W ein Sauerstoffatom ist,
X,Y unabhängig voneinander (C₁-C₄)Alkyl, (C₁-C₄)Alkyloxy, wobei jeder der letztgenannten zwei Reste unsubstituiert oder durch einen oder mehrere Halogenatome substituiert ist, oder (C₁-C₄)Alkylthio, Halogen oder NH(C₁₋C₄)Alkyl oder N((C₁-C₄)Alkyl)₂ bedeuten, und
V,Z unabhängig voneinander CH oder N bedeuten.

3. Verbindung der Formel (I) und/oder deren Salze gemäß Anspruch 1 oder 2, worin
R (C₁-C₄)Alkyl, (C₂-C₄)Alkenyl, (C₂-C₄)Alkinyl, (C₃-C₆)Cycloalkyl, (C₁₋C₄)Alkyloxy, (C₂-C₄)Alkenyloxy, (C₂-C₄)Alkinyloxy, (C₃-C₆)Cycloalkyloxy, Phenyl, Phenyloxy, F, Br, I, CN, NO₂, NH₂, N((C₁-C₄)Alkyl)₂, NH(C₁-C₄)Alkyl, NH(C₂-C₄)Alkenyl, NH(C₂-C₄)Alkinyl, NH(C₃-C₆)Cycloalkyl, N(C₁-C₄)Alkyl (C₃₋C₆)Cycloalkyl, S(C₁-C₄)Alkyl, S(C₂-C₄)Alkenyl, S(C₂-C₄)Alkinyl, S(C₃₋C₆)Cycloalkyl, S(O)(C₁-C₄)Alkyl, S(O)(C₁-C₄)Alkenyl, S(O)(C₂-C₄)Alkinyl, S(O)(C₃-C₆)Cycloalkyl, SO₂(C₁-C₄)Alkyl, SO₂(C₂-C₄)Alkenyl, SO₂(C₂₋C₄)Alkinyl, SO₂(C₃-C₆)Cycloalkyl, SO₂NH(C₁-C₄)Alkyl, SO₂N((C₁-C₄)Alkyl)₂, SO₂NH(C₃-C₆)Cycloalkyl, OSO₂(C₁-C₄)Alkyl, OSO₂NH(C₁-C₄)Alkyl, OSO₂N((C₁-C₄)Alkyl)₂ oder NHC(O)R³, NHSO₂R³, OC(O)R³, worin R³ gleich H, (C₁-C₄)Alkyl, (C₂-C₄)Alkenyl, (C₂-C₄)Alkinyl, (C₃₋C₆)Cycloalkyl, (C₁-C₄)Alkyloxy, (C₂-C₄)Alkenyloxy, (C₂-C₄)Alkinyloxy, (C₃₋C₆)Cycloalkyloxy, (C₁-C₄)Haloalkyl, NH(C₁-C₄)Alkyl oder N((C₁-C₄)Alkyl)₂ ist, wobei die genannten Reste Alkyl, Alkenyl, Alkinyl, Cycloalkyl, Alkyloxy, Alkenyloxy, Alkinyloxy, Cycloalkyloxy, Phenyl, Phenyloxy unsubstituiert sind oder substituiert sind,
R¹ Halogen, (C₁-C₄)Alkyl, (C₁-C₄)Alkyloxy, (C₁-C₄)Haloalkyl, (C₁-C₄)Haloalkyloxy,
I 0 oder 1, vorzugsweise 0 ist,
R² H oder (C₁-C₄)Alkyl wie Methyl,
W ein Sauerstoffatom,
X,Y unabhängig voneinander (C₁-C₄)Alkyl, (C₁-C₄)Haloalkyl, (C₁-C₄)Alkyloxy, (C₁₋C₄)Haloalkyloxy, Halogen, (C₁-C₄)Alkylthio, NH(C₁-C₄)Alkyl, N((C₁-C₄)Alkyl)₂,
V ein Stickstoffatom, und
Z CH oder N bedeuten.

4. Verbindung der Formel (I) und/oder deren Salze gemäß einem oder mehreren der Ansprüche 1 bis 3, worin
R CH₃, CH₂CH₃, (CH₂)₂CH₃, CH(CH₃)₂, C(CH₃)₃, CH=CH₂, C≡CH, CH₂CH=CH₂, CH₂C≡CH, Cyclopropyl, Phenyl, F, Br, I, CN, NO₂, NH₂, CH₂OCH₃, CF₃, CHF₂, NHCH₃, N(CH₃)₂, NH-cyclopropyl, N(CH₃)-cyclopropyl, NHC(O)H, NHC(O)CH₃, NHC(O)OCH₃, NHSO₂CH₃, NHSO₂CF₃, NHSO₂CHF₂, OCH₃, OCH₂CH₃, O(CH₂)₂CH₃, OCH(CH₃)₂, O(CH₂)₃CH₃, OCH₂CH(CH₃)₂, OCH(CH₃)CH₂CH₃, OC(CH₃)₃, OCH=CH₂, OC≡CH, OCH₂CH=CH₂, OCH₂C≡CH, O-cyclopropyl, OCH₂-cyclopropyl, O(CH₂)₂Cl, O(CH₂)₃Cl, OCH₂OCH₃, OPhenyl, OCH₂Phenyl, OCF₃, OCHF₂, OCH₂F, OCH₂CF₃, OCH₂CHF₂, OCH(CH₃)CF₃, OCH₂CF₂CF₃, SCH₃, SCH₂CH₃, S(O)CH₃, S(O)CH₂CH₃, SO₂CH₃, SO₂CH₂CH₃, SO₂NHCH₃, SO₂N(CH₃)₂, SO₂NHCF₃, SO₂NHCHF₂, OSO₂CH₃, OSO₂CF₃, OSO₂CHF₂, OSO₂N(CH₃)₂, OSO₂NHCF₃, OSO₂NHCHF₂, OC(O)H, OC(O)CH₃, OC(O)OCH₃, OC(O)N(CH₃)₂,
I 0,
R² H,
W Sauerstoff,
X,Y unabhängig voneinander CH₃, CH₂CH₃, CF₃, CHF₂, CH₂CF₃, CH₂CHF₂, OCH₃, OCH₂CH₃, OCF₃, OCHF₂, OCH₂CF₃, OCH₂CHF₂, F, Cl, Br, I, SCH₃, NHCH₃, N(CH₃)₂, vorzugsweise CH₃, OCH₃, OCH₂CH₃, Cl, N(CH₃)₂,
V N, und
Z CH oder N bedeuten.

5. Verfahren zur Herstellung einer Verbindung der Formel (I) und/oder deren Salze, wie in einem oder mehreren der Ansprüche 1 bis 4 definiert, wobei man
a) eine Verbindung der Formel (II) 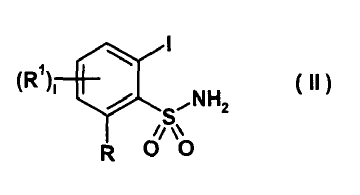 mit einem heterocyclischen (Thio)Carbamat der Formel (III), 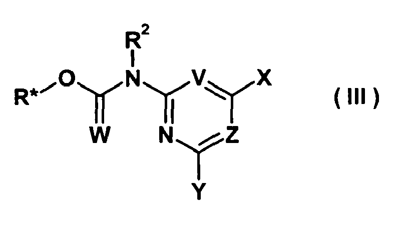 worin R* ein substituierter oder unsubstituierter C₁-C₂₀-Kohlenwasserstoffrest bedeutet, umsetzt, oder
b) ein Sulfonyl(thio)carbamat der Formel (IV), 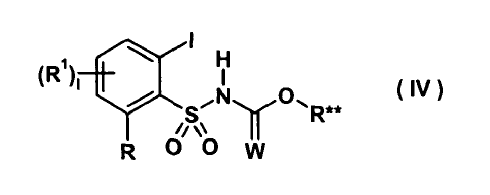 worin R** ein substituierter oder unsubstituierter C₁-C₂₀-Kohlenwasserstoffrest bedeutet, mit einem Aminoheterocyclus der Formel (V) 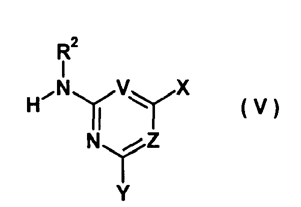 umsetzt, oder
c) ein Sulfonyliso(thio)cyanat der Formel (VI) 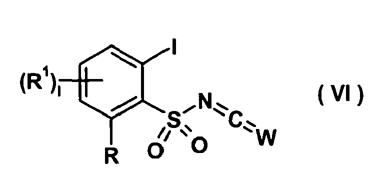 mit einem Aminoheterocyclus der Formel (V) umsetzt, oder
d) ein Sulfonamid der Formel (II) mit einem Iso(thio)cyanat der Formel (VII) 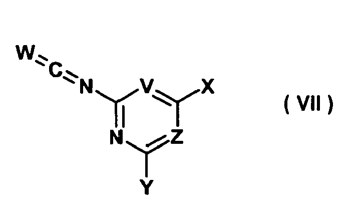 in Gegenwart einer Base umsetzt, oder
e) einen Aminoheterocyclus der Formel (V) zunächst basenkatalysiert mit einem Kohlensäureester umsetzt und das gebildete Intermediat in einer Eintopfreaktion mit einem Sulfonamid der Formel (II) (siehe Variante a) umsetzt, oder
f) ein Sulfonsäurehalogenid der Formel (VIII), 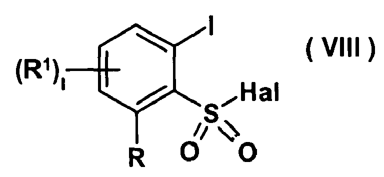 wobei Hal ein Halogenatom ist, mit einem (Thio)Cyanat, zu einem Iso(thio)cyanat der Formel (VI) oder einem solvatisierten (stabilisierten) Derivat davon umsetzt, und dieses anschließend mit einem Aminoheterocyclus der Formel (V) umsetzt,
wobei in den Formeln (II) bis (VIII) die Reste, Gruppen bzw. Indizes R, R¹, R², V, W, X, Y, Z und I wie in Formel (I) gemäß einem oder mehreren der Ansprüche 1 bis 4 definiert sind.

6. Agrochemisches Mittel, enthaltend a) mindestens eine Verbindung der Formel (I) und/oder deren Salze, wie in einem oder mehreren der Ansprüche 1 bis 4 definiert, und b) im Pflanzenschutz übliche Hilfs- und Zusatzstoffe.

7. Agrochemisches Mittel, enthaltend a) mindestens eine Verbindung der Formel (I) und/oder deren Salze, wie in einem oder mehreren der Ansprüche 1 bis 4 definiert, und b) einen oder mehrere von Komponente a) verschiedene agrochemische Wirkstoffe, und optional c) im Pflanzenschutz übliche Hilfs- und Zusatzstoffe.

8. Verfahren zur Bekämpfung von unerwünschten Pflanzen oder zur Wachstumsregulierung von Pflanzen, wobei eine wirksame Menge von mindestens einer Verbindung der Formel (I) und/oder deren Salze, wie in einem oder mehreren der Ansprüche 1 bis 4 definiert, auf die Pflanzen, das Saatgut oder die Fläche, auf der die Pflanzen wachsen, appliziert wird.

9. Verwendung von Verbindungen der Formel (I) und/oder deren Salze, wie in einem oder mehreren der Ansprüche 1 bis 4 definiert, als Herbizide oder Pflanzenwachstumsregulatoren.

10. Verwendung nach Anspruch 9, wobei die Verbindungen der Formel (I) und/oder deren Salze zur Bekämpfung von Schadpflanzen oder zur Wachstumsregulierung in Pflanzenkulturen eingesetzt werden.

11. Verwendung nach Anspruch 10, wobei die Kulturpflanzen transgene oder nicht transgene Kulturpflanzen sind.

12. Verwendung einer Verbindung der Formel (II*) 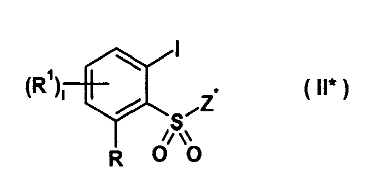 worin Z* = NH₂, NH-tert. Butyl, NH-C(O)OR**, NH-C(S)OR**, NCO, NCS oder Halogen bedeutet und R, R¹ und der Index I wie in Formel (I) gemäß einem oder mehreren der Ansprüche 1 bis 4 definiert sind und R** wie in Formel (IV) in Anspruch 5 definiert ist, zur Herstellung einer Verbindung der Formel (I) und/oder deren Salze wie in einem oder mehreren der Ansprüche 1 bis 4 definiert.

13. Verbindung der Formel (II*) wie in Anspruch 12 definiert, wobei ausgenommen sind Verbindungen worin Z*=NH₂, I=0 und R ein Rest NH₂, NH-CO-CO-O-C₂H₅, O(CH₂)₂F, O(CH₂)₃F bedeutet, oder worin Z*=NH₂ oder Chlor und R = lod bedeutet.
